# EUROPEAN PATENT APPLICATION

(11) **EP 1 632 470 A2**
(43) Date of publication of application: **08.03.2006**
(21) Application number: 05077693.9
(22) Date of filing: 21.08.1992
(51) Int. Cl.: C07C 211/27, C07D 209/14, C07C 229/38, C07C 217/58

(54) **Calcium receptor active molecules**

(30) Priority: 23.08.1991 US 749451; 11.02.1992 US 834044
(62) Divisional of application: 02016612.0
(71) Applicant: NPS PHARMACEUTICALS, INC., Salt Lake City Utah 84108 (US)
(72) Inventor: Nemeth, Edward F., Toronto Ontaria M5G 1K2 (CA); Van Wagenen, Bradford C., Salt Lake City Utah 84124 (US); Balandrin, Manuel F., Sandy Utah 84093 (US)
(74) Representative: Bradley, Adrian

(57) **Abstract**

Method and composition useful for treating a patient having a disease characterized by an abnormal level of one or more components, the activity of which is regulated or affected by activity of one or more Ca²⁺ receptors. Novel compounds useful in these methods and compositions are also provided. The method includes administering to the patient a therapeutically effective amount of a molecule active at one or more Ca²⁺ receptors as an agonist or antagonist. Preferably, the molecule is able to act as either a selective agonist or antagonist at a Ca²⁺ receptor of one or more but not all cells chosen from the group consisting of parathyroid cells, bone osteoclasts, juxtaglomerular kidney cells, proximal tubule kidney cells, keratinocytes, parafollicular thyroid cells and placental throphoblasts and a pharmaceutically acceptable carrier.

## Description

### Related Application

This application is a continuation-in-part of Nemeth, et al., entitled Calcium Receptor Active Molecules, filed February 11, 1992, which is a continuation-in-part of Nemeth et al., entitled Calcium Receptor Activators, U.S. Serial No. 07/749,451, filed August 23, 1991, the whole of both of which, including drawings, are hereby incorporated herein by reference.

### Field of the Invention

This invention relates to the design, development, composition and use of novel calcimimetic molecules able to act in a manner analogous to extracellular calcium ions on cells, to calcilytic molecules which block the activity of extracellular calcium ions on cells, and to methods for their use and identification.

### Background of the Invention

The following description provides a summary of information relevant to the present invention. It is not an admission that any of the information provided herein is prior art to the presently claimed invention, nor that any of the publications specifically or implicitly referenced are prior art to that invention.

Certain cells in the body respond not only to chemical signals, but also to ions such as extracellular calcium ions (Ca²⁺). Changes in the concentration of extracellular Ca²⁺ (referred to herein as "[Ca²⁺]") alter the functional responses of these cells. One such specialized cell is the parathyroid cell which secretes parathyroid, hormone (PTH). PTH is the principal endocrine factor regulating Ca²⁺ homeostasis in the blood and extracellular fluids.

PTH, by acting on bone and kidney cells, increases the level of Ca²⁺ in the blood. This increase in [Ca²⁺] then acts as a negative feedback signal, depressing PTH secretion. The reciprocal relationship between [Ca²⁺] and PTH secretion forms the essential mechanism maintaining bodily Ca²⁺ homeostasis.

Extracellular Ca²⁺ acts directly on the parathyroid cell to regulate PTH secretion. The existence of a parathyroid cell surface protein which detects changes in [Ca²⁺] has been suggested. This protein acts as a receptor for extracellular Ca²⁺ ("the Ca²⁺ receptor"), and is suggested to detect changes in [Ca²⁺] and to initiate a functional cellular response, PTH secretion. For example, the role of Ca²⁺ receptors and extracellular Ca²⁺ in the regulation of intracellular Ca²⁺ and cell function is reviewed in Nemeth et al., 11 Cell Calcium 319, 1990; the role of Ca²⁺ receptors in parafollicular and parathyroid cells is discussed in Nemeth, 11 Cell Calcium 323, 1990; and the role of Ca²⁺ receptors on bone osteoclasts is discussed by Zaidi, 10 Bioscience Reports 493, 1990.

Other cells in the body, specifically the osteoclast in bone, the juxtaglomerular and the proximal tubule cells in the kidney, the keratinocyte in the epidermis, the parafollicular cell in the thyroid, and the trophoblast in the placenta, have the capacity to sense changes in [Ca²⁺] . It has been suggested that cell surface Ca²⁺ receptors may also be present on these cells, imparting to them the ability to detect and to initiate or enable a response to changes in [Ca²⁺].

In parathyroid cells, osteoclasts, parafollicular cells (C-cells), keratinocytes, juxtaglomerular cells and trophoblasts, an increase in [Ca²⁺] evokes an increase in intracellular free Ca²⁺ concentration ("[Ca²⁺]ᵢ") . Such an increase may be caused by influx of extracellular Ca²⁺ or by mobilization of Ca²⁺ from intracellular organelles. Changes in [Ca²⁺]ᵢ are readily monitored and quantitated using fluorimetric indicators such as fura-2 or indo-1 (Molecular Probes, Eugene, OR). Measurement of [Ca²⁺]ᵢ provides an assay to assess the ability of molecules to act as agonists or antagonists at the Ca²⁺ receptor.

In parathyroid cells, increases in the concentration of extracellular Ca²⁺ evoke rapid and transient increases in [Ca²⁺]ᵢ which are followed by lower yet sustained increases in [Ca²⁺]ᵢ. The transient increases in [Ca²⁺]ᵢ arise from the mobilization of intracellular Ca²⁺, whereas the lower, sustained increases result from the influx of extracellular Ca²⁺. The mobilization of intracellular Ca²⁺ is accompanied by increased formation of inositol-1,4,5-trisphosphate (IP₃) and diacylglycerol, two biochemical indicators which are associated with receptor-dependent mobilization of intracellular Ca²⁺ in various other cells.

In addition to Ca²⁺, various other di- and trivalent cations, such as Mg²⁺, Sr²⁺, Ba²⁺, La³⁺, and Gd³⁺ also cause the mobilization of intracellular Ca²⁺ in parathyroid cells. Mg²⁺ and La³⁺ also increase the formation of IP₃; all these inorganic cations depress the secretion of PTH. The postulated Ca²⁺ receptor on the parathyroid cell is therefore promiscuous because it detects a variety of extracellular di- and trivalent cations.

The ability of various compounds to mimic extracellular Ca²⁺ in vitro is discussed by Nemeth et al., (spermine and spermidine) in "Calcium-Binding Proteins in Health and Disease", 1987, Academic Press, Inc., pp. 33-35; Brown et al., (e.g., neomycin) 128 Endocrinology 3047, 1991; Chen et al., (diltiazem and its analog, TA-3090) 5 J. Bone and Mineral Res. 581, 1990; and Zaidi et al., (verapamil) 167 Biochem Biophys Res Comm 807, 1990.

Brown et al., 6 J. Bone and Mineral Res. 11, 1991 discuss the existing theories regarding the effects of Ca²⁺ ions on parathyroid cells, and propose that the results may be explained by both a receptor-like mechanism and a receptor-independent mechanism as follows:
Polyvalent cations [e.g., divalent and trivalent cations] exert a variety of effects on parathyroid function, such as inhibition of parathyroid hormone (PTH) secretion and cAMP accumulation, stimulation of the accumulation of inositol phosphates, and elevation of the cytosolic calcium concentration. These actions are thought to be mediated through a "receptor-like" mechanism. The inhibition of agonist-stimulated cAMP accumulation by divalent and trivalent cations, for example, is blocked following preincubation with pertussis toxin. Thus, the putative polyvalent cation receptor may be coupled to inhibition of adenylate cyclase by the inhibitory guanine nucleotide regulatory (G) protein, Gᵢ.
We recently showed that the polycationic antibiotic, neomycin, mimics the actions of di-and trivalent cations in several aspects of parathyroid function. To determine whether these actions were specific to this agent or represented a more generalized action of polycations, we tested the effects of the highly basic peptides, polyarginine and polylysine, as well as protamine on the same parameters in dispersed bovine parathyroid cells. The results demonstrate that the parathyroid cell responds to a variety of polycations as well as to polyvalent cations, potentially via similar biochemical pathways. These results are discussed in terms of the recently postulated, "receptor-independent" modulation of G proteins by polycations in other systems.
The Ca²⁺ receptor has been presumed to be analogous to other G protein-coupled receptors [e.g., a glycoprotein], but recent studies with other cell types have raised the possibility that polycations can modulate cell function by alternative or additional mechanisms. In mast cells, for example, a variety of amphipathic cations, including mastoparan, a peptide from wasp venom, 48/80, a synthetic polycation, and polylysine, enhance secretion by a pertussis toxin-sensitive mechanism, suggesting the involvement of a G protein. No classic cell surface receptor has been identified that could mediate the actions of these diverse agents. Furthermore, these same compounds have been shown to activate directly purified G proteins in solution or in artificial phospholipid vesicles. On the basis of these observations, it has been proposed that amphipathic cations activate G proteins and, in turn, mast cell secretion by a "receptor-independent" mechanism.
Polycations have also been shown to interact strongly with acidic phospholipids. Polylysines of varying chain lengths (20-1000 amino acids) bind to artificial phospholipid vesicles with dissociation constants in the range of 0.5 nM to 1.5 µM. The binding affinity is directly related to the length of the polylysine chain, with polymers of 1000 amino acids having a K_{d} of 0.5 nM, shorter polymers having higher Kd values, and lysine not interacting to a significant extent. This relationship between potency and chain length is similar to that observed for the effects of polylysine _{10,200}, polylysine ₃₈₀₀, and lysine on parathyroid function.
It is possible that the binding of polycations to biomembranes produces some of their biologic actions. The permeabilization of the plasma membrane induced in some cell types by a variety of pore-forming agents, including polycations, has been postulated to be mediated by their interaction with a phosphatidylserine-like structure. In addition, the "receptor-independent" activation of purified G proteins by amphipathic cations is potentiated when these proteins are incorporated into phospholipid vesicles.
Calcium ions, in the millimolar concentration range, also produce marked changes in membrane structure. In some cases, calcium can either antagonize or potentiate the interaction of polycations with membrane lipids. These considerations raise the possibility that the actions of both polyvalent cations and polycations on parathyroid cells could involve a receptor-independent mechanism not requiring the presence of a classic, cell surface, G protein-coupled receptor. Further studies, however, are required to elucidate the molecular basis for Ca²⁺ sensing by this and other cell types. [Citations omitted.]

Shoback and Chen (6 (Supplement 1), J. Bone and Mineral Res. 1991, S135) and Racke et al. (6 (Supplement 1), J. Bone and Mineral Res. 1991, S118) describe experiments which are said to indicate that a Ca²⁺ receptor or Ca²⁺ sensor is present in parathyroid cells. Messenger RNA isolated from such cells can be expressed in oocytes and caused to provide those oocytes with a phenotype which might be explained by the presence of a Ca²⁺ receptor protein.

### Summary of the Invention

Applicant has demonstrated that Ca²⁺ receptor proteins enable certain specialized cells involved in bodily Ca²⁺ metabolism to detect and respond to changes in the concentration of extracellular Ca²⁺. Although these receptors share certain general characteristics, they can be selectively affected by different pharmacological agents. As detailed below, certain molecules are identified with selective activity on Ca²⁺ receptors at parathyroid cells, osteoclasts, and C-cells.

Ca²⁺ receptors constitute discrete molecular targets for a new class of molecules that mimic ("calcimimetics") or antagonize ("calcilytics") the actions of extracellular Ca²⁺. Such receptors are present on cell surfaces and have a low affinity for extracellular Ca²⁺ (apparent K_{d} generally greater than about 0.5 mM). Such receptors may include a free or bound effector mechanism, as defined by Cooper, Bloom and Roth, "The Biochemical Basis of Neuropharmacology", Ch. 4. Such receptors are thus distinct from intracellular Ca²⁺ receptors, e.g., calmodulin and the troponins. Calcimimetics, for example, act on Ca²⁺ receptors selectively to directly or indirectly depress the function of parathyroid cells or osteoclasts or to stimulate the function of C-cells. Calcimimetics and calcilytics of this invention allow novel therapies for hyperparathyroidism, osteoporosis and other Ca²⁺-related diseases. This application concerns targeting Ca²⁺ receptors on each of these three cell types and other cell types that detect and respond to changes in [Ca²⁺].

Applicant is the first to demonstrate a Ca²⁺ receptor protein in parathyroid cells, and to pharmacologically differentiate such Ca²⁺ receptors in other cells, such as C-cells and osteoclasts. Applicant is also the first to describe methods by which molecules active at these Ca²⁺ receptors can be identified and used as lead molecules in the discovery, development, design, modification and/or construction of useful calcimimetics or calcilytics which are active at Ca²⁺ receptors. Such calcimimetics or calcilytics are useful in the treatment of various disease states characterized by abnormal levels of one or more components, e.g., polypeptides such as hormones, enzymes or growth factors, the expression and/or secretion of which is regulated or affected by activity at one or more Ca²⁺ receptors. Further, the identification of different Ca²⁺ receptors in different cell types, and the specific response of such receptors to different lead molecules allows design and construction of specific molecules active in treatment of specific diseases which can be affected by action at such specific Ca²⁺ receptors. For example, abnormal levels of parathyroid hormone secretion can be affected by such specific molecules without affecting the level of secretion of other Ca²⁺ regulated hormones and the like.

Identification of such lead molecules was impeded by the prior lack of a high-throughput screening system to discover active molecules, and the absence of a structural data base upon which to design effective drug candidates. These barriers are now removed by cloning the parathyroid cell Ca²⁺ receptor and functionally related receptors, and systematically examining the structural features of certain lead molecules that activate such cloned Ca²⁺ receptors and functionally related receptors. Cloning of the Ca²⁺ receptor also enables development of transfected cell lines suitable for high-throughput screening of natural product or molecule libraries and synthetic molecules. This, together with structure-activity studies discussed below, provides the technology necessary to develop novel calcimimetics and calcilytics.

Applicant enables such procedures in this application. For example, the human parathyroid cell Ca²⁺ receptor cDNA can be cloned by screening for functional expression in Xenopnus oocytes, and the structural features of organic molecules necessary for activity on the Ca²⁺ receptor can be determined through the testing of selected natural products or other molecule libraries and subsequent structure-activity studies.

Thus, in a first aspect, the invention features a pharmaceutical composition including a molecule which either mimics the activity of extracellular Ca²⁺ by evoking an increase in [Ca²⁺], in a cell, or blocks an increase in [Ca²⁺]ᵢ elicited by extracellular Ca²⁺. The molecule has an EC₅₀ of less than or equal to 5 µM, and is not protamine.

By "mimic" is meant that the molecule has one or more of the specific actions of extracellular Ca²⁺ on an extracellular Ca²⁺ responsive cell. The term does not require that all of the biological functions of extracellular Ca²⁺ are mimicked, but rather than at least one such function is mimicked. In addition it does not require that the molecule bind to the same site on the receptor as does extracellular Ca²⁺ receptor (see for example, the novel compound NPS 467 and its action in Example 20 below). By "block" is meant that one such action of Ca²⁺ is reduced or prevented by the molecule. The EC₅₀ can be determined in assays as described below, where the activity mimicked is measured and the concentration of molecule which mimics at half the maximum mimicking effect is the EC₅₀. Conversely, the IC₅₀ of a calcilytic is that amount which blocks half maximal activity. Preferably, such assays measure [Ca²⁺]ᵢ increases and are confirmed to be specific to a Ca²⁺ receptor by methods described below, or their equivalent.

In preferred embodiments, bioassays described herein demonstrated that the increase in [Ca²⁺]ᵢ in a cell is transient, having a duration of less than one minute, and the increase in [Ca²⁺]ᵢ is rapid, occurring within thirty seconds; and the molecule also (a) evokes a sustained increase (greater than thirty seconds) in [Ca²⁺]ᵢ, (b) evokes an increase in inositol-1,4,5-trisphosphate and/or diacylglycerol levels, e.g., within less than 60 seconds, and (c) inhibits dopamine- or isoproterenol-stimulated cyclic AMP formation. In addition, the transient increase in [Ca²⁺]ᵢ is abolished by pretreatment of the cell for ten minutes with 10 mM sodium fluoride, or the transient increase is diminished by brief pretreatment (not more than ten minutes) of the cell with an activator of protein kinase C, e.g., phorbol myristate acetate (PMA), mezerein or (-)indolactam V.

In a parathyroid cell, those molecules which are active in all of the assays described above are particularly useful in this invention since they are specific in their actions to a Ca²⁺ receptor of such a cell. This is particularly true for the PMA pretreatment effect described above.

In a more preferred embodiment, the cell is a parathyroid cell, and the molecule inhibits parathyroid hormone secretion from the cell; and the molecule elicits an increase in Cl⁻ conductance in a Xenopus oocyte injected with mRNA from a parathyroid cell, bone osteoclast, juxtaglomerular kidney cell, proximal tubule kidney cell, keratinocyte, parafollicular thyroid cell or placental trophoblast.

In other preferred embodiments, the molecule evokes the mobilization of intracellular Ca²⁺ to cause the increase in [Ca²⁺]ᵢ; the cell is a C-cell or an osteoclast and the molecule inhibits bone resorption in vivo; the cell is an osteoclast and the molecule inhibits bone resorption in vitro; or the cell is a C-cell and the molecule stimulates calcitonin secretion in vitro or in vivo; and most preferably the molecule is either a calcimimetic or calcilytic having an EC₅₀ or IC₅₀ at a Ca²⁺ receptor of less than or equal to 5 µM, and even more preferably less than or equal to 1 µM, 100 nmolar, 10 nmolar, or 1 nmolar. Such lower EC₅₀'s or IC₅₀'s are advantageous since they allow lower concentrations of molecules to be used in vivo or in vitro for therapy or diagnosis. The discovery of molecules with such low EC₅₀'s and IC₅₀'s enables the design and synthesis of similarly potent and efficacious molecules.

By "calcimimetic" molecule is meant any molecule which has one or more activities of extracellular Ca²⁺, and preferably mimics the activity of Ca²⁺ at a Ca²⁺ receptor. For example, when used in reference to a parathyroid cell it is a molecule, which when tested on parathyroid cells, in vitro, possesses one or more, and preferably all of the following characteristics as measured by techniques well known to those in the art:
1. The molecule causes a rapid (time to peak < 5 sec) and transient increase in [Ca²⁺]ᵢ that is refractory to inhibition by 1 µM La³⁺ or Gd³⁺. The increase in [Ca²⁺]ᵢ persists in the absence of extracellular Ca²⁺ but is abolished by pretreatment with ionomycin (in the absence of extracellular Ca²⁺);
2. The increase in [Ca²⁺]ᵢ elicited by extracellular Ca²⁺ is not inhibited by dihydropyridines.
3. The transient increase in [Ca²⁺]ᵢ caused by the molecule is abolished by pretreatment for 10 min. with 10 mM sodium fluoride;
4. The transient increase in [Ca²⁺]ᵢ caused by the molecule is diminished by pretreatment with an activator of protein kinase C (PKC), such as phorbol myristate acetate (PMA), mezerein or (-)-indolactam V. The overall effect of the protein kinase C activator is to shift the concentration-response curve of the molecule to the right without affecting the maximal response;
5. The molecule causes a rapid (< 30 sec.) increase in the formation of inositol-1,4,5-trisphosphate and or diacylglycerol;
6. The molecule inhibits dopamine- or isoproterenol-stimulated cyclic AMP formation;
7. The molecule inhibits PTH secretion;
8. Pretreatment with pertussis toxin (100 ng/ml for > 4 hrs.) blocks the inhibitory affect of the molecule on cyclic AMP formation but does not effect increases in [Ca²⁺]ᵢ, inositol-1,4,5-trisphosphate, or diacylglycerol, nor decreases in PTH secretion;
9. The molecule elicits increases in Cl⁻ conductance in Xenopus oocytes injected with poly(A)⁺- enriched mRNA from bovine or human parathyroid cells but is without effect in Xenopus oocytes injected with water or rat brain or liver mRNA; and
10. Similarly, using a cloned receptor from parathyroid cells, the molecule will elicit a response in Xenopus oocytes injected with the specific cDNA or mRNA encoding the receptor.

By "calcilytic" molecule is meant any molecule which blocks one or more of the activities of extracellular Ca²⁺ on an extracellular Ca²⁺-sensing cell, preferably by acting as an antagonist at the Ca²⁺ receptor. For example, when used in reference to a parathyroid cell, it is a molecule which, when tested on parathyroid cells in vitro, possesses one or more, and preferably all of the following characteristics as measured by techniques well known to those in the art:
1. The molecule blocks, either partially or completely, the ability of increased concentrations of extracellular Ca²⁺ to:
   a) increase [Ca²⁺]ᵢ,
   b) mobilize intracellular Ca²⁺,
   c) increase the formation of inositol-1,4,5-trisphosphate,
   d) decrease dopamine- or isoproterenol-stimulated cyclic AMP formation, and
   e) inhibit PTH secretion;
2. At low [Ca²⁺], i.e., 0.5 mM, the molecule by itself does not change [Ca²⁺]ᵢ;
3. The molecule blocks increases in Cl⁻ conductance in Xenopus oocytes injected with poly (A)⁺-mRNA from bovine or human parathyroid cells elicited by extracellular Ca²⁺ or calcimimetic compounds but not in Xenopus oocytes injected with water or rat brain or liver mRNA;
4. Similarly, using a cloned receptor from parathyroid cells, the molecule will block a response in Xenopus oocytes injected with the specific cDNA or mRNA encoding the Ca²⁺ receptor, elicited by extracellular Ca²⁺ or a calcimimetic compound.

Parallel definitions of useful calcimimetics and calcilytics at Ca²⁺ receptors on other cell types are evident from the examples provided below.

The Ca²⁺ receptor is able to detect and respond to certain inorganic polycations and polycationic organic molecules. For example, the parathyroid cell is unable to distinguish increases in extracellular Ca²⁺ concentration from the addition of these organic polycations, presumably because these organic molecules act just like extracellular Ca²⁺ at the Ca²⁺ receptor. The calcimimetic molecules of this invention are particularly good agonists of the Ca²⁺ receptor and may be used as drugs that alter selected cellular functions, e.g., secretion of PTH from parathyroid cells. Unlike Ca²⁺ most of these molecules act only at one or more, but not all Ca²⁺ receptors, and thus provide an ability to specifically target one Ca²⁺ receptor.

These molecules also provide lead structures for the development of further novel therapeutics effective in the treatment of various diseases where [Ca²⁺]ᵢ and [Ca²⁺] play a role, such as hyperparathyroidism, osteoporosis, Paget's disease, hypertension, renal disease, and cancer.

The calcimimetics and calcilytics can be formulated as pharmaceutical compositions which are useful for regulating the level of extracellular free Ca²⁺ in a patient and for mimicking the effect of extracellular Ca²⁺ on a cell selected from the group described above, by administering to the patient such a pharmaceutical composition. Prior to this invention, applicant was unaware of any such molecules acting on the Ca²⁺ receptor useful in treatment of diseases caused by irregularity in operation or regulation of a Ca²⁺ receptor or diseases in an animal having normal Ca²⁺ receptors but which can be treated by activating or deactivating such Ca²⁺ receptors.

In yet another preferred embodiment, the molecule has an EC₅₀ less than or equal to 5 µM at one or more but not all cells chosen from the group consisting of parathyroid cells, bone osteoclasts, juxtaglomerular kidney cells, proximal tubule kidney cells, keratinocytes, parafollicular thyroid cells (C-cells) and placental trophoblasts.

It is the specificity of action of such molecules that is particularly advantageous in this invention since it allows specific in vivo and in vitro therapy and diagnosis and discovery of additional calcimimetic or calcilytic molecules.

In specific preferred embodiments, the molecule is positively charged at physiological pH, and is selected from the group consisting of branched or cyclic polyamines, positively charged polyamino acids, and arylalkylamines, e.g., the branched polyamine has the formula H₂N-(CH₂)ⱼ-(NRᵢ-(CH₂)ⱼ)ₖ-NH₂ where k is an integer from 1 to 10, each j is the same or different and is an integer from 2 to 20, and each Rᵢ is the same or different and is selected from the group consisting of hydrogen and -(CH2)ⱼ-NH₂, where j is as defined above, and at least one Rᵢ is not hydrogen.

In an alternative embodiment, the molecule has the formula 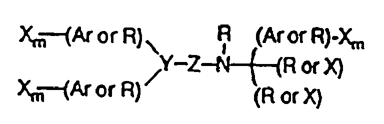 where each X independently is selected from the group consisting of H, CH₃, CH₃O, CH₃CH₂O, Br, Cl, F, CF₃, CHF₂, CH₂F, CF₃O, CH₃S, OH, CH₂OH, CONH₂, CN, NO₂, and CH₃CH₂; Ar is a hydrophobic entity; each R independently is selected from the group consisting of hydrogen, methyl, ethyl, propyl, isopropyl, butyl, isobutyl, cyclopentyl, cyclohexyl, cycloheptyl, cyclooctyl, indenyl, indanyl, dihydroindolyl, thiodihydroindolyl, 2-, 3-, or 4-piperid(in)yl; Y is selected from the group consisting of CH, nitrogen and an unsaturated carbon; Z is selected from the group consisting of oxygen, nitrogen, sulfur, 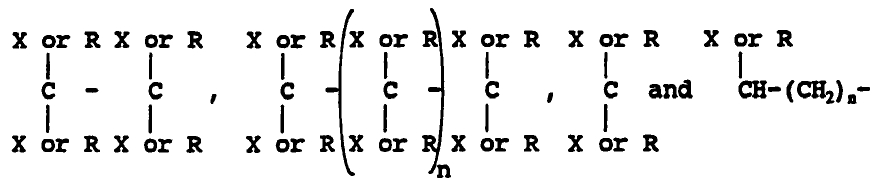 where each n is independently between 1 and 4 inclusive, and each m is independently between 0 and 5 inclusive. Most preferably the molecule is either a calcimimetic or calcilytic.

In preferred embodiments, the hydrophobic entity is selected from the group consisting of phenyl, 2-, 3-, or 4-pyridyl, 1- or 2-naphthyl, 1- or 2-quinolinyl, 2- or 3-indolyl, benzyl, and phenoxy; the molecule is an R-diphenylpropyl-α-phenethylamine derivative, and the molecule has the formula: 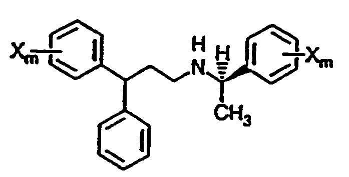 with each X preferably being independently selected from the group consisting of Cl, F, CF₃, CH₃, and CH₃O.

According to a preferred aspect of the present invention, novel phenyl-α-phenethylamine analogs and derivatives are provided having the formula: 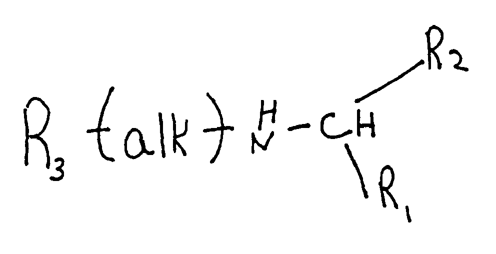 wherein alk is straight or branched chain alkylene of from 1 to 6 carbon atoms; R₁ is lower alkyl of from 1 to 3 carbon atoms or lower haloalkyl of from 1 to 3 carbon atoms substituted with from 1 to 7 halogen atoms; R₂ and R₃ are independently selected carbocyclic aryl or cycloalkyl groups, either monocyclic or bicyclic, having 5- or 6-membered rings optionally substituted with 1 to 5 substituents independently selected from lower alkyl of 1 to 3 carbon atoms, lower haloalkyl of 1 to 3 carbon atoms substituted with 1 to 7 halogen atoms, lower alkoxy of 1 to 3 carbon atoms, halogen, nitro, amino, alkylamino, amido, lower alkylamido of 1 to 3 carbon atoms, cyano, hydroxy, acyl of 2 to 4 carbon atoms lower hydroxyalkyl of 1 to 3 carbon atoms or lower thioalkyl of 1 to 3 carbon atoms. Suitable carbocyclic aryl groups are groups having one or two rings, at least one of which having aromatic character and include carbocyclic aryl groups such as phenyl and bicyclic carbocyclic aryl groups such as naphthyl. As is apparent from the above formula, the compounds encompassed therein may exist as racemic mixtures and as individual stereoisomers. Especially preferred are R-phenylpropyl α-phenethylamine derivatives which are believed to exhibit enhanced activity in lowering serum ionized calcium.

Preferred compounds include those where alk is n-propylene. Also preferred are compounds where R₁ is methyl. Also preferred are those compounds where R₂ and R₃ are optionally substituted phenyl.

Especially preferred compounds include those where R₂ is monosubstituted phenyl, more preferably meta-substituted. Especially preferred R₃ groups include unsubstituted or monosubstituted phenyl, especially ortho-substituted. Preferred substitutents for R₂ include halogen, haloalkyl, preferably trihalomethyl, and alkoxy, preferably methoxy. Preferred substituents for R₃ include halogen.

In a second related aspect, the invention features a method for treating a patient having a disease or condition characterized by an abnormal [Ca²⁴] or [Ca²⁺]ᵢ in one or more cells or in the blood or plasma or extracellular fluids. The method includes the step of administering to the patient a therapeutically effective amount of a molecule which either mimics the activity of extracellular Ca²⁺ by evoking an increase in [Ca²⁺]ᵢ in a cell or blocks an increase in [Ca²+]ᵢ elicited by extracellular Ca²⁺.

By "abnormal" is meant that the patient, compared to the general population, has a different Ca²⁺ metabolism that is affected by one or more proteins (e.g., hormones) in the blood or extracellular body fluids, or other molecules which affect the level of extracellular and/or intracellular Ca²⁺. Thus, the diseases include hyperparathyroidism, osteoporosis and other bone and mineral-related disorders, and the like (as described, e.g., in standard medical text books, such as "Harrison's Principles of Internal Medicine"). Such diseases are treated in this invention by molecules which mimic or block one or more of the effects of Ca²⁺ and thereby directly or indirectly affect the levels of the proteins or other molecules in the body of the patient.

By "therapeutically effective amount" is meant an amount that relieves to some extent one or more symptoms of the disease or condition in the patient. Additionally, by "therapeutically effective amount" is meant an amount that returns to normal, either partially or completely, physiological or biochemical parameters associated with or causative of the disease or condition. Generally, it is an amount between about 1 nmole and 1 µmole of the molecule, dependent on its EC₅₀ and on the age, size, and disease associated with the patient.

In preferred embodiments, the molecule has an EC₅₀ of less than or equal to 5 µM, and is not protamine; and most preferably interacts at a ca²⁺ receptor as a calcimimetic or calcilytic. Most preferably the molecule is chosen from one of those described above.

In other preferred embodiments, the patient has a disease characterized by an abnormal level of one or more components the level of which is regulated or affected by activity of one or more Ca²⁺ receptors, and the molecule is active on a Ca²⁺ receptor of a cell selected from the group consisting of parathyroid cells, bone osteoclasts, juxtaglomerular kidney cells, proximal tubule kidney cells, keratinocytes, parafollicular thyroid cells, and placental throphoblasts.

In still other preferred embodiments, the molecule reduces the level of parathyroid hormone in the serum of the patient, e.g., to that level present in a normal individual, or to a degree sufficient to cause a decrease in plasma Ca²⁺; and the molecule is provided in an amount sufficient to have a therapeutically relevant effect on the patient.

In a third aspect, the invention features a method for diagnosis of a disease or condition in a patient by identifying the number and/or location (and/or functional integrity) of one or more Ca²⁺ receptors within the patient and comparing that number and/or location (and/or functional integrity) with that observed in normal patients as an indication of the presence of the disease or condition.

In preferred embodiments, the method is an immunoassay in which an antibody to a Ca²⁺ receptor is used to identify the number and/or location and/or functional integrity of the Ca²⁺ receptors, or the assay involves providing a labelled calcimimetic or calcilytic molecule which binds to a Ca²⁺ receptor; and the disease diagnosed is a cancer, e.g., an ectopic tumor of the parathyroid, or a condition characterized by an above normal level in the number of osteoclasts in bone or an increased level of activity of osteoclasts in bone.

In a fourth aspect, the invention features a method for identifying a molecule useful as a therapeutic molecule. The method includes screening a potentially useful molecule for either an ability to mimic the activity of extracellular Ca²⁺ in a cell, or to block an increase in [Ca²⁺]ᵢ elicited by extracellular Ca²⁺, and determining whether the molecule has an EC₅₀ or IC₅₀ of less than or equal to 5 µM.

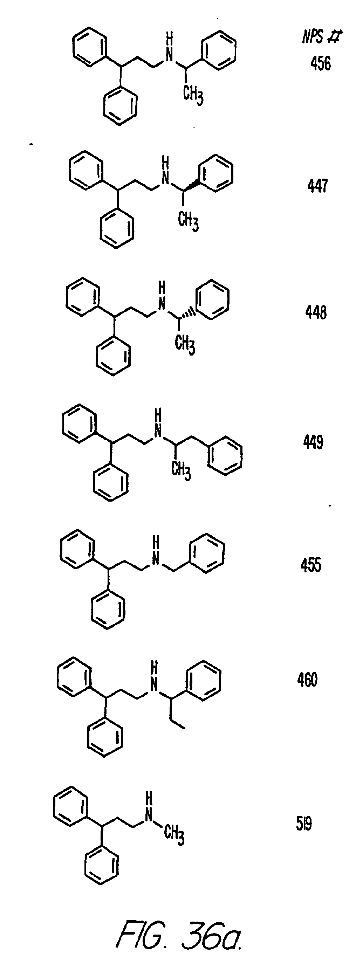

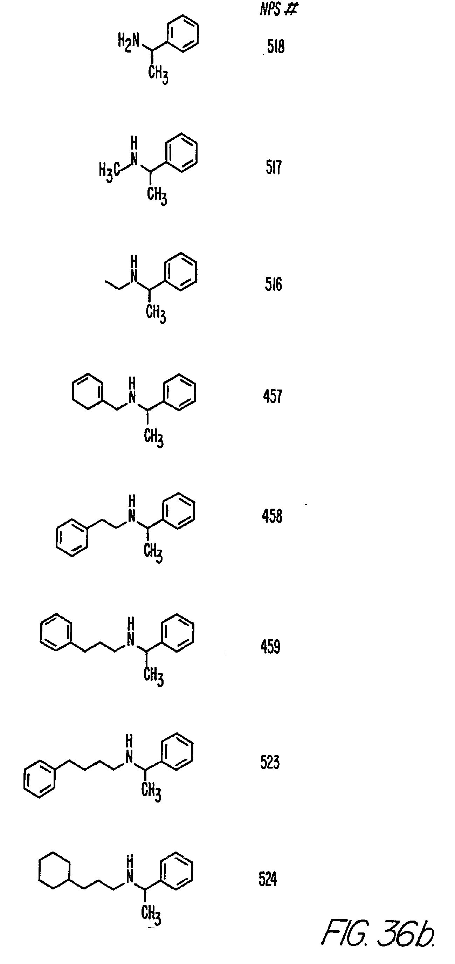

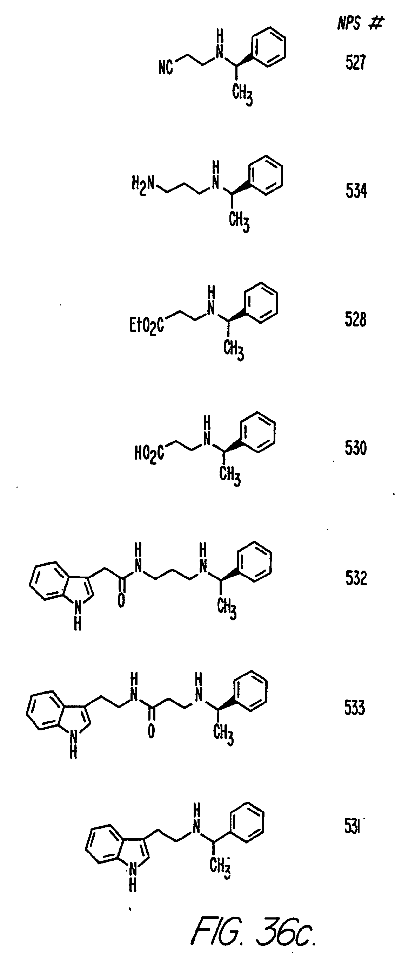

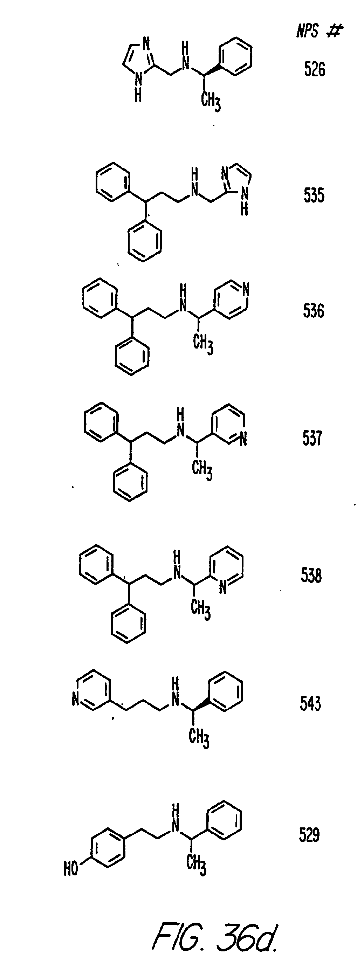

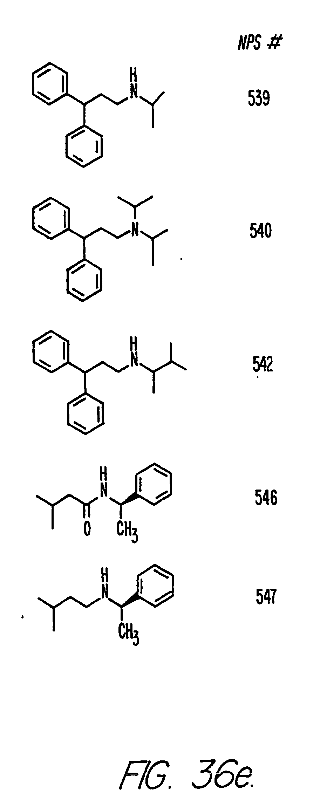

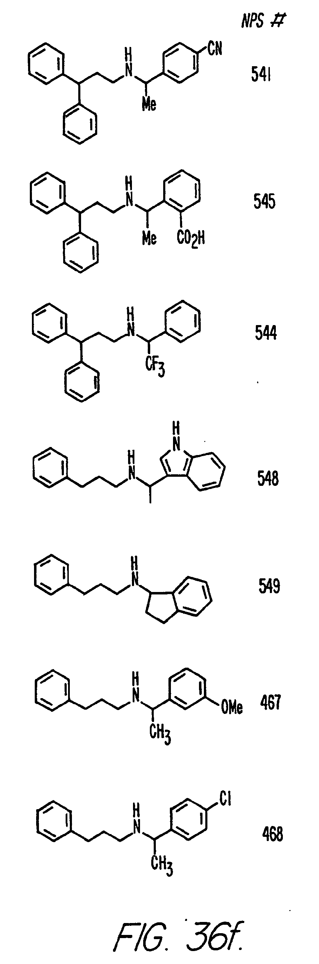

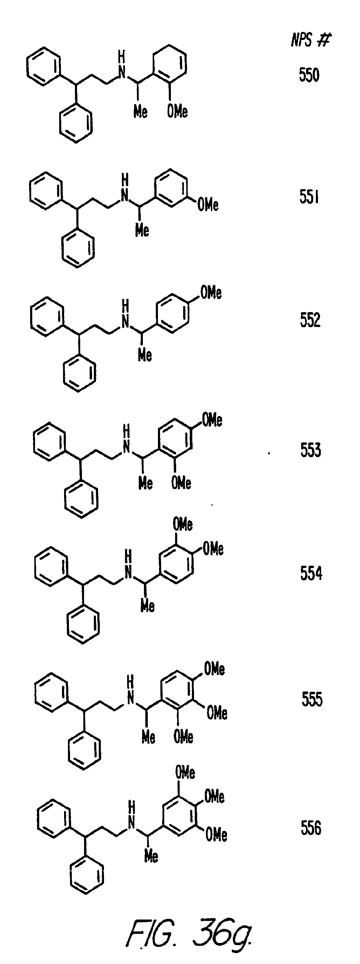

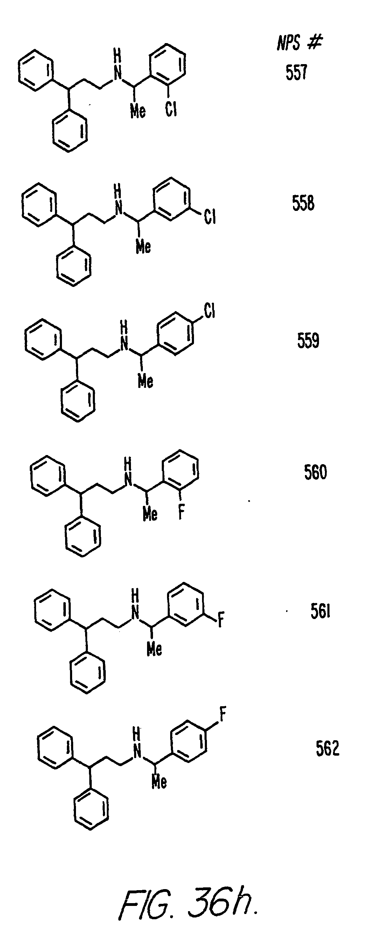

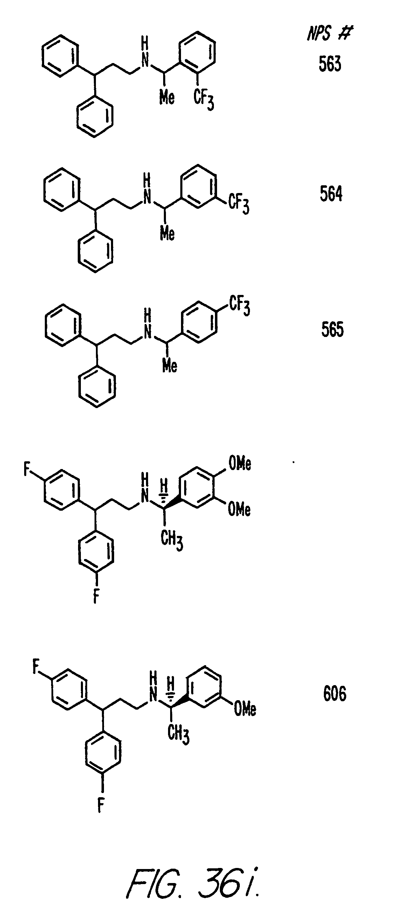

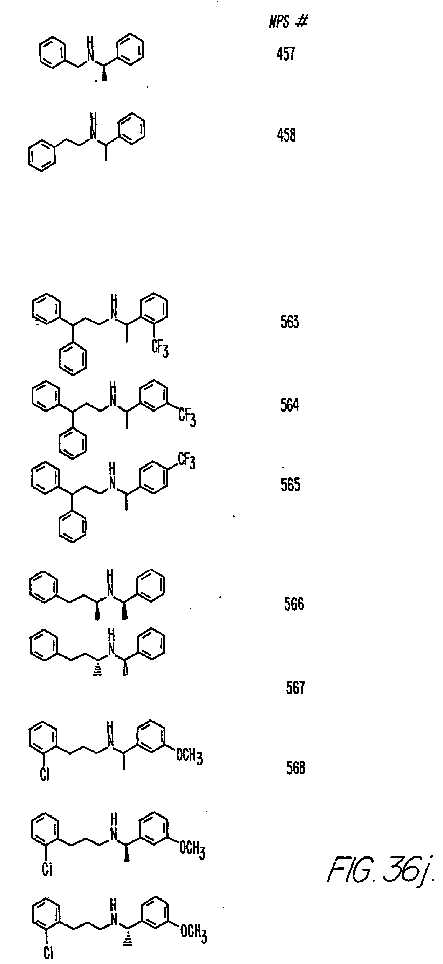

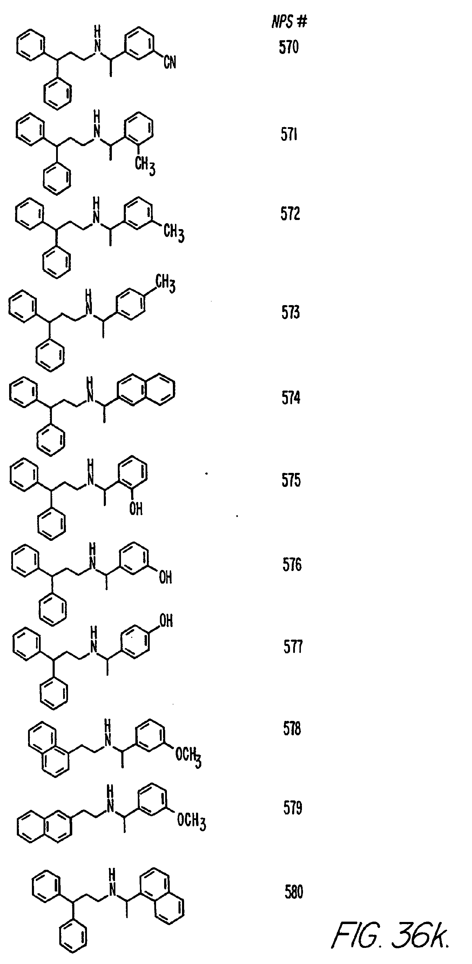

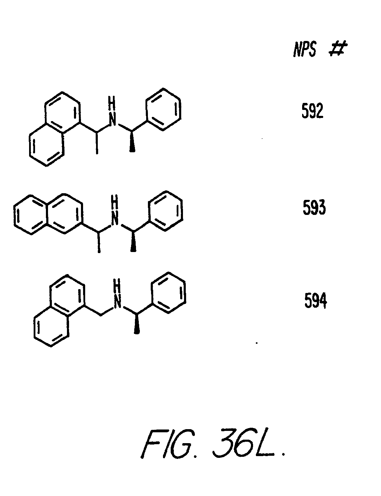

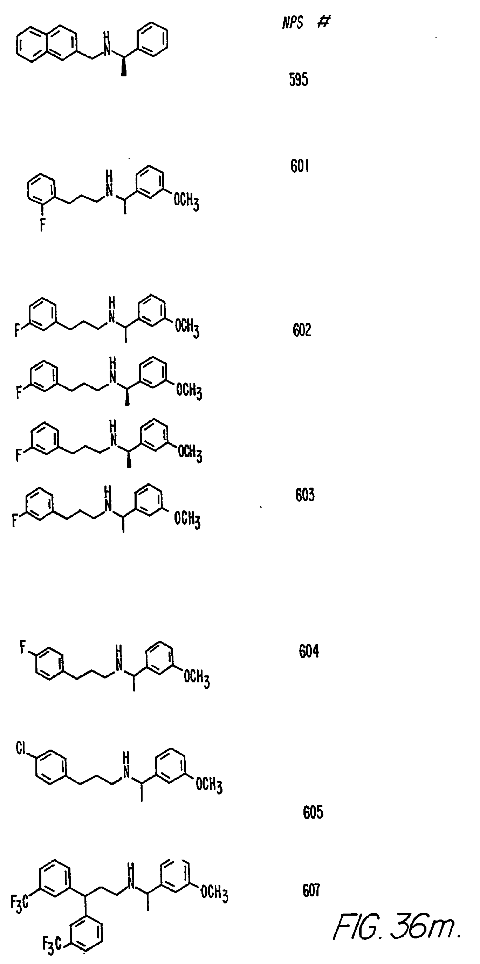

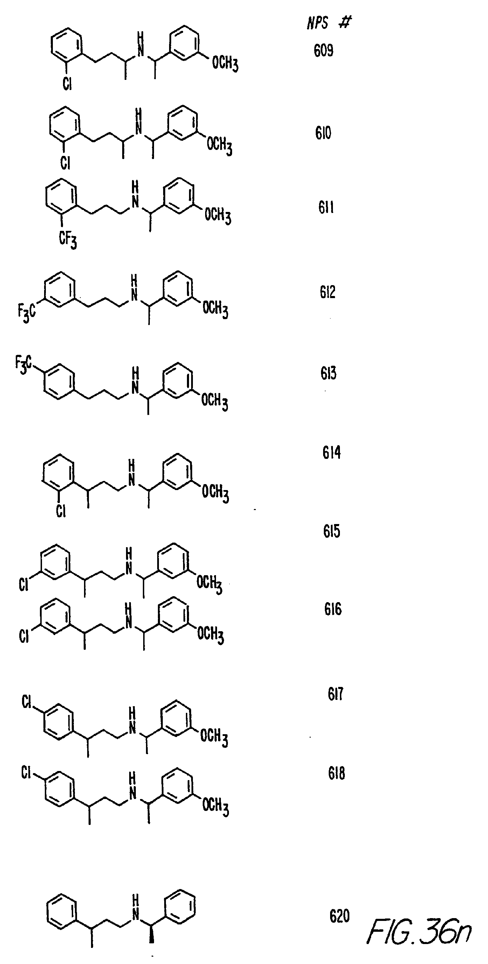

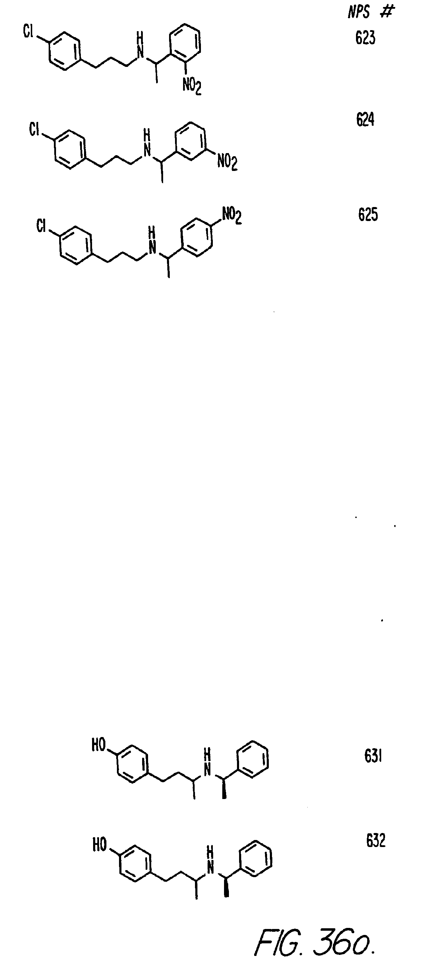

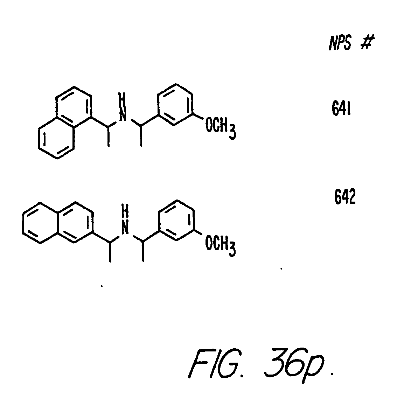

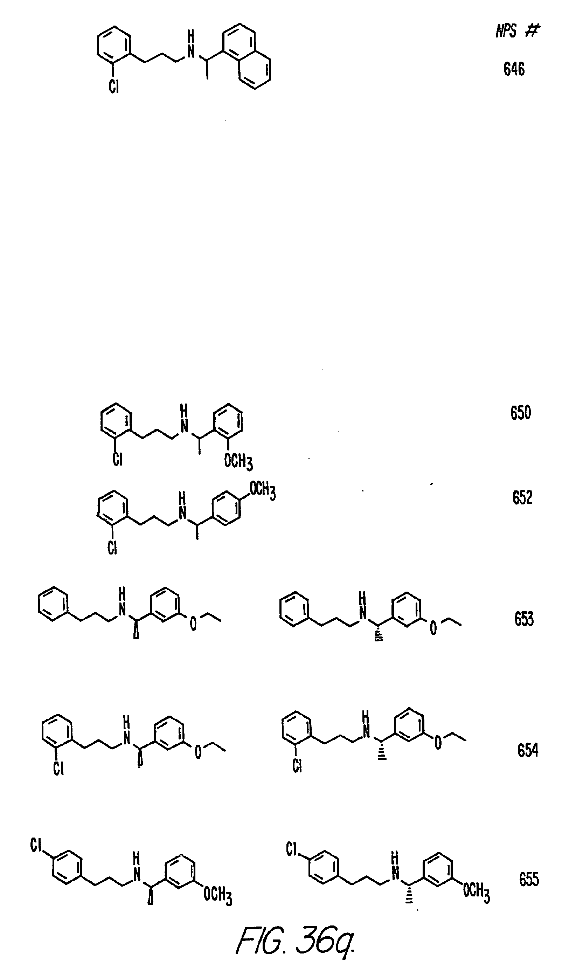

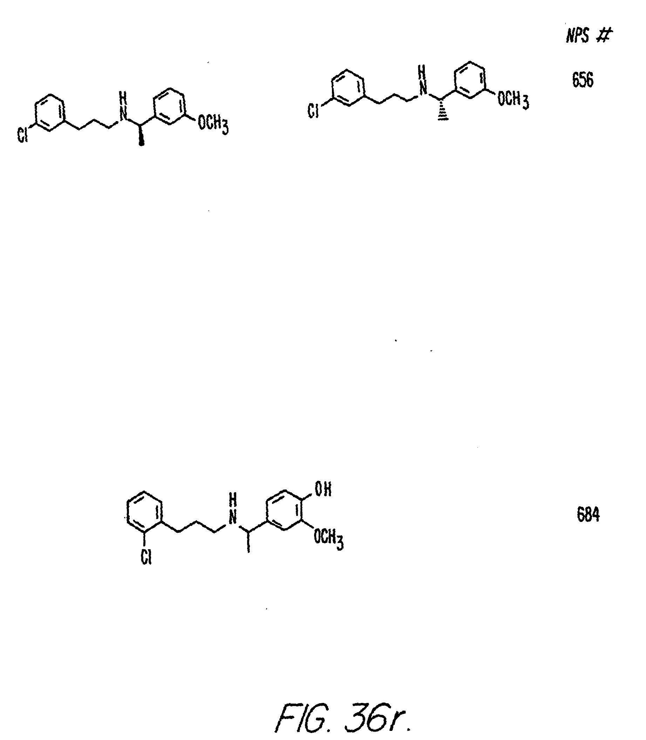

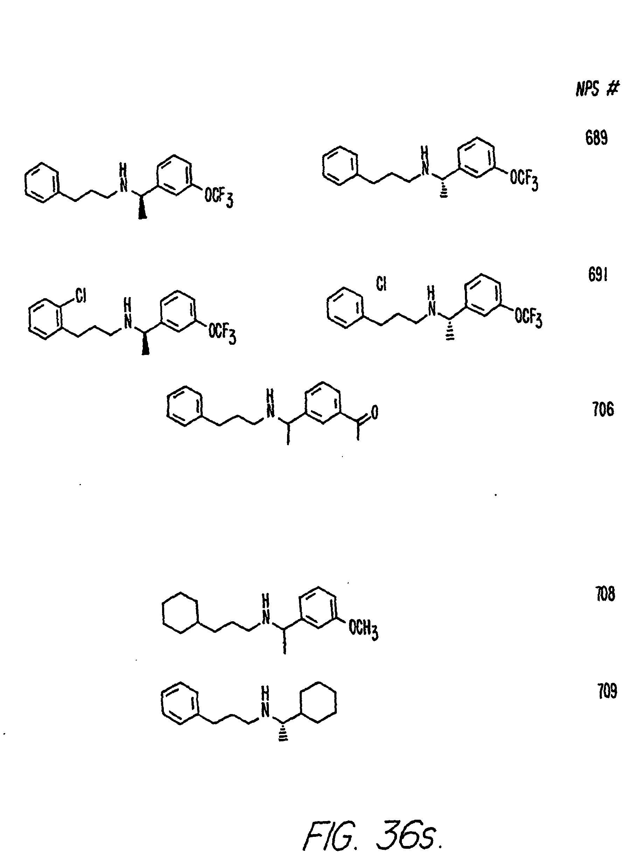

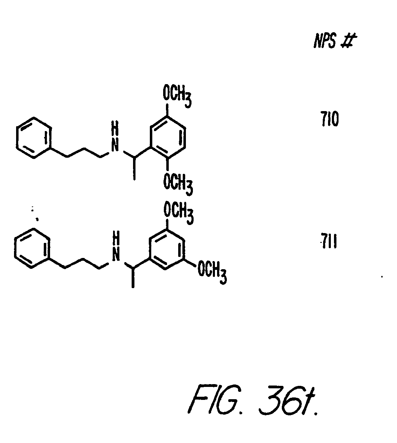

In other aspects, the invention features a recombinant Ca²⁺ receptor, a cell including a recombinant Ca²⁺ receptor, purified nucleic acid encoding a Ca²⁺ receptor, the biological activity and use of the molecule NPS 019, the novel compounds or compositions of matter of NPS 459, NPS 467, NPS 551, and NPS 568 (see Fig. 36) and a method for identifying a useful calcimimetic or calcilytic molecule by identifying a molecule which mimics or blocks one or more activities of Ca²⁺ at a first Ca²⁺ receptor but not at a second Ca²⁺ receptor, e.g., by use of a recombinant Ca²⁺ receptor.

By "recombinant" is meant to include any Ca²⁺ receptor produced by recombinant DNA techniques such that it is distinct from the naturally occurring Ca²⁺ receptor either in its location, purity or structure. Generally, such a receptor will be present in a cell in an amount different from those normally observed in nature.

By "purified" is meant that the antibody or nucleic acid is distinct from naturally occurring antibody or nucleic acid, being separated from antibody or nucleicacid with which it naturally occurs, e.g., in a vector system, such that it can be used to express recombinant Ca²⁺ receptor. Preferably, the antibody or nucleic acid is provided as a homogeneous preparation by standard techniques.

Such cloned receptors can be expressed in a desired cell, and isolated and crystallized to allow structure determination. Such a structure will allow design of useful molecules of this invention which can bind to the Ca²⁺ receptor. In addition, equivalent such receptors can be cloned using a first clone as a probe for clones in other cell, cDNA or genomic libraries.

Antibodies to the cloned receptor can be isolated and used as therapeutics in this invention, or as diagnostic tools for determining Ca²⁺ receptor numbers and/or locations and/or functional integrity to diagnose Ca²⁺-related diseases or conditions. Such antibodies can also be used in vivo by intravenous administration as calcimimetics or calcilytics.

Thus, in general, the invention features calcimimetic or calcilytic molecules able to act as either selective agonists or antagonists respectively at a Ca²⁺ receptor of one or more but not all cells chosen from the group consisting of parathyroid cells, bone osteoclasts, juxtaglomerular kidney cells, proximal tubule kidney cells, keratinocytes, parafollicular thyroid cells and placental throphoblasts. Such a composition may include any pharmaceutically acceptable carrier known to those in the art to provide a pharmaceutical composition.

The invention also features modulation of the the number of Ca²⁺ receptors in a patient by standard techniques, e.g., antisense and related technologies (e.g., ribozymes), as a therapeutic for a disease state.

This invention provides methods for identifying molecules which affect the activity of a Ca²⁺ receptor using assays, as defined below, to detect calcimimetics and/or calcilytics. Further, molecules found to be effective to reduce or enhance expression of Ca²⁺ receptor at a transcriptional or translational level by use of the assays or antibodies or other techniques described below can be defined for therapeutic uses.

Other features and advantages of the invention will be apparent from the following description of the preferred embodiments thereof, and from the claims.

### Description of the Preferred Embodiments

The drawings will first briefly be described.

### Drawings

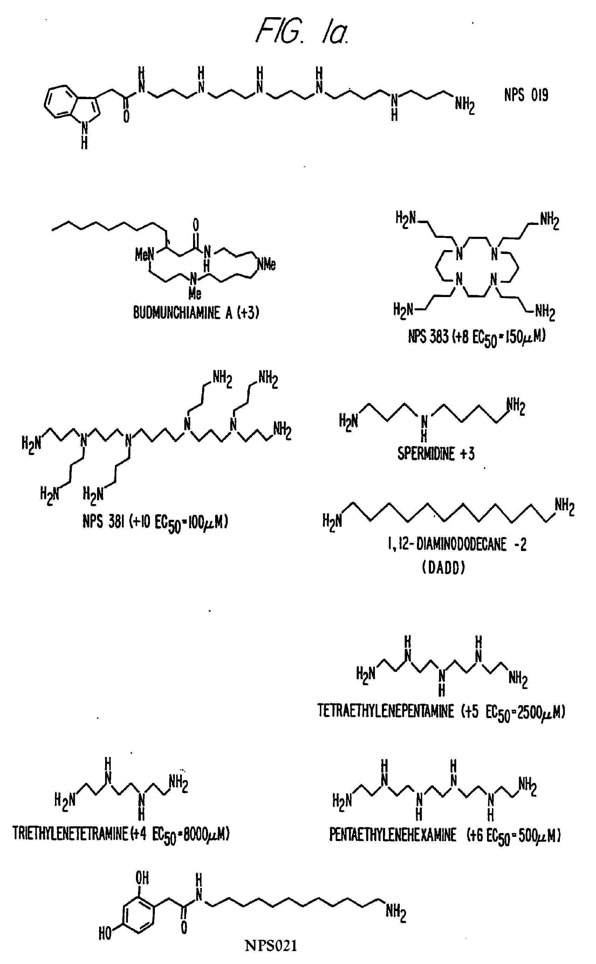

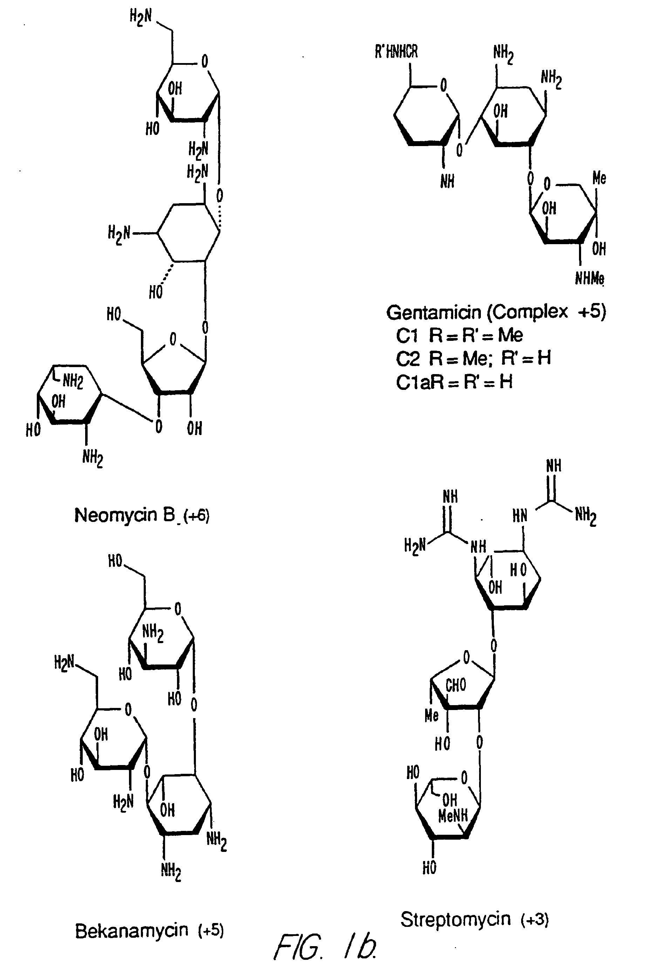

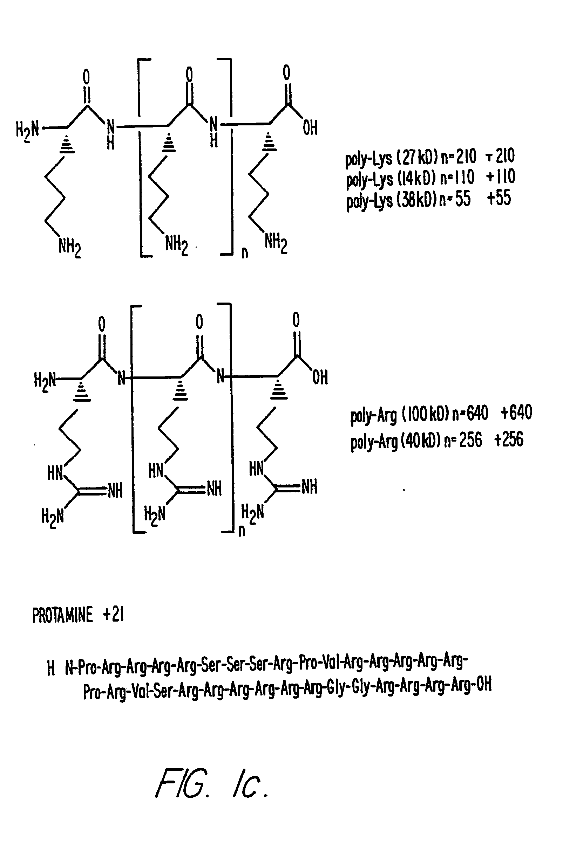

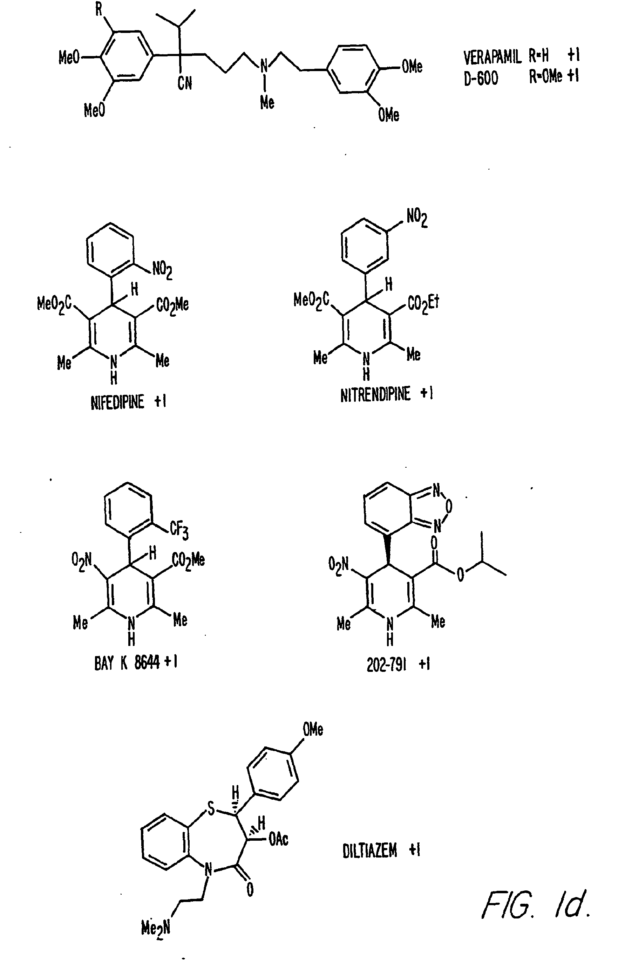

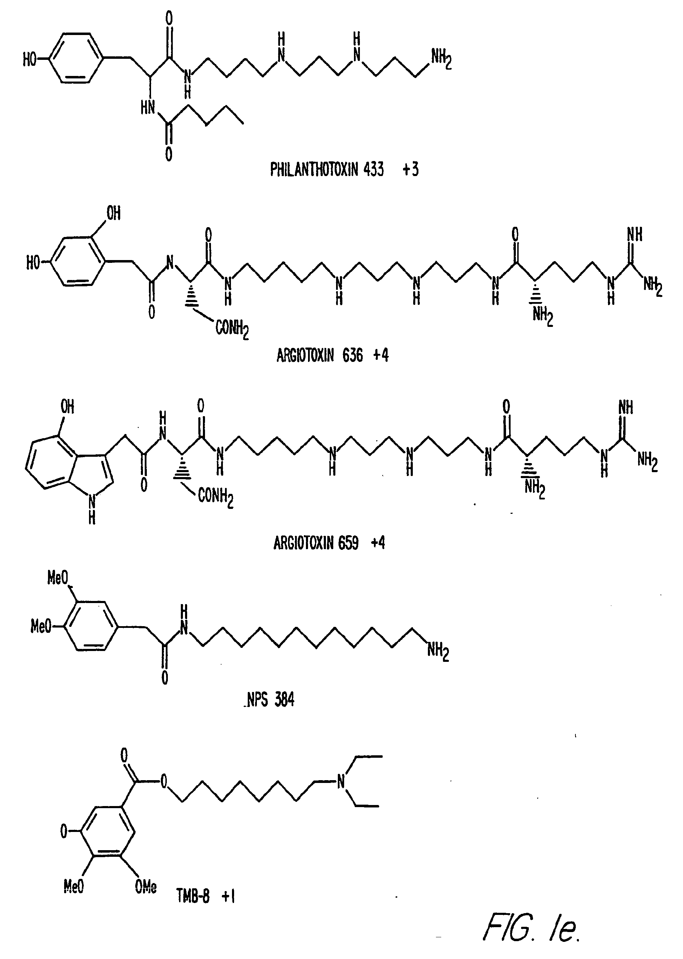

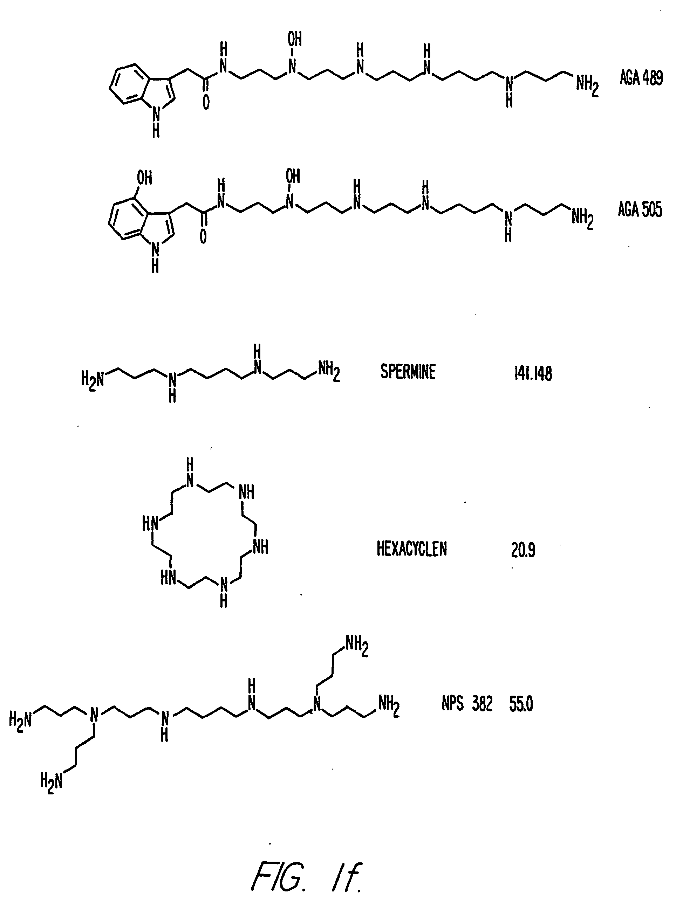

Fig. 1 depicts representative molecules useful in the invention.

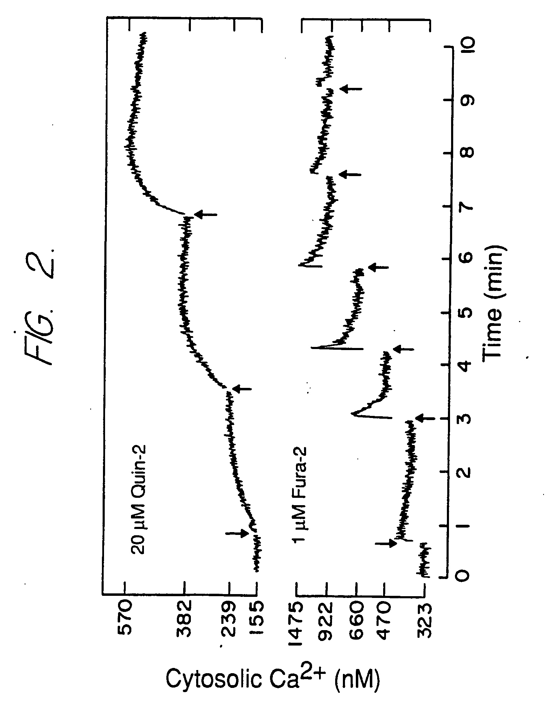

Fig. 2 is a graphical representation showing increases in [Ca²⁺]ᵢ induced by extracellular Ca²⁺ in quin-2- or fura-2-loaded bovine parathyroid cells. The initial [Ca²⁺] was 0.5 mM (using CaCl₂) and, at each of the arrows, was increased in 0.5 mM increments.

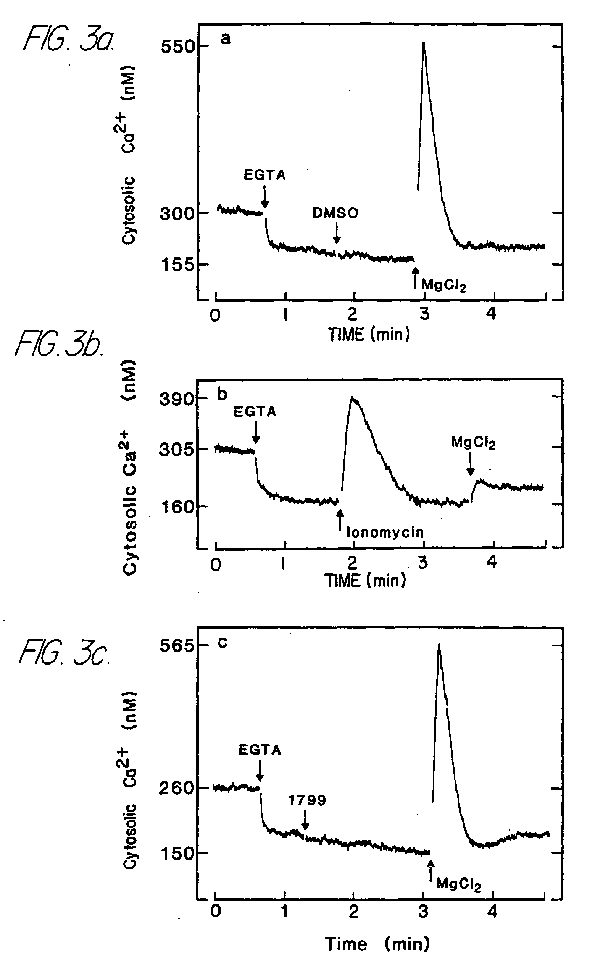

Fig. 3 is a graphical representation showing mobilization of [Ca²⁺]ᵢ in bovine parathyroid cells. The initial [Ca²⁺] was 0.5 mM and was decreased to < 1 µM by the addition of EGTA as indicated. (a) Extracellular Mg²⁺ (8 mM, final) elicits an increase in [ca²⁺]ᵢ in the absence of extracellular Ca²⁺. (b) Pretreatment with ionomycin (1 µM) blocks the response to Mg²⁺. (c) Pretreatment with 5 µM molecule 1799 (a mitochondrial uncoupler) is without effect on the response to Mg²⁺.

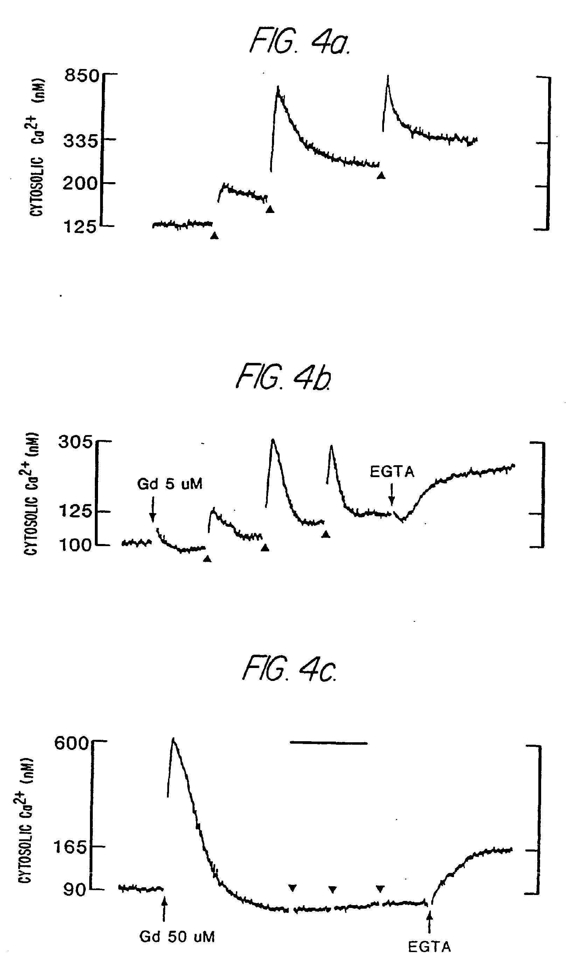

Fig. 4 is a graphical representation showing preferential inhibitory effects of a low concentration of Gd³⁺ on steady=state increases in [Ca²⁺]ᵢ and that a high concentration of Gd³⁺ elicits a transient increase in [Ca]ᵢ. Top panel: Control. Initial concentration of extracellular Ca²⁺ was 0.5 mM and was increased by 0.5 mM at each of the arrowheads. Middle panel: Gd³⁺ (5 *µ*M) blocks steady-state but not transient increases in [Ca²⁺]ᵢ elicited by extracellular Ca²⁺. Lower panel: Gd³⁺ (50 *µ*M) elicits a tran-sient increase in [Ca²⁺]ᵢ and abolishes both transient and sustained responses to extracellular Ca²⁺. In the middle and lower panels, just enough EGTA was added to chelate preferentially Gd³⁺: the block of Ca²⁺ influx is removed and [Ca²⁺]ᵢ rises promptly.

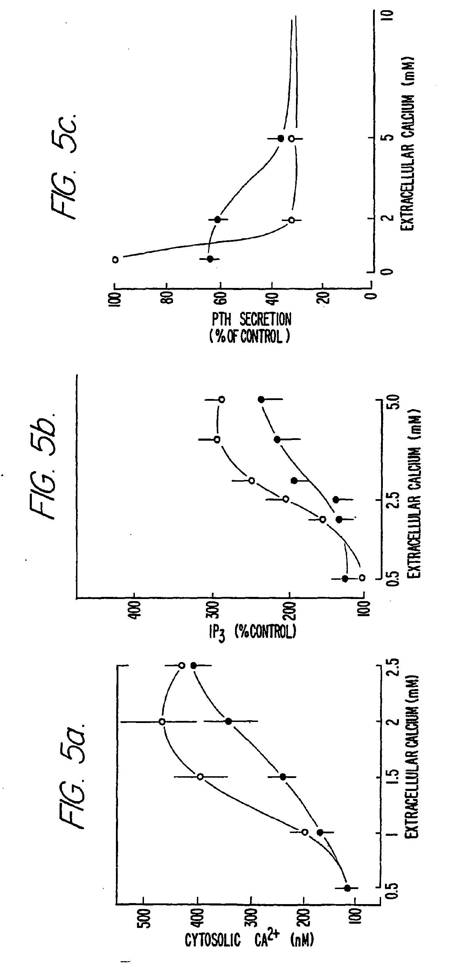

Fig. 5 is a graphical representation showing that the effects of PMA on [Ca²⁺]ᵢ, IP₃ formation, and PTH secretion are overcome by increasing concentrations of extracellular Ca²⁺. For each variable, there is a shift to the right in the concentration-response curve for extracellular Ca²⁺. Note also that the concentration-response curves vary sigmoidally as [Ca²⁺] increases linearly.

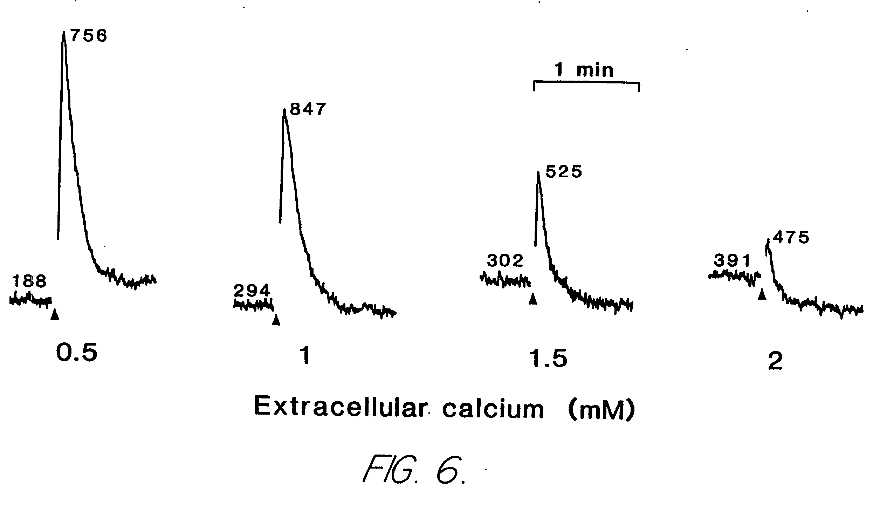

Fig. 6 is a graphical representation showing that increases in [Ca²⁺]ᵢ elicited by spermine are progressively depressed by increasing [Ca²⁺]. Spermine (200 µM) was added at the time shown by arrowheads. In this and all subsequent figures, the numbers accompanying the traces are [Ca²⁺]ᵢ in nM.

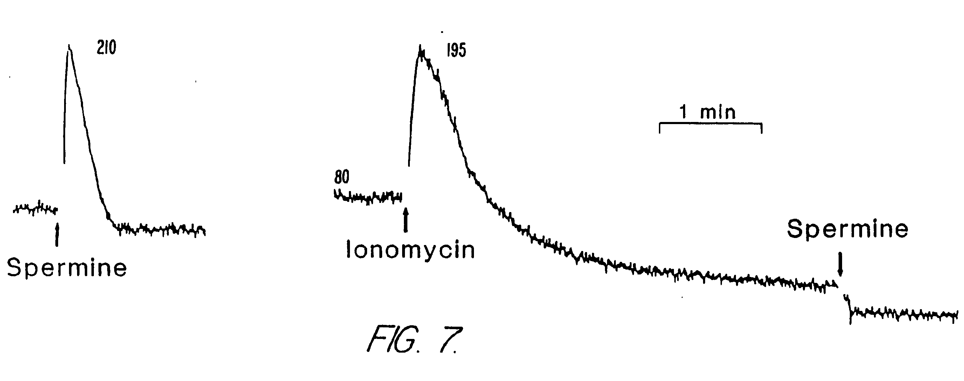

Fig. 7 is a graphical representation showing that spermine mobilizes intracellular Ca²⁺ in bovine parathyroid cells. EGTA was added to reduce [Ca²⁺] to <1 µM before the addition of spermine (200 µM) as indicated (left trace). Pretreatment with ionomycin (1 µM) blocks the response to spermine (right trace).

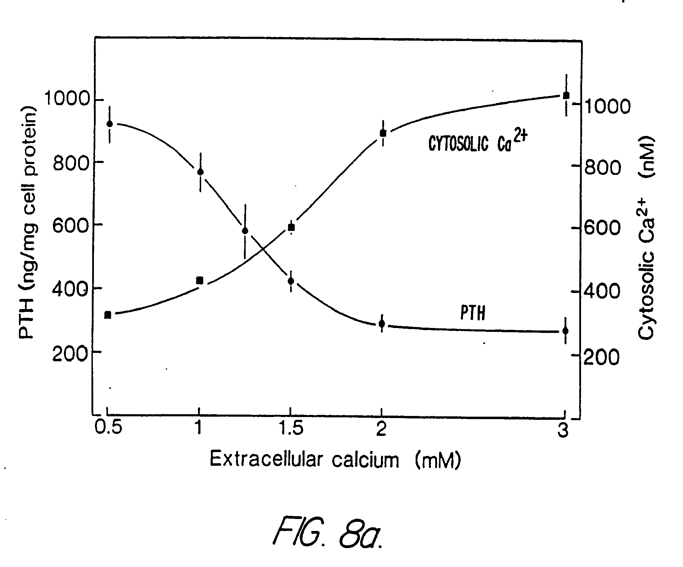

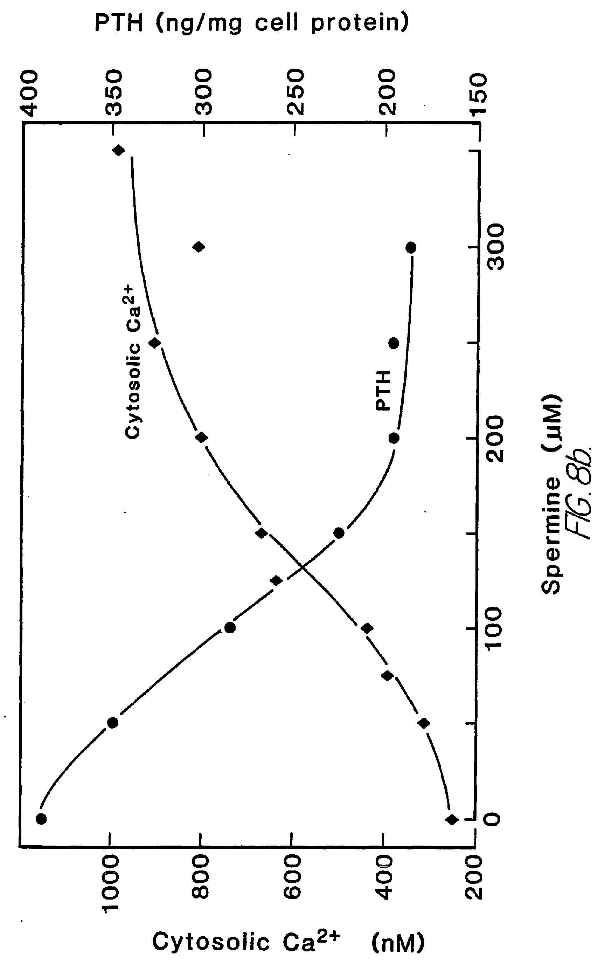

Figs. 8A and B are graphical representations showing that spermine increases [Ca²⁺]ᵢ and inhibits PTH secretion in bovine parathyroid cells similarly to extracellular Ca²⁺. The data points for the spermine dose-concentration response curves are the means of two experiments.

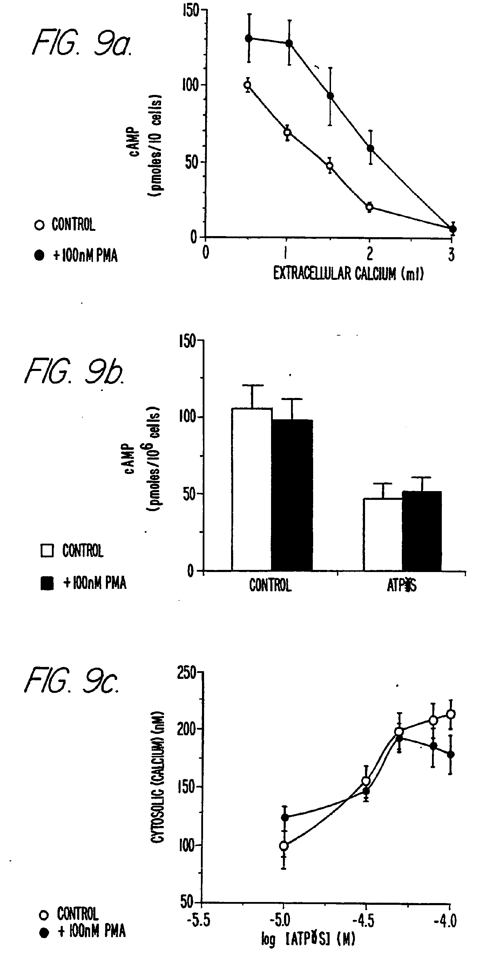

Fig. 9 is a graphical representation showing the contrasting effects of PMA on responses to extracellular Ca²⁺ and on responses to ATPγS in bovine parathyroid cells. Left panel: The concentration-response curve for extracellular Ca²⁺-induced inhibition of cyclic AMP formation is shifted to the right by PMA (100 nM). Middle panel: PMA does not affect the ability of ATPγS to increases [Ca²⁺]ᵢ. Note also that the concentration-response curve to ATPγS shows classical sigmoidal behavior as a function of the log concentration, in contrast to extracellular divalent cations.

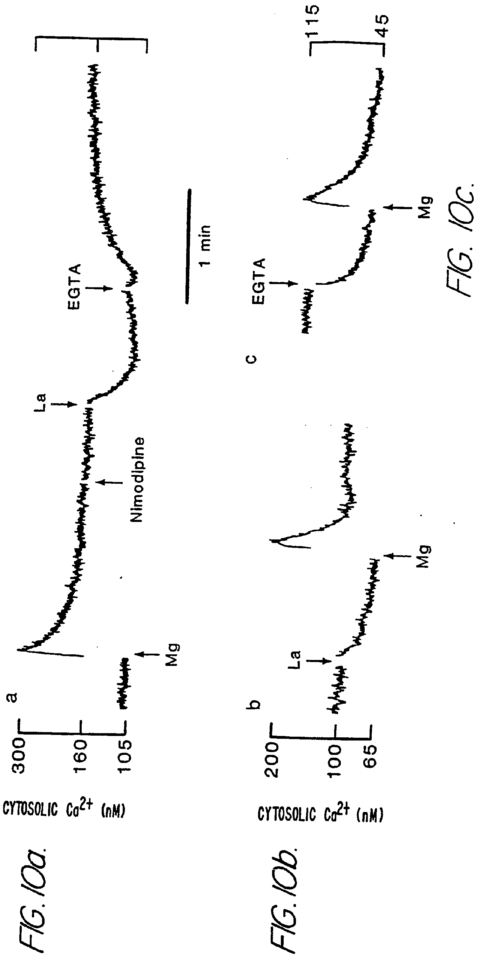

Fig. 10 is a graphical representation showing mobilization of intracellular Ca²⁺ in human parathyroid cells evoked by extracellular Mg²⁺. Cells were obtained from an adenoma and bathed in buffer containing 0.5 mM extracellular Ca²⁺. (a) Transient and sustained increases in [Ca²⁺]ᵢ elicited by extracellular Mg²⁺ (10 mM, final) shows that sustained increases are not affected by nimodipine (1 µM) but are depressed by La³⁺ (1 µM) and return promptly when La³⁺ is selectively chelated by a low concentration of EGTA. (b) La³⁺ (1 µM) blocks the sustained but not the transient increase in [Ca²⁺]ᵢ elicited by extracellular Mg²⁺. (c) Cytosolic Ca²⁺ transients elicited by extracellular Mg²⁺ persist in the absence of extracellular Ca²⁺.

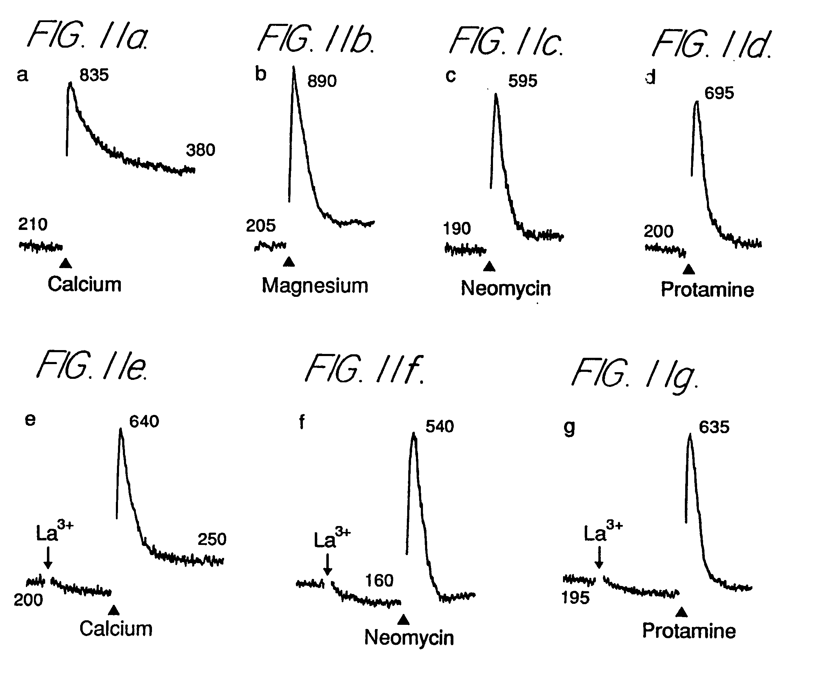

Fig. 11 is a graphical representation showing mobilization of intracellular Ca²⁺ evoked by neomycin or protamine in bovine parathyroid cells. In all traces, the initial [Ca²⁺] and [Mg²⁺] was 0.5 and 1 mM, respectively. In trace (a) and (b), the Ca²⁺ and Mg²⁺ concentrations were increased to 2 and 8 mM, from 0.5 and 1mM respectively. In the other traces, (c) through (i), neomycin B (30 µM) or protamine (1 ug/ml) were added as indicated. La³⁺ (1 µM) EGTA (1 mM), or ionomycin (100 nM) were added as indicated. Each trace is representative of the pattern seen in 5 or more trials using at least 3 different cell preparations. Bar = 1 min.

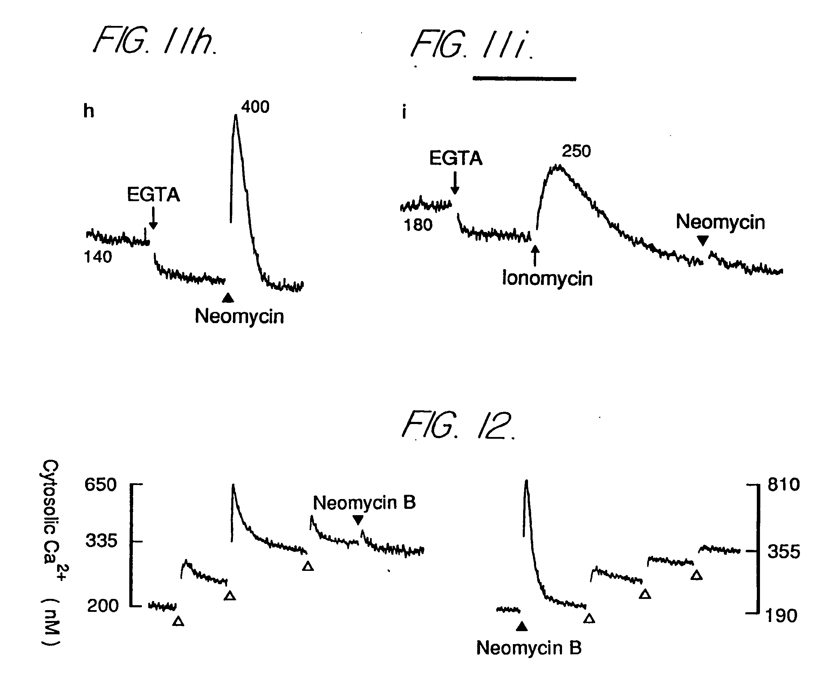

Fig. 12 is a graphical representation showing that neomycin B blocks transient but does not block steady-state increases in [Ca²⁺]ᵢ elicited by extracellular Ca²⁺. Left control: [Ca²⁺] was initially 0.5 mM and was increased in 0.5 mM increments at each of the open arrowheads before the addition of neomycin B (30 µM). Right: Neomycin B (30 µM) was added before [Ca²⁺]. Bar = 1 min.

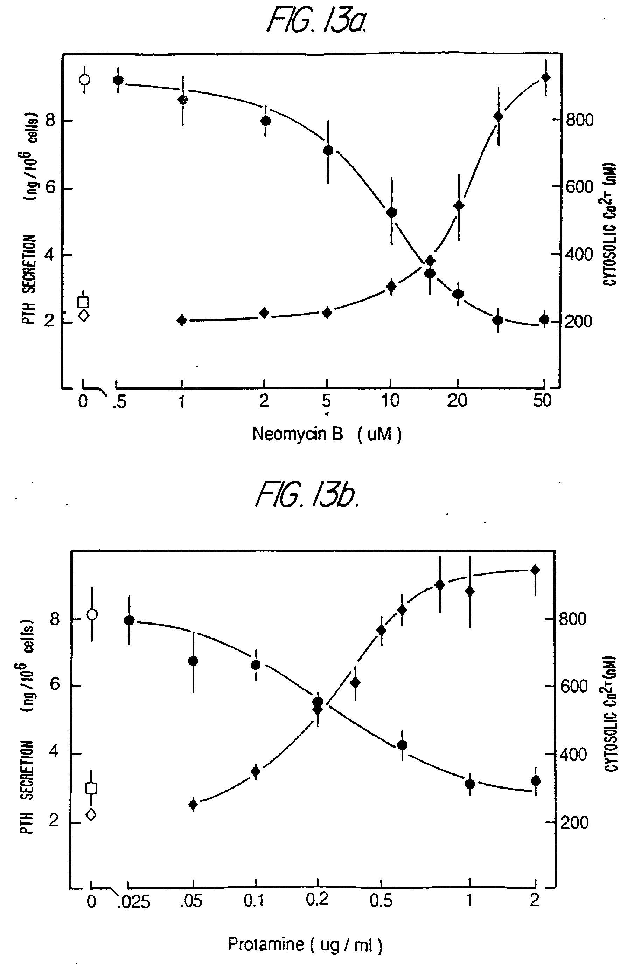

Fig. 13 is a graphical representation showing that neomycin B or protamine inhibit PTH secretion at concentrations which evoked increases in [Ca²⁺]ᵢ. Cells were incubated with the indicated concentrations of organic polycation for 30 min. in the presence of 0.5 mM extracellular Ca²⁺. Open symbols: control responses for PTH secretion in the presence of 0.5 (circles) or 2 mM (diamonds) extracellular Ca²⁺. Values for [Ca²⁺]ᵢ are diamond symbols. Bovine cells were used in the experiments with protamine and human (adenoma) parathyroid cells were used in the experiments with neomycin B. Each point is the mean ± SEM of 3 experiments.

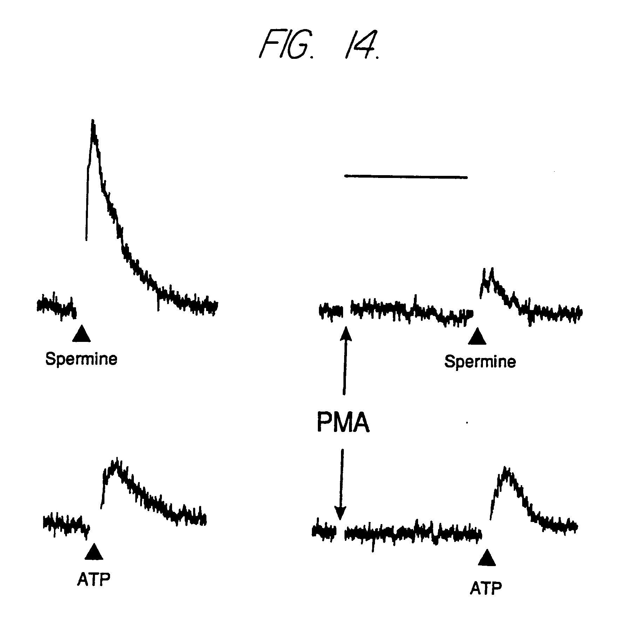

Fig. 14 is a graphical representation showing the preferential inhibitory effects of PMA on cytosolic Ca²⁺ transients elicited by spermine. Initial [Ca²⁺] was 0.5 mM; spermine (200 µM) or ATP (50 µM) were added as indicated. Bar = 1 min.

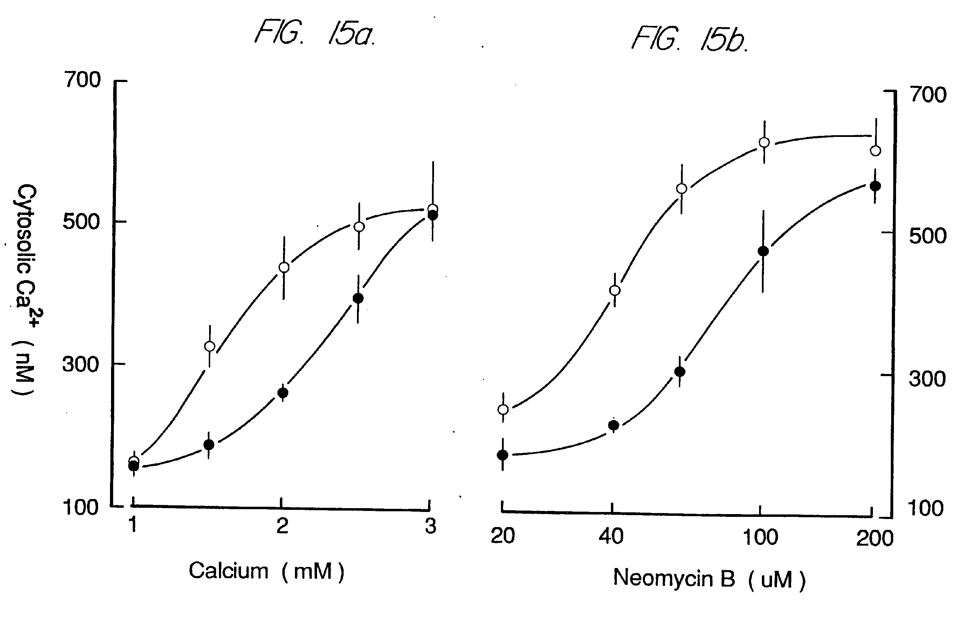

Fig. 15 is a graphical representation showing that PMA shifts to the right the concentration-response curves for extracellular Ca²⁺- and neomycin B-induced increases in [Ca²⁺]ᵢ. Cells were pretreated with PMA for 1 min. before increasing [Ca²⁺] or before adding neomycin B as indicated. Each point is the mean ± SEM of 3 to 5 experiments.

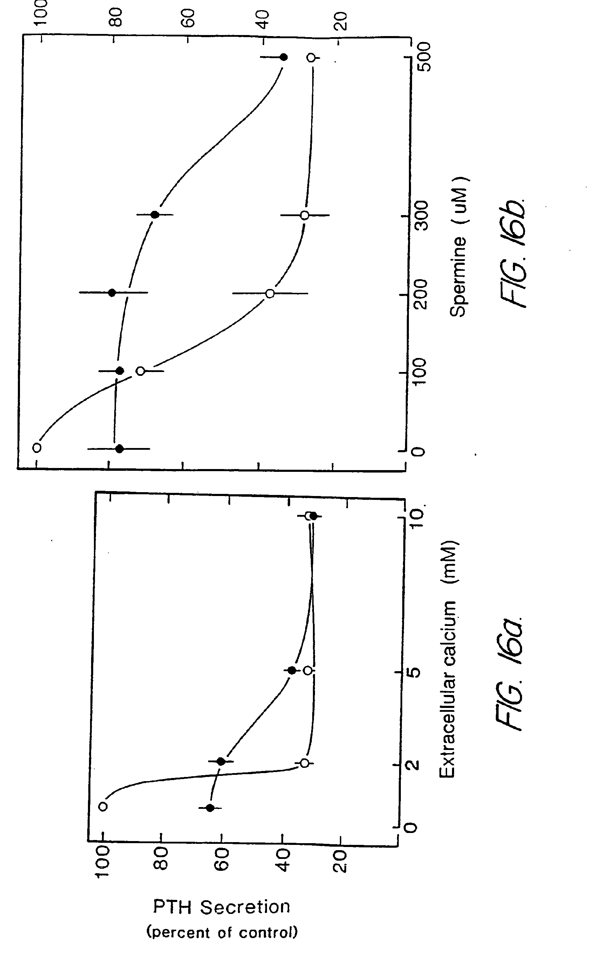

Fig. 16 is a graphical representation showing that PMA shifts to the right the concentration-response curves for extracellular Ca²⁺- and spermine-induced inhibition of PTH secretion. Cells were incubated with the indicated [Ca²⁺] and spermine for 30 min. in the presence (closed circles) or absence (open circles) of 100 nM PMA. Each point is the mean ± SEM of 3 experiments.

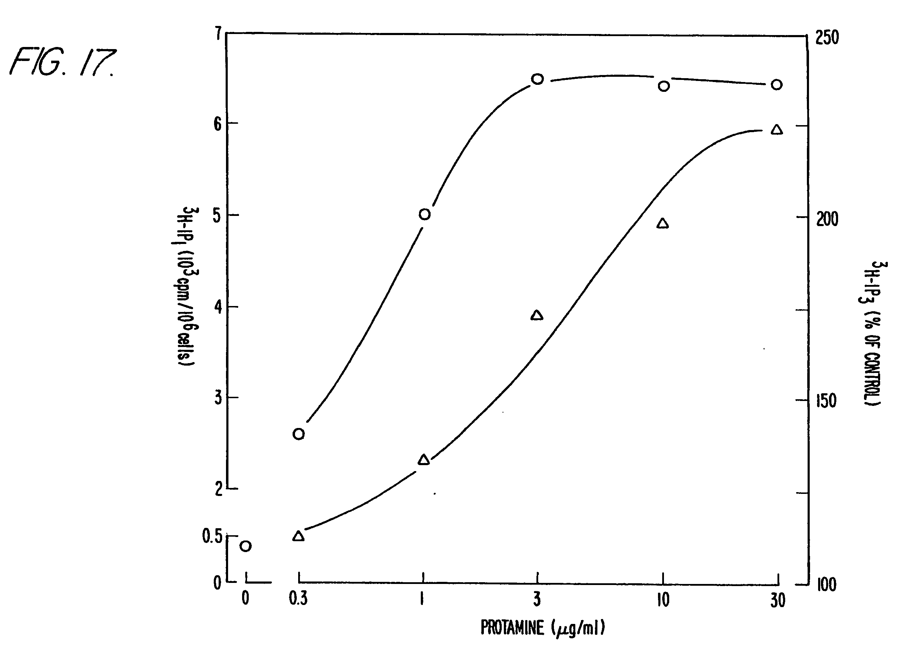

Fig. 17 is a graphical representation showing that protamine increases the formation of inositol phosphates. Parathyroid cells were incubated overnight in culture media containing 4 uCi/ml ³H-myo-inositol, washed, and incubated with the indicated concentration of protamine at 37°. After 30 sec. the reaction was terminated by the addition of CHCl₃:MeOH:HCl and IP₁ (circles) and IP₃ (triangles) separated by anion exchange chromatography. Each point is the mean of 2 experiments, each performed in triplicate.

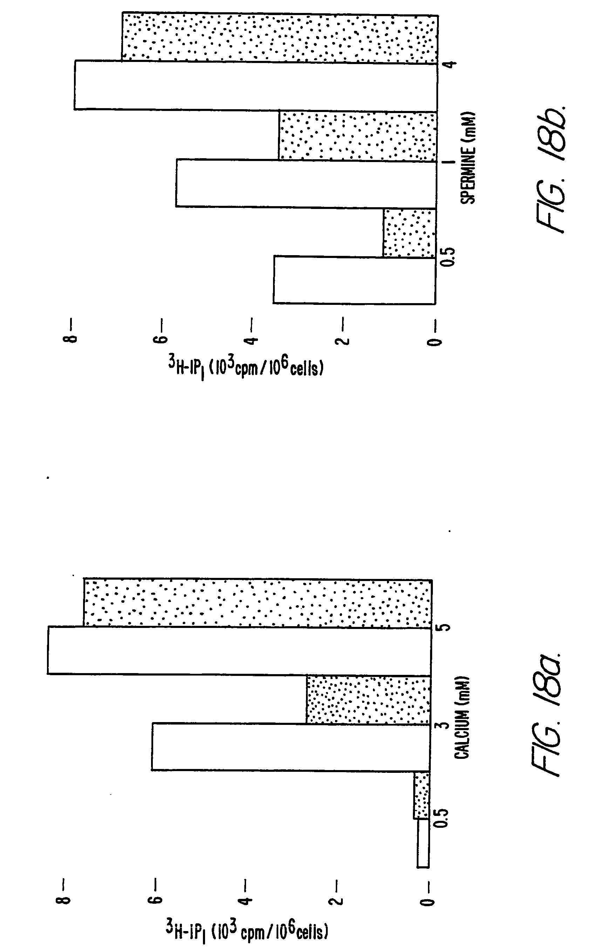

Fig. 18 is a graphical representation showing that PMA depresses the formation of IP₁ evoked by extracellular Ca²⁺ or spermine. ³H-myo-insoitol-labeled cells were exposed to the indicated [Ca²⁺] or spermine for 30 sec. before terminating the reaction and determining IP₁ by anion exchange chromatography. Hatched columns: Cells were pretreated with PMA (100 nM) for 5 min. before increasing [Ca²⁺] or adding spermine. Each value is the mean of 2 experiments, each performed in triplicate.

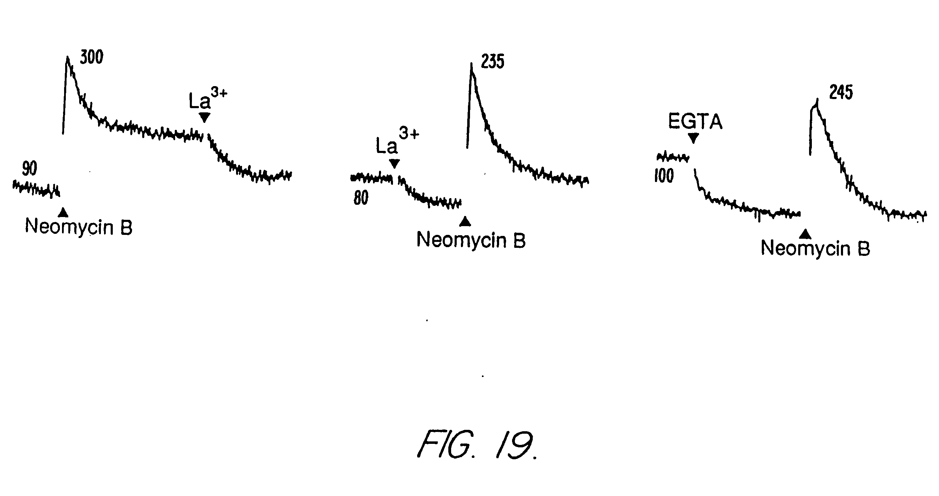

Fig. 19 is a graphical representation showing transient and sustained increases in [Ca²⁺]ᵢ elicited by neomycin B in human (adenoma) parathyroid cells. [Ca²⁺] was 0.5 mM. (a) The sustained increase in [Ca²⁺]ᵢ elicited by neomycin B (10 µM) was depressed by La³⁺. (b) The transient increase in [Ca²⁺]ᵢ evoked by neomycin B was unaffected by La³⁺. (c) Transient increases in [Ca²⁺]ᵢ persisted in the absence of extracellular Ca²⁺.

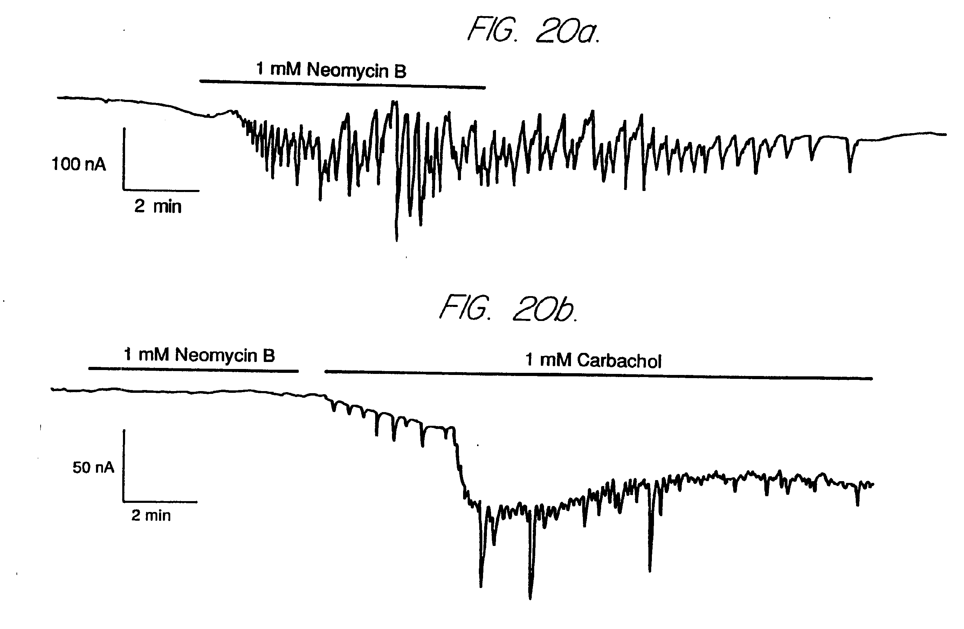

Fig. 20 is a graphical representation showing that neomycin B evokes oscillating increases the Cl⁻ conductance in Xenopus oocytes expressing the Ca²⁺ receptor. Upper trace from an oocyte three days after injection with human (hyperplastic) parathyroid cell poly (A) ⁺-mRNA. Lower trace from an oocyte injected with water. Neomycin B failed to elicit a response in five water-injected oocytes and carbachol elicited a response in one, which is shown. In both traces, the holding potential was -76 mV.

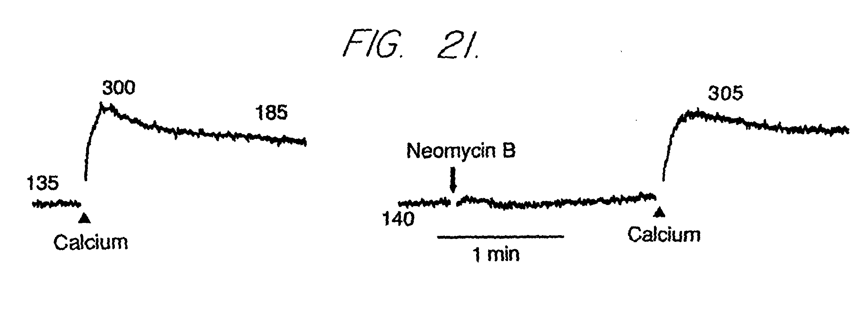

Fig. 21 is a graphical representation showing that neomycin B fails to affect basal or evoked increases in C-cells. Control, left trace: Fura-2-loaded rMTC 6-23 cells were initially bathed in buffer containing 1 mM Ca²⁺ before increasing [Ca²⁺] to 3 mM. Right trace: pretreatment with 5 mM neomycin B.

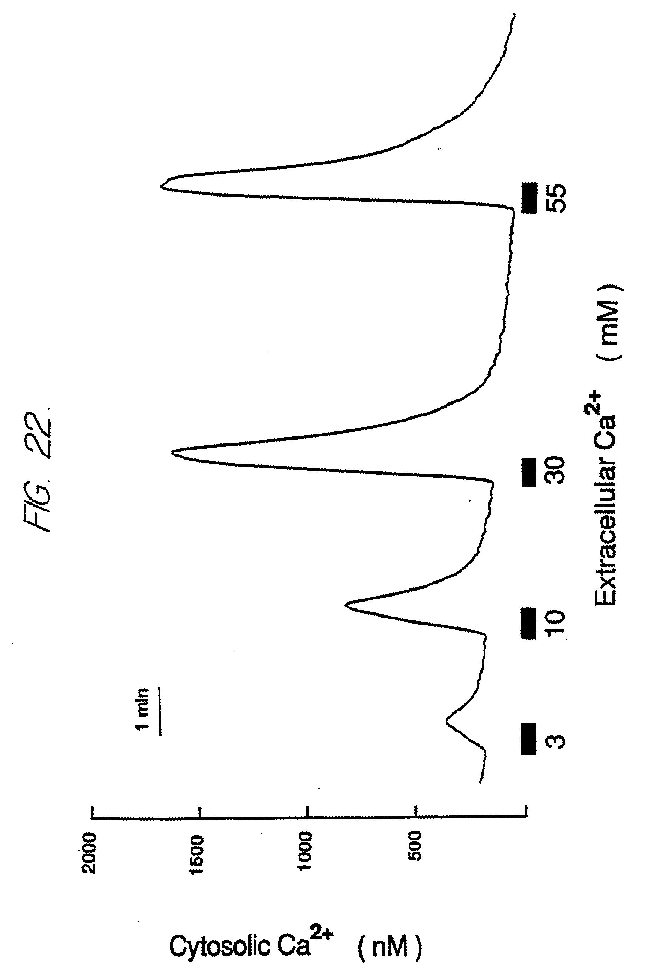

Fig. 22 is a graphical representation showing that extracellular Ca²⁺ evokes increases in [Ca²⁺]ᵢ in rat osteo-clasts. Microfluorimetric recording in a single rat osteoclast loaded with indo-1 and superfused for the indicated times (bars) with buffer containing the indicated _{[}Ca²⁺]. Normal buffer, superfused between the bars, contained 1 mM Ca²⁺.

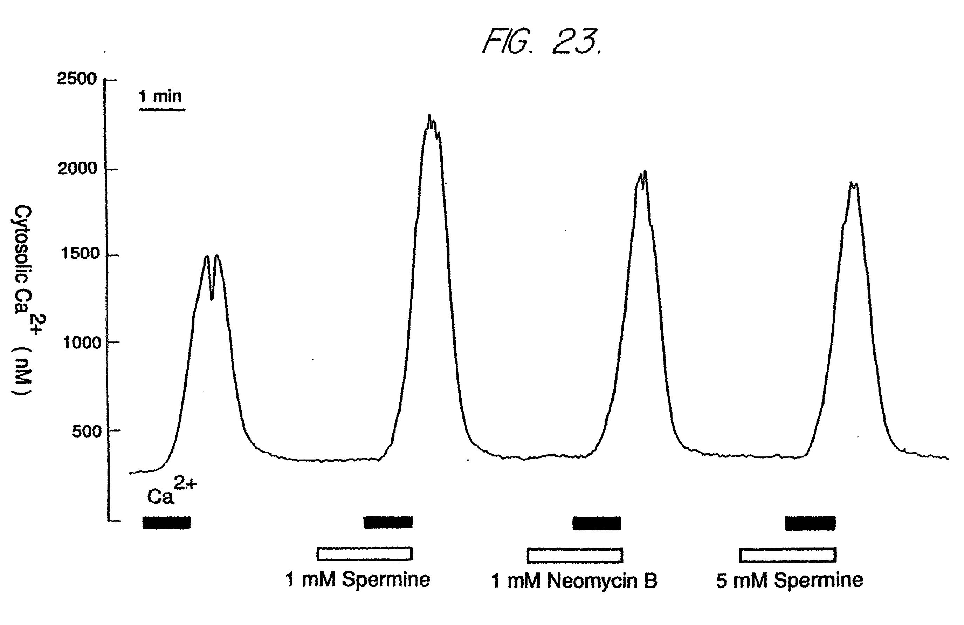

Fig. 23 is a graphical representation showing that spermine or neomycin B fail to evoke increases in [ca²⁺]ᵢ in rat osteoclasts. An indo-1-loaded osteoclast was superfused with the indicated concentration of spermine or neomycin B (open bars) alone or together with 20 mM Ca²⁺ (solid bars).

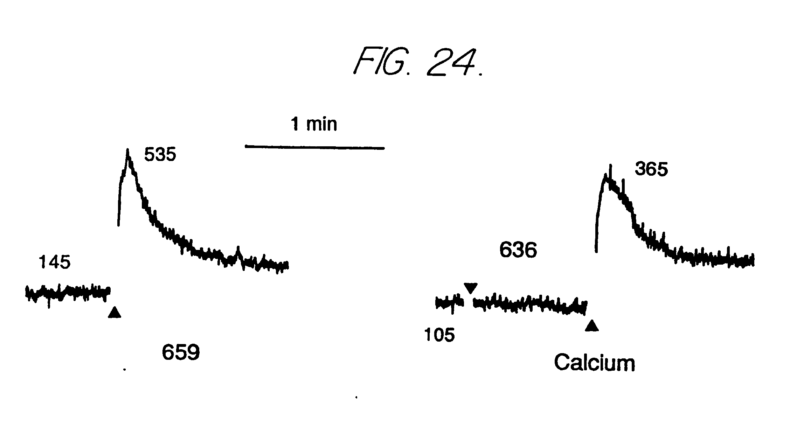

Fig. 24 is a graphical representation showing the differential effects of argiotoxin (shown as argiopine in the figure, structures also shown in Fig. 1) 659 and argiotoxin 636 on [Ca²⁺]ᵢ in bovine parathyroid cells. The initial [Ca²⁺] was 0.5 mM and was increased to 1.5 mM where indicated (right trace). Where indicated, argiotoxin 659 (300 µM) or argiotoxin 636 (400 µM) was added.

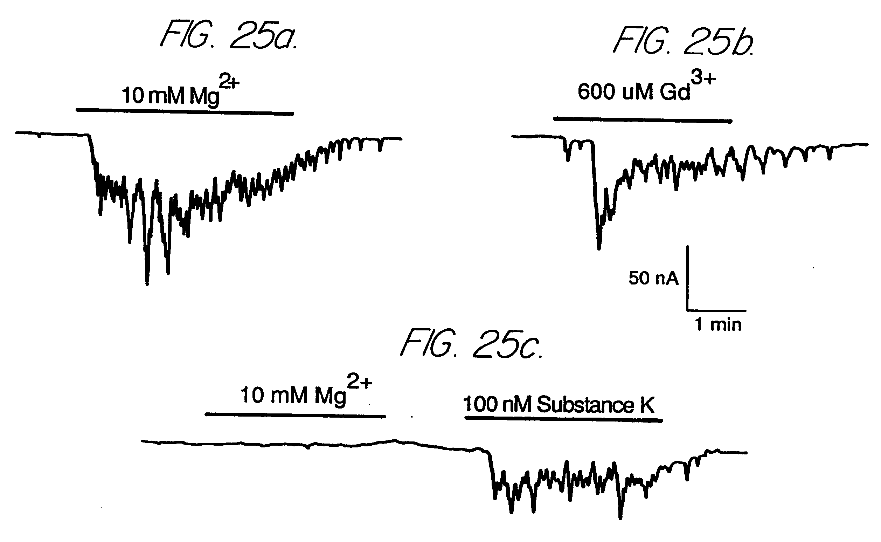

Fig. 25 is a graphical representation showing that extracellular Mg²⁺ or Gd³⁺ evoke oscillatory increases in Cl⁻ conductance in Xenopus oocytes injected with bovine parathyroid cell poly(A)⁺-mRNA. In trace (a), the concentration of extracellular Ca²⁺ was < 1 µM and in trace (b), 0.7 mM. Trace (c) shows that extracellular Mg²⁺ fails to elicit a response in an oocyte injected only with the mRNA for the substance K receptor, although superfusion with substance K evokes a response. Holding potential was -70 to -80 mV.

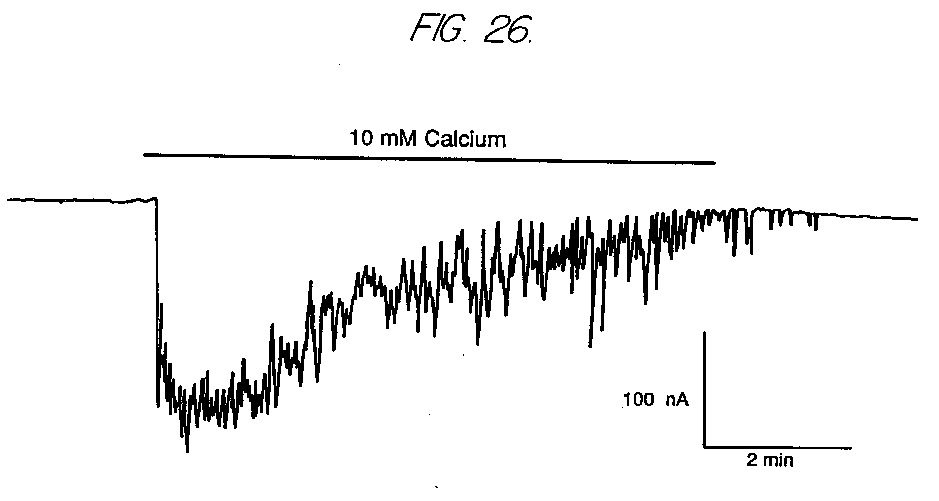

Fig. 26 is a graphical representation showing that extracellular Ca²⁺ elicits oscillatory increases in Cl⁻ conductance in Xenopus oocytes injected with human (hyperplastic) parathyroid tissue poly(A) ⁺-mRNA. The oocyte was tested for responsivity to extracellular Ca²⁺ three days after injection of 50 ng poly (A) ⁺-mRNA. Holding potential was -80 mV.

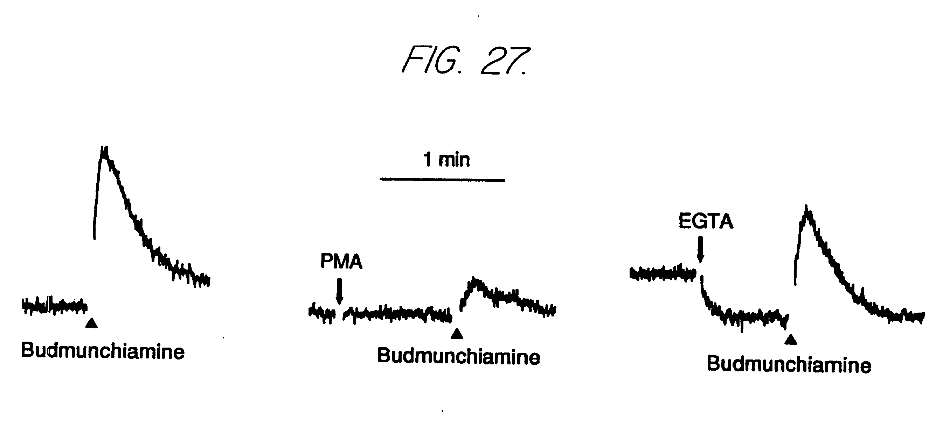

Fig. 27 is a graphical representation showing the mobilization of intracellular Ca²⁺ in bovine parathyroid cells elicited by budmunchiamine. Budmunchiamine (300 µM, structure also shown) was added where indicated.

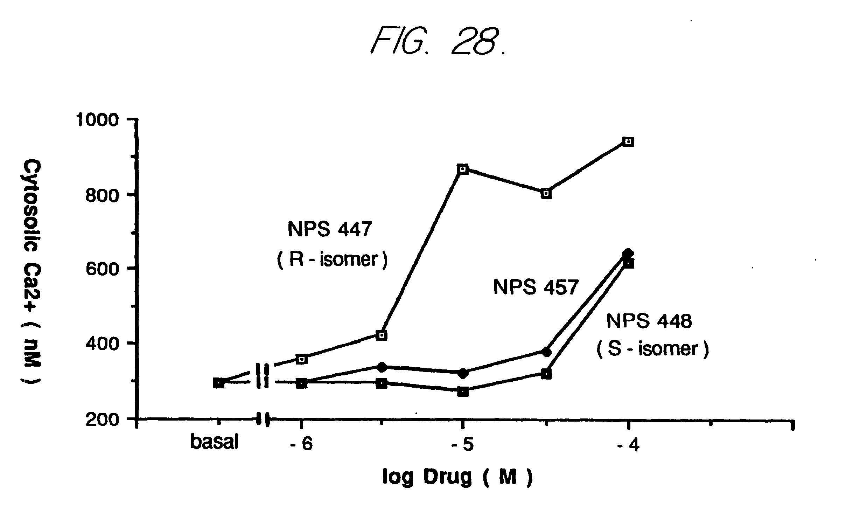

Fig. 28 is a graphical representation showing that the ability to mobilize intracellular Ca²⁺ in parathyroid cells is stereospecific. Bovine parathyroid cells loaded with fura-2 were initially suspended in buffer containing 0.5 mM extracellular Ca²⁺ before the addition of the indicated concentration of each molecule.

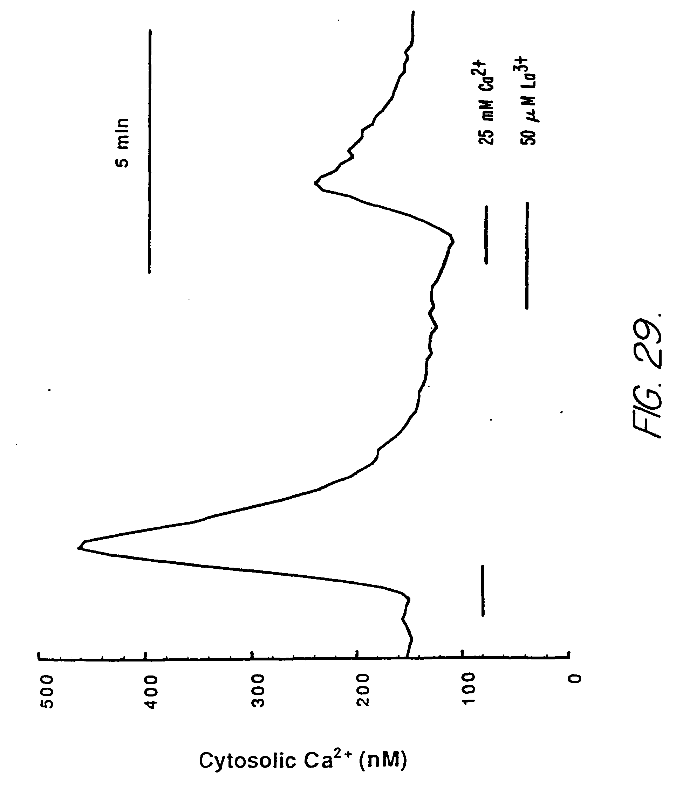

Fig. 29 is a graphical representation showing effects of La³⁺ on [Ca²⁺]ᵢ in osteoclasts. A representative trace from a single rat osteoclast loaded with indo-1 is shown. At low concentrations, La³⁺ partially blocks increases in [Ca²⁺]ᵢ, elicited by extracellular Ca²⁺.

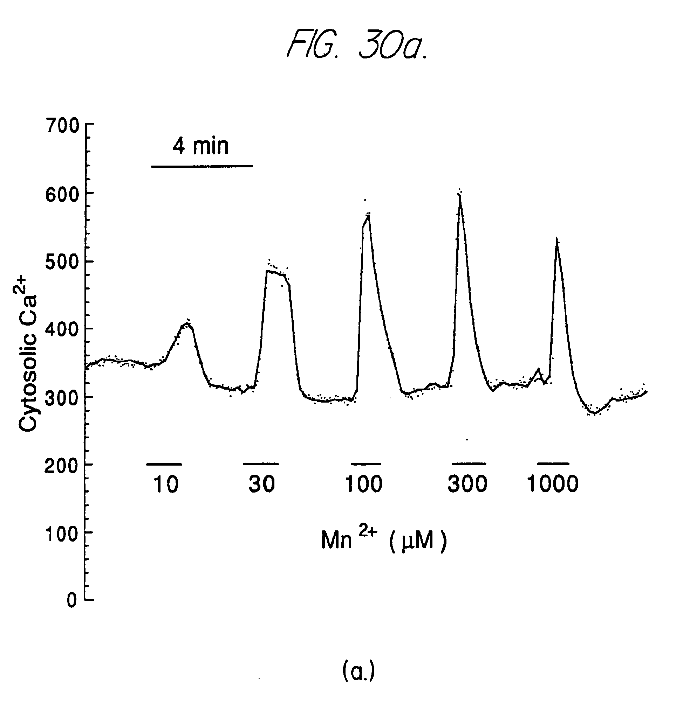

Figs. 30A and B are graphical representations showing the mobilization of intracellular Ca²⁺ elicited by extracellular Mn²⁺ in rat osteoclasts. Extracellular Mn²⁺ evokes concentration-dependent increases in [Ca²⁺]ᵢ (Fig. 30A) that persist in the absence of extracellular Ca²⁺ (Fig. 30B).

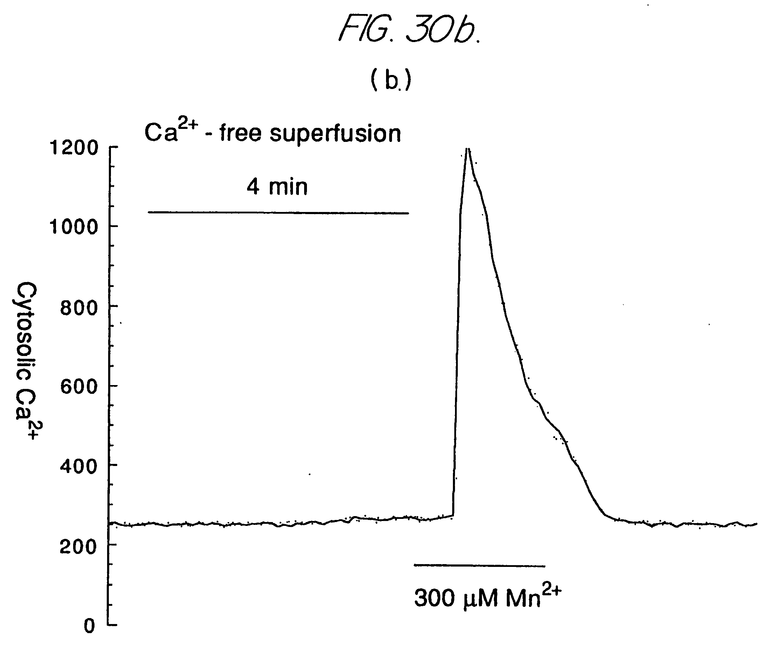

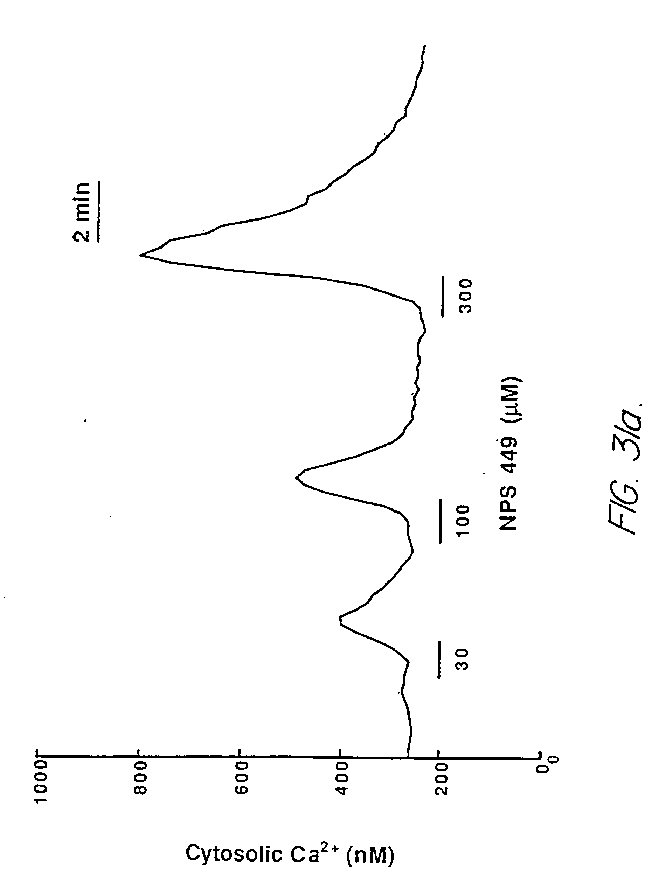

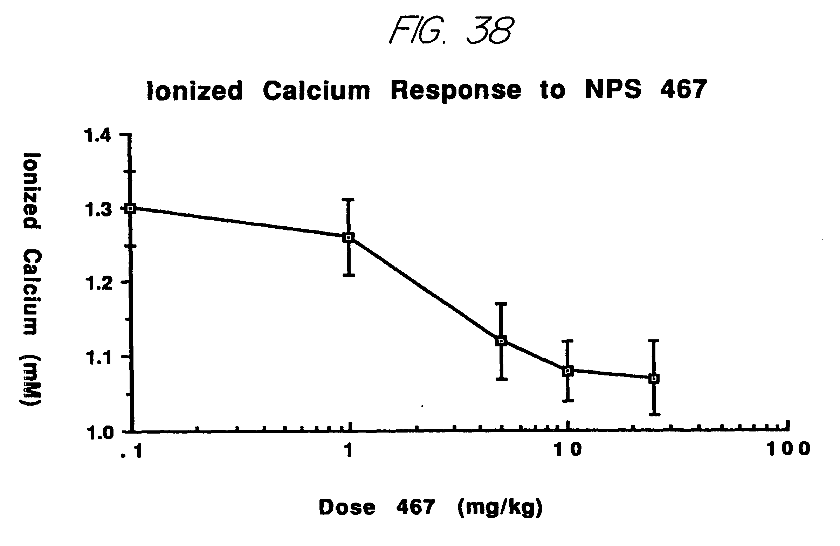

Figs. 31A and 31B are graphical representations showing mobilization of [Ca²⁺]ᵢ in rat osteoclasts elicited by a molecule termed NPS 449 (see Fig. 38). Isolated rat osteoclasts loaded with indo-1 were superfused with the indicated concentrations of NPS 449 in the presence (Fig. 31A) or absence (Fig. 31B) of 1 mM extracellular CaCl₂.

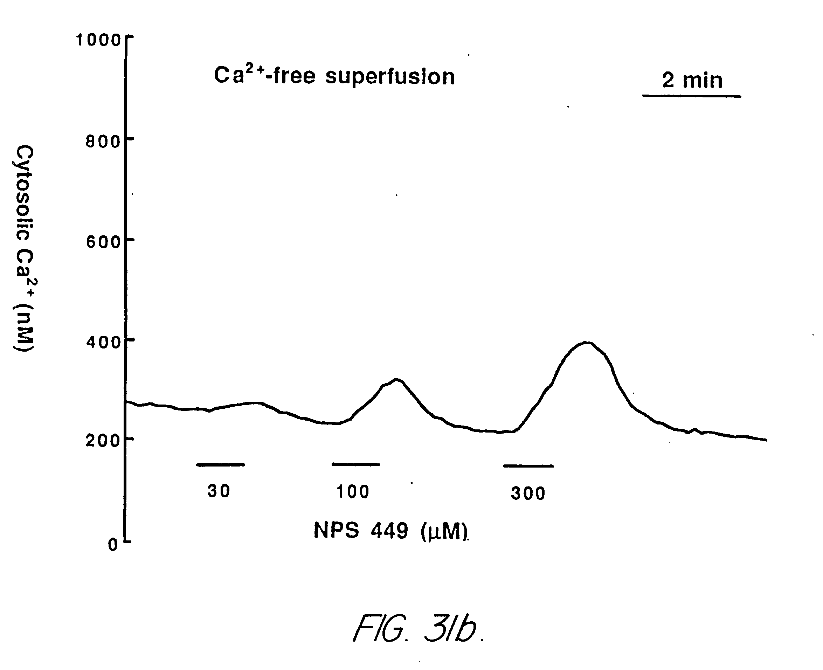

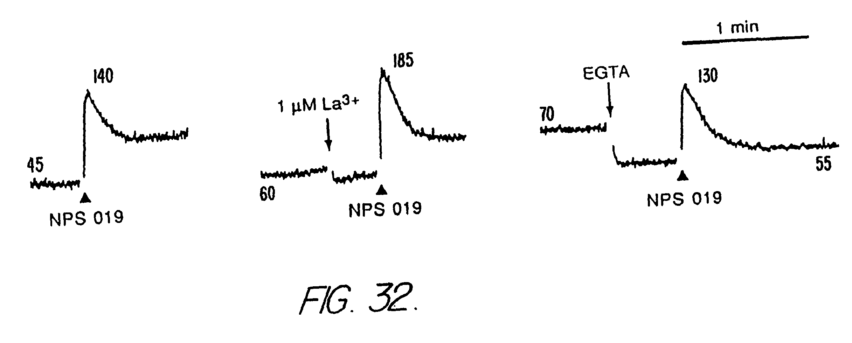

Fig. 32 is a graphical representation showing the mobilization of intracellular Ca²⁺ in C-cells evoked by NPS 019 (see Fig. 1). rMTC 6-23 cells were loaded with fura-2 and bathed in buffer containing 0.5 mM [Ca²⁺]. Where indicated, NPS 019 was added to a final concentration of 10 µM. Representative traces show that the transient increase in [Ca²⁺]ᵢ elicited by NPS 019 is refractory to inhibition by La³⁺ (middle trace) and persists in the absence of extracellular Ca²⁺ (right trace).

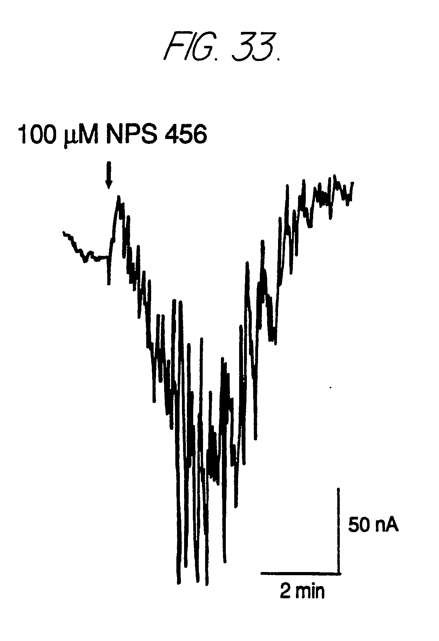

Fig. 33 is a graphical representation showing that NPS 456 (Fig. 36) evokes oscillatory increases in Cl⁻ current in Xenopus oocytes which have been injected with bovine parathyroid cell poly(A)⁺-mRNA.

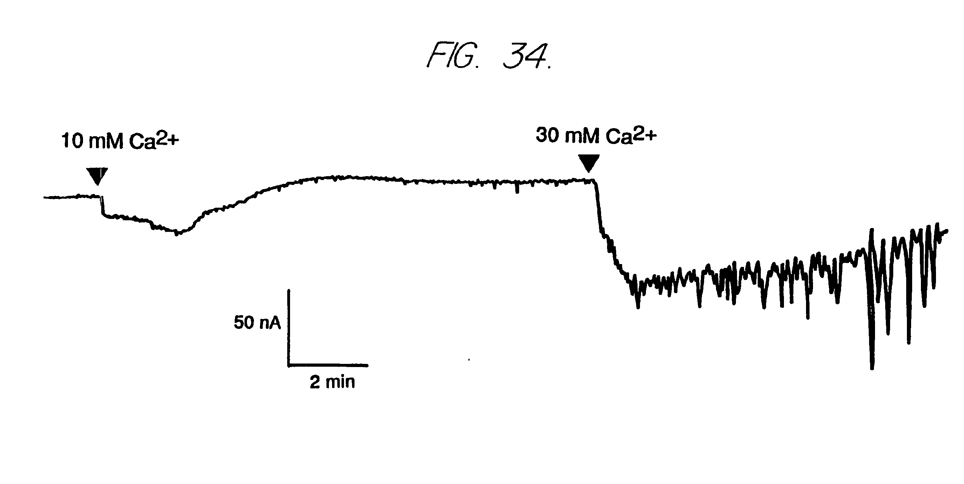

Fig. 34 is a graphical representation showing that extracellular Ca²⁺ evokes oscillatory increases in Cl⁻ current in Xenopus oocytes which have been injected with human osteoclast mRNA. The oocyte was tested for responsivity to extracellular Ca²⁺ three days after injection of 50 ng of total poly(A)⁺ mRNA.

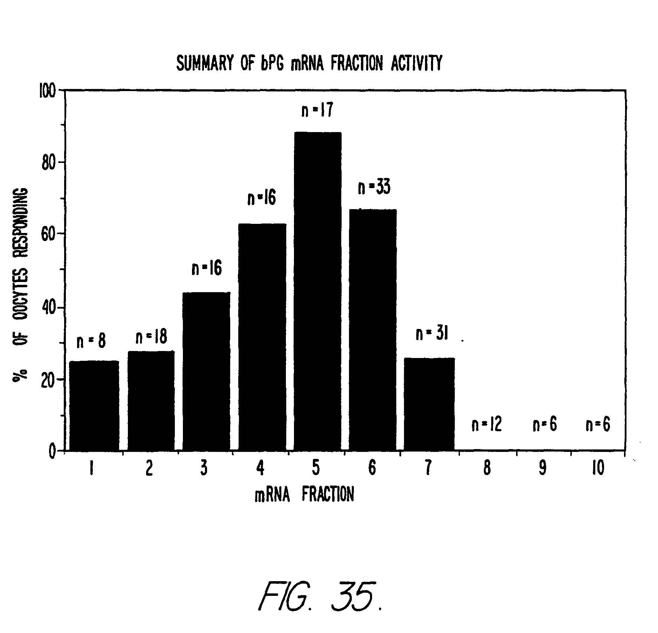

Fig. 35 is a graphical representation showing that the parathyroid cell Ca²⁺ receptor is encoded by mRNA in a size range of 2.5-3.5 kb. Bovine parathyroid cell poly (A)⁺ -mRNA was size fractionated on denaturing glycerol gradients and pooled into ten fractions. Each fraction was injected (50 ng/fraction) separately into Xenopus oocytes. After three days, the oocytes were examined for their ability to respond to extracellular Ca²⁺ with oscillatory increases in the Cl⁻ conductance.
Fig. 36 shows the chemical structures of molecules derived from diphenylpropyl-α-phenethylamine illustrating a family of molecules which were prepared and screened to find the useful molecules of the invention.

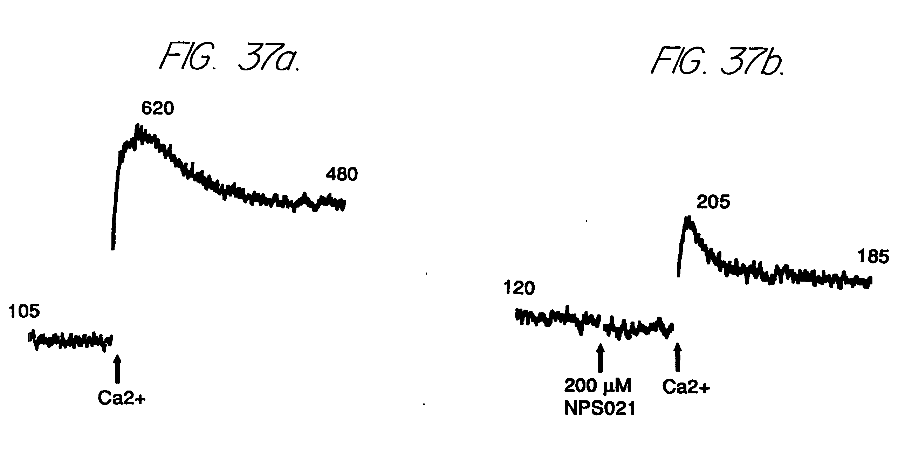

Fig. 37 is a graphical representation showing that NPS 021 is a calcilytic compound that blocks the effects of extracellular Ca²⁺ on [Ca²⁺]ᵢ in bovine parathyroid cells. Cells were initially bathed in buffer containing 0.5 mM CaCl₂ and, where indicated, the [Ca²⁺] was increased to a final of 2mM (left trace). The addition of NPS 021 (200 µM) caused no change in [Ca²⁺]ᵢ but inhibited the increase in [Ca²⁺]ᵢ elicited by extracellular Ca²⁺ (right trace).
Fig. 38 is a graph showing in vivo Ca²⁺ response to NPS 467.

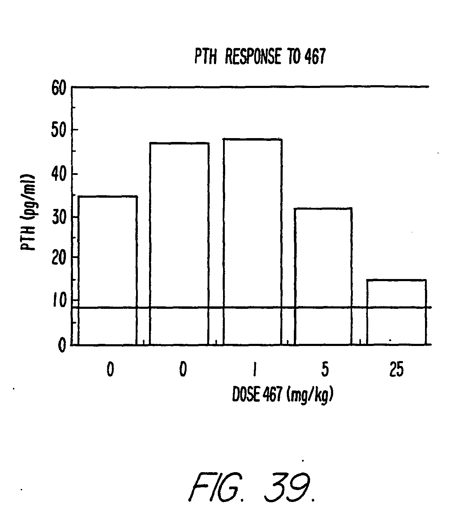

Fig. 39 is a graph showing in vivo PTH response to NPS 467.

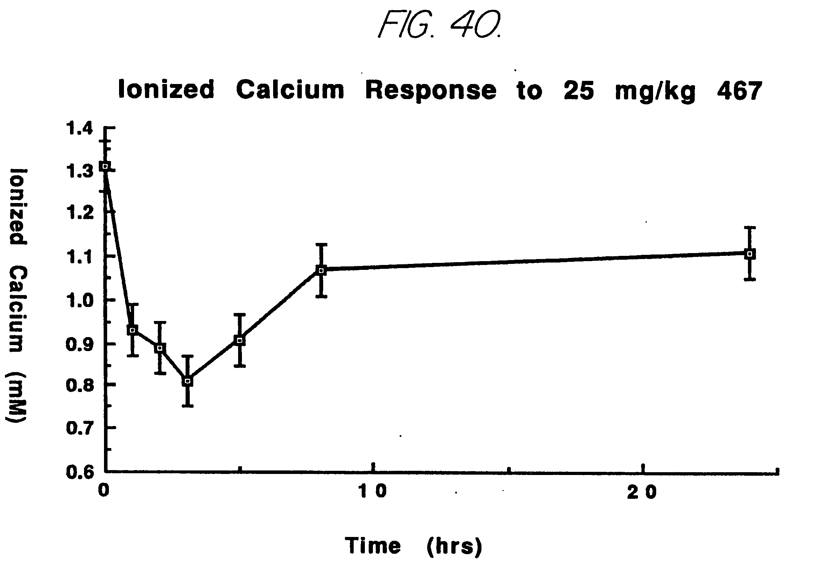

Fig. 40 is a graph showing in vivo Ca²⁺ response to 25 mg/kg NPS 467.

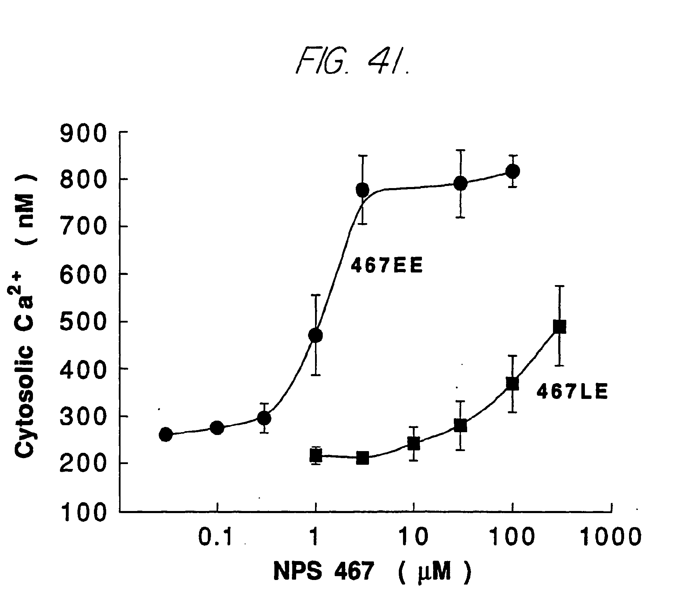

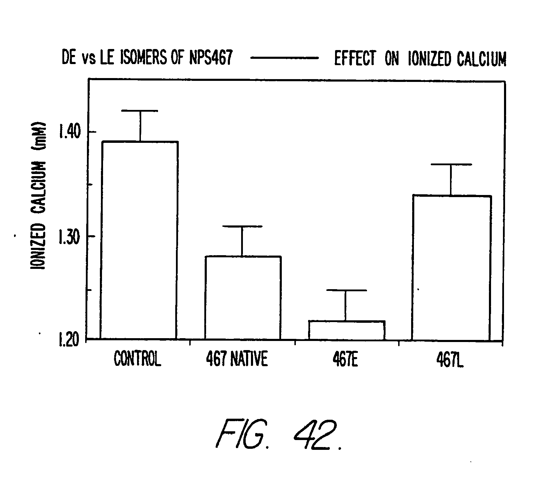

Figs. 41 and 42 are graphs showing in vivo Ca²⁺ responses to different enantiomers of NPS 467.

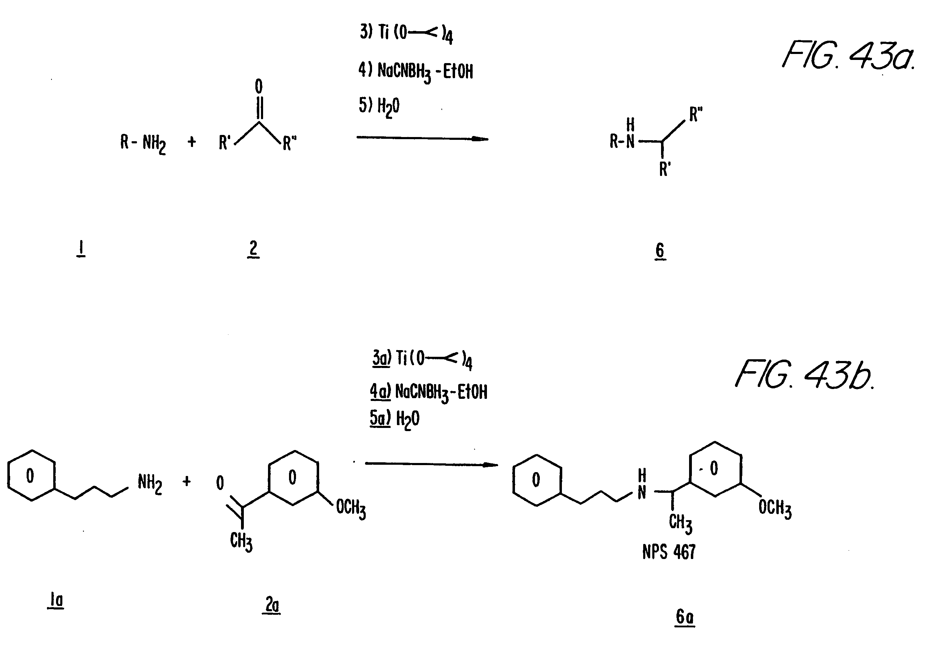

Fig. 43a depicts a reaction scheme for the preparation of fendiline or fendiline analogs or derivatives depicted in Figure 36. Fig. 43b depicts a reaction scheme for the synthesis of NPS 467.

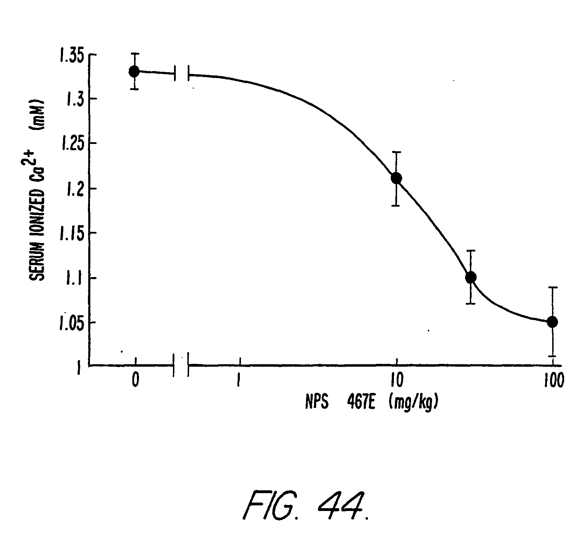

Fig. 44 depicts a dose response curve showing that NPS 467 lowers serum ionized calcium when administered orally.

### Calcimimetic and Calcilytic Molecules

Calcimimetic and calcilytic molecules useful in the invention are generally described above. These molecules can be readily identified using screening procedures to define molecules which mimic or antagonize the activity of Ca²⁺ at Ca²⁺ receptors. Examples of such procedures are provided below. These examples are not limiting in the invention but merely illustrate methods which are readily used or adapted by those skilled in the art.

Generally, calcinimetic and calcilytic molecules are identified by screening molecules which are modelled after those described below (called lead molecules). As can be seen below there are several specific calcimimetics and calcilytics useful at various Ca²⁺ receptors. Derivative molecules are readily designed by standard procedures and tested in one of many protocols known to those skilled in the art. Many molecules may be screened easily to identify the most useful in this invention.

Organic cationic molecules which mimic or antagonize the actions of Ca²⁺ in other systems contain the requisite structure for activity on a Ca²⁺ receptor. Rational design of other useful molecules involves the study of a molecule known to be calcimimetic or calcilytic and then modifying the strucure of the known molecule. For example, polyamines are potentially calcimimetic since spermine mimics the action of Ca²⁺ in several in vitro systems. Results show that spermine does indeed cause changes in [Ca²⁺]ᵢ and PTH secretion reminiscent of those elicited by extracellular di- and trivalent cations (see below). The experiments outlined below are therefore aimed at demonstrating that this phenomenology, obtained with spermine, involves the same mechanisms used by extracellular Ca²⁺. To do this, the effects of spermine on a variety of physiological and biochemical parameters which characterize activation of the Ca²⁺ receptor were assessed. Those molecules having similar effects are useful in this invention and can be discovered by selecting or making molecules having a structure similar to spermine. Once another useful molecule is discovered this selection process can be readily repeated.

For clarity, below is provided a specific series of screening protocols to identify such useful molecules which are active at a parathyroid cell Ca²⁺ receptor, or which act as agonists or antagonists of the cellular response to changes in [Ca²⁺]. Equivalent assays can be used for molecules active at other Ca²⁺ receptors, or which otherwise mimic or antagonize cellular functions regulated by [Ca²⁺]. These assays exemplify the procedures which are useful to find calcimimetic molecules of this invention. Equivalent procedures can be used to find calcilytic molecules by screening for those molecules most antagonistic to the actions of extracellular Ca²⁺. In vitro assays can be used to characterize the selectivity, saturability, and reversibility of these calcimimetics and calcilytics by standard techniques.

### Screening Procedure

Generally, bovine parathyroid cells loaded with fura-2 are initially suspended in buffer containing 0.5 mM CaCl₂. The test substance is added to the cuvette in a small volume (5-15 µl) and any change in the fluorescence signal noted. Cumulative increases in the concentration of the test substance are made in the cuvette until some predetermined concentration is achieved or changes in fluorescence noted. If no changes in fluorescence are noted, the molecule is considered inactive and no further testing is performed. In initial studies, e.g., with polyamine-type molecules, molecules were tested at concentrations as high as 5 or 10 mM. As more potent molecules are now known (see below), the ceiling concentration is lowered. For example, newer molecules are tested at concentrations up to 500 µM or less. If no changes in fluorescence are noted at this concentration, the molecule can be considered inactive.

Molecules causing increases in [Ca²⁺]ᵢ are subjected to additional testing. The two essential characteristics of the molecule important for its consideration as a calcimimetic molecule are the mobilization of intracellular Ca²⁺ and sensitivity to PKC activators. Molecules causing the mobilization of intracellular Ca²⁺ in a PMA-sensitive manner have invariably been found to be calcimimetic molecules and to inhibit PTH secretion. Additional testing can, if needed, be performed to solidify this belief. Typically, all the various tests for calcimimetic or calcilytic activity (see above) are not performed. Rather, if a molecule causes the mobilization of intracellular Ca²⁺ in a PMA-sensitive manner, it is advanced to screening on human parathyroid cells. For example, measurements of [Ca²⁺]ᵢ are performed to determine the EC₅₀, and to measure the ability of the molecule to inhibit PTH secretion in human parathyroid cells which have been obtained from patients undergoing surgery for primary or secondary hyperparathyroidism. The lower the EC₅₀ or IC₅₀ the more potent the molecule as a calcimimetic or calcilytic.

Measuring [Ca²⁺]ᵢ with fura-2 provides a very rapid means of screening new organic molecules for activity. In a single afternoon, 10-15 molecules can be examined and their ability to mobilize intracellular Ca²⁺ (or not) assessed. The sensitivity of any observed increase in [Ca²⁺]ᵢ to depression by PMA can also be assessed. Moreover, a single cell preparation can provide data on [Ca²⁺]ᵢ, cyclic AMP levels, IP₃ and PTH secretion. A typical procedure is to load cells with fura-2 and then split the cell suspension in two; most of the cells are used for measurement of [Ca²⁺]ᵢ and the remainder are incubated with molecules to assess their effects on cyclic AMP and PTH secretion. Because of the sensitivity of the radioimmunoassays for cyclic AMP and PTH, both variables can be determined in a single incubation tube containing 0.3 ml cell suspension (about 500,000 cells). Measurements of inositol phosphates are a time-consuming aspect of the screening. However, ion exchange columns eluted with chloride (rather than formate) provide a very rapid means of screening for IP₃ formation since rotary evaporation (which takes around 30 hrs) is not required. This method allows processing of nearly 100 samples in a single afternoon. Those molecules that prove interesting, as assessed by measurements of [Ca²⁺]ᵢ, cyclic AMP, IP₃, and PTH are then subjected to a more rigorous analysis by examining formation of various inositol phosphates and assessing their isomeric form by HPLC.

Interesting molecules detected in these protocols are then assessed for specificity, e.g., by examining their effects on [Ca²⁺]ᵢ in calcitonin-secreting C-cells using, e.g., the rat MTC 6-23 cell line.

The following is illustrative of methods useful in these screening procedures. Examples of typical results for various test calcimimetic or calcilytic molecules are provided in Figs. 2-34.

### Parathyroid Cell Preparation

Parathyroid glands were obtained from freshly slaughtered calves (12-15 weeks old) at a local abattoir and transported to the laboratory in ice-cold parathyroid cell buffer (PCB) which contains (mM): Nacl, 126; KCl, 4; MgCl₂, 1; Na-HEPES, 20; pH 7.4; glucose, 5.6, and variable amounts of CaCl₂, e.g., 1.25 mM. Human parathyroid glands, obtained from patients undergoing surgical removal of parathyroid tissue for primary or uremic hyperparathyroidism (HPT), were treated similarly to bovine tissue. Glands were trimmed of excess fat and connective tissue and then minced with a fine scissors into approximate cubes of 2-3 mm. Dissociated parathyroid cells were prepared by collagenase digestion. Dissociated cells were then purified by centrifugation in Percoll buffer. The resultant parathyroid cell preparation was essentially devoid of red blood cells, adipocytes, and capillary tissue as assessed by phase contrast microscopy and Sudan black B staining. Dissociated and purified parathyroid cells were present as small clusters containing 5 to 20 cells. Cellular viability, as indexed by exclusion of trypan blue or ethidium bromide, was routinely 95%.

Although cells can be used for experimental purposes at this point, physiological responses (suppressibility of PTH secretion and resting levels of [Ca²⁺]ᵢ) are better after culturing the cells overnight. Primary culture also has the advantage that cells can be labeled with isotopes to near isotopic equilibrium, as is necessary for studies involving measurements of inositol phosphate metabolism (see below). After purification on Percoll gradients, cells were washed several times in a 1:1 mixture of Ham's F12-Dulbecco's modified Eagles medium (GIBCO) supplemented with 50 ug/ml streptomycin, 100 U/ml penicillin, 5 ug/ml gentamicin and ITS⁺. ITS⁺ is a premixed solution containing insulin, transferrin, selenium, and bovine serum albumin (BSA)-linolenic acid (Collaborative Research, Bedford, MA). The cells were then transferred to plastic flasks (75 or 150 cm²; Falcon) and incubated at 37°C in a humid atmosphere of 5% CO₂. No serum is added to these overnight cultures, since its presence allows the cells to attach to the plastic, undergo proliferation, and dedifferentiate. Cells cultured under the above conditions were readily removed from the flasks by decanting, and show the same viability as freshly prepared cells.

### Measurement of Cytosolic Ca²⁺

Purified parathyroid cells were resuspended in 1.25 mM CaCl₂-2% BSA-PCB containing 1 µM fura-2-acetoxymethylester and incubated at 37°C for 20 min. The cells were then pelleted, resuspended in the same buffer lacking the ester, and incubated a further 15 min at 37°C. The cells were subsequently washed twice with PCB containing 0.5 mM CaCl₂ and 0.5% BSA and maintained at room temperature (about 20°C) . Immediately before use, the cells were diluted five-fold with prewarmed 0.5 mM CaCl₂-PCB to obtain a final BSA concentration of 0.1%. The concentration of cells in the cuvette used for fluorescence recording was 1-2 x 10⁶/ml.

The fluorescence of indicator-loaded cells was measured at 37°C in a spectrofluorimeter (Biomedical Instrumentation Group, University of Pennsylvania, Philadelphia, PA) equipped with a thermostated cuvette holder and magnetic stirrer using excitation and emission wavelengths of 340 and 510 nm, respectively. This fluorescence indicates the level of cytosolic Ca²⁺. Fluorescence signals were calibrated using digitonin (50 ug/ml, final) to obtain maximum fluorescence (Fₘₐₓ), and EGTA (10 mM, pH 8.3, final) to obtain minimal fluorescence (Fₘᵢₙ), and a dissociation constant of 224 nM. Leakage of dye is dependent on temperature and most occurs within the first 2 min after warming the cells in the cuvette; dye leakage increases only very slowly thereafter. To correct the calibration for dye leakage, cells were placed in the cuvette and stirred at 37°C for 2-3 min. The cell suspension was then removed, the cells pelleted, and the supernatant returned to a clean cuvette. The supernatant was then treated with digitonin and EGTA as above to obtain as estimate of dye leakage, which is typically 10-15% of the total Ca²⁺⁻dependent fluorescent signal. This estimate was subtracted from the apparent Fₘᵢₙ.

### Measurement of PTH Secretion

In most experiments, cells loaded with fura-2 were used in studies of PTH secretion. Loading parathyroid cells with fura-2 does not change their secretory response to extracellular Ca²⁺. Cells were suspended in PCB containing 0.5 mM CaCl₂ and 0.1% BSA. Incubations were performed in plastic tubes (Falcon 2058) containing 0.3 ml of the cell suspension with or without small volumes of CaCl₂ and/or organic polycations. After incubation at 37°C for various times (typically 30 min), the tubes were placed on ice and the cells pelleted at 2°C. Samples of the supernatant were brought to pH 4.5 with acetic acid and stored at -70°C. This protocol was used for both bovine and human parathyroid cells.

For bovine cells, the amount of PTH in sample supernatants was determined by a homologous radioimmunoassay using GW-1 antibody or its equivalent at a final dilution of 1/45,000. ¹²⁵I-PTH (65-84; INCSTAR, Stillwater, MN) was used as tracer and fractions separated by dextran-activated charcoal. Counting of samples and data reduction were performed on a Packard Cobra 5005 gamma counter.

For human cells, a commercially available radioimmunoassay kit (INS-PTH; Nichols Institute, Los Angeles, CA) which recognizes intact and N-terminal human PTH was used because GW-1 antibody recognizes human PTH poorly.

### Measurement of cyclic AMP

Cells were incubated as above for PTH secretion studies and at the end of incubation, a 0.15 ml sample was taken and transferred to 0.85 ml hot (70°C) water and heated at this temperature for 5-10 min. The tubes were subsequently frozen and thawed several times and the cellular debris sedimented by centrifugation. Portions of the supernatant were acetylated and cyclic AMP concentrations determined by radioimmunoassay.

### Measurement of Inositol Phosphate Formation

Membrane phospholipids were labeled by incubating parathyroid cells with 4 µCi/ml ³H-myo-inositol for 20-24 hrs. Cells were then washed and resuspended in PCB containing 0.5 mM CaCl₂ and 0.1% BSA. Incubations were performed in microfuge tubes in the absence or presence of various concentrations of organic polycation for different times. Reactions were terminated by the addition of 1 ml chloroform/methanol/12 N HCl (200:100:1; v/v/v). Phytic acid hydrolysate (200 µl; 25 µg phosphate/tube) water was then added. The tubes were centrifuged and 600 µl of the aqueous phase was diluted into 10 ml water.

Inositol phosphates were separated by ion exchange chromatography using AG1-X8 in either the chloride- or formate-form. When only IP₃ levels were to be determined, the chloride-form was used, whereas the formate form was used to resolve the major inositol phosphates (IP₃, IP₂, and IP₁). For determination of just IP₃, the diluted sample was applied to the chloride-form column and the column washed with 10 ml 30 mM HCl followed by 6 ml 90 mM HCl and the IP₃ eluted with 3 ml 500 mM HCl. The last eluate was diluted and counted. For determination of all major inositol phosphates, the diluted sample was applied to the formate-form column and IP₁, IP₂, and IP₃ eluted sequentially by increasing concentrations of formate buffer. The eluted samples from the formate columns were rotary evaporated, the residues brought up in cocktail, and counted.

The isomeric forms of IP₃ were evaluated by HPLC. The reactions were terminated by the addition of 1 ml 0.45 M perchloric acid and stored on ice for 10 min. Following centrifugation, the supernatant was adjusted to pH 7-8 with NaHCO₃. The extract was then applied to a Partisil SAX anion-exchange column and eluted with a linear gradient of ammonium formate. The various fractions were then desalted with Dowex followed by rotary evaporation prior to liquid scintillation counting in a Packard Tri-carb 1500 LSC.

For all inositol phosphate separation methods, appropriate controls using authentic standards were used to determine if organic polycations interfered with the separation. If so, the samples were treated with cation-exchange resin to remove the offending molecule prior to separation of inositol phosphates.

### Measurement of cytosolic Ca²⁺ in C-cells

Neoplastic C-cells derived from a rat medullary thyroid carcinoma (rMTC 6-23) obtained from American Type Culture Collection (ATCC No. 1607) were cultured as monolayers in Dulbecco's Modified Eagle's medium (DMEM) plus 15% horse serum in the absence of antibiotics. For measurements of [Ca²⁺]ᵢ, the cells were harvested with 0.02% EDTA/0.05% trypsin, washed twice with PCB containing 1.25 mM CaCl₂ and 0.5% BSA, and loaded with fura-2 as described above for parathyroid cells. Measurements of [Ca²⁺], were performed as described above with appropriate corrections for dye leakage.

### Measurement of [Ca²⁺]ᵢ in Rat Osteoclasts

Osteoclasts were obtained from 1-2 day old Sprague-Dawley rats using aseptic conditions. The rat pups were sacrificed by decapitation, the hind legs removed, and the femora rapidly freed of soft tissue and placed in prewarmed F-12/DMEM media (DMEM containing 10% fetal calf serum and antibiotics (penicillin-streptomycin-gentamicin; 100 U/ml-100 ug/ml-100 ug/ml)). The bones from two pups were cut lengthwise and placed in 1 ml culture medium. Bone cells were obtained by gentle trituration of the bone fragments with a plastic pipet and diluted with culture medium. The bone fragments were allowed to settle and equal portions (about 1 ml) of the medium transferred to a 6 well culture plate containing 25 mm glass coverslips. The cells were allowed to settle for 1 hr at 37°C in a humidified 5% CO₂-air atmosphere. The coverslips were then washed 3 times with fresh media to remove nonadherent cells. Measurements of [Ca²⁺]ᵢ in osteoclasts were performed within 6-8 hrs of removing nonadherent cells.

Cells attached to the coverslip were loaded with indo-1 by incubation with 5 µM indo-1 acetoxymethylester / 0.01% Pluronic F28 for 30 min at 37°C in F-12/DMEM lacking serum and containing instead 0.5% BSA. The coverslips were subsequently washed and incubated an additional 15 min at 37°C in F-12/DMEM lacking ester before being transferred to a superfusion chamber mounted on the stage of a Nikon Diaphot inverted microscope equipped for micro-fluorimetry. Osteoclasts were easily identified by their large size and presence of multiple nuclei. The cells were superfused with buffer (typically PCB containing 0.1% BSA and 1 mM Ca²⁺) at 1 ml/min with or without test substance. The fluorescence emitted by excitation at 340 nm was directed through the video port of the microscope onto a 440 nm dichroic mirror and fluorescence intensity at 495 and 405 nm collected by photomultiplier tubes. The outputs from the photomultiplier tubes were amplified, digitized, and stored in an 80386 PC. Ratios of fluorescence intensity were used to estimate [Ca²⁺]ᵢ.

### Oocyte Expression

In additional studies, Xenopus oocytes injected with mRNA from bovine or human parathyroid cells were used in screening protocols, and Cl⁻ conductance measured as an indirect means of monitoring increases in [ca²⁺]ᵢ. The following is an example to test the effect of neomycin.

Oocytes were injected with poly (A)⁺-enriched mRNA from human parathyroid tissue (hyperplastic glands from a case of secondary HPT). After 3 days, the oocytes were tested for their response to neomycin. Neomycin B evoked oscillatory increases in the Cl⁻ conductance which ceased upon superfusion with drug-free saline (see Fig. 20). Responses to neomycin B were observed at concentrations between 100 µM and 10 mM. To ensure that the response evoked by neomycin B was contingent upon injection of parathyroid mRNA, the effect of neomycin B on currents in water-injected oocytes was determined. In each of five oocytes examined, neomycin B (10 mM) failed to cause any change in the current. About 40% of oocytes are known to respond to carbachol, an effect mediated by an endogenous muscarinic receptor. In five oocytes examined, one showed inward currents in response to carbachol, and this is shown in the lower trace of Fig. 20. Thus, in cells expressing a muscarinic receptor coupled to increases in [Ca²⁺]ᵢ and Cl⁻ conductance, neomycin B fails to evoke a response. This shows that the response to neomycin B depends on expression of a specific protein encoded by parathyroid cell mRNA. It suggests quite strongly that in intact cells, neomycin B acts directly on the Ca²⁺ receptor to alter parathyroid cell function.

### Drug Design From Lead Molecules

Certain organic molecules mimic or antagonize the action of extracellular Ca²⁺ by acting at the Ca²⁺ receptor as shown herein. The molecules tested, however, are not necessarily suitable as drug candidates, but they serve to demonstrate that the hypothesis underlying Ca²⁺ receptor-based therapies is correct. These molecules can be used to determine the structural features that enable them to act on the Ca²⁺ receptor, and thus to select molecules useful in this invention.

An example of one such analytical procedure follows: This example is detailed in the examples below, but is used here to demonstrate the rationale that can be used to design useful molecules of this invention from lead molecules discussed herein. Those in the art will recognize the analytic steps defined in the example and that analogous analysis can be conducted on other lead molecules until the most desired calcimimetic or calcilytic is defined.

Other examples are also provided below. Together the data presented demonstrate that useful lead molecules will have aromatic groups which are preferably substituted at one or more positions, and may have branched or linear substitued or unsubstituted alkyl groups as desired. In addition, it is important to choose molecules of correct stereospecificity to ensure higher affinity for the desired Ca²⁺ receptor. These data thus point those in the art to appropriate lead molecules which can be derivatised to find optimum desired molecules of this invention, much as described below.

Although structurally diverse, molecules that are tested may have common features that can be studied. In this example, the correlation between net positive charge and potency in mobilizing intracellular Ca²⁺ was tested. Protamine (+21; EC₅₀ = 40 nM) was more effective than neomycin B (+6; EC₅₀ = 20 µM in human parathyroid cells and 40 µM in bovine parathyroid cells) which was more effective than spermine (+4; EC₅₀ = 150 µM) in causing the mobilization of [Ca²⁺]ᵢ in parathyroid cells. These results raise the question of whether positive charge alone determines potency, or if there are other structural features that contribute to activity on the Ca²⁺ receptor. This is important to determine at the outset because it profoundly impacts on the view that the Ca²⁺ receptor can be targeted with effective and specific therapeutic molecules. Thus, a variety of other organic polycations related to neomycin B and spermine can be studied to determine the relationship between the net positive charge of a molecule and its potency to mobilize intracellular Ca²⁺.

The first series of molecules studied were the aminoglycosides. The molecules were examined on bovine parathyroid cells and their EC₅₀'s for the mobilization of intracellular Ca²⁺ determined. For the aminoglycosides, the rank order of potency for eliciting cytosolic Ca²⁺ transients was neomycin B (EC₅₀ = 20 or 40 µM) > gentamicin (150 µM) > bekanamycin (200 µM) > streptomycin (600 µM). Kanamycin and lincomycin were without effect when tested at a concentration of 500 µM. The net positive charge on these aminoglycosides at pH 7.3 is neomycin B (+6) > gentamicin (+5) = bekanamycin (+5) > kanamycin (average +4.5) > streptomycin (+3) > lincomycin (+1). Within the aminoglycoside series, then, there is some correlation between net positive charge but it is not absolute, and kanamycin, which would be predicted to be more potent than streptomycin, is without activity.

Testing of various polyamines revealed additional and more marked discrepancies between net positive charge and potency. Three structural classes of polyamines were examined: (1) straight chain, (2) branched chain, and (3) cyclic. The structures of the polyamines tested are provided in Fig. 1. Amongst the straight chain polyamines, spermine (+4; EC₅₀ = 150 µM) was more potent than pentaethylenehexamine (+6; EC₅₀ = 500 µM) and tetraethylenepentamine (+5; EC₅₀= 2.5 mM) even though the latter molecules have a greater net positive charge.

We synthesized some branched chain polyamines that have different numbers of secondary and primary amino groups and thus vary in net positive charge. Two of these molecules, NPS 381 and NPS 382, were examined for effects on [Ca²⁺]ᵢ in bovine parathyroid cells. NPS 382 (+8; EC₅₀ = 50 µM) was about twice as potent as NPS 381 (+10; EC₅₀ = 100 µM) even though it contains two fewer positive charges.

A similar discrepancy between positive charge and potency was noted in experiments with cyclic polyamines. For example, hexacyclen (+6; EC₅₀ = 20 µM) was more potent than NPS 383 (+8; EC₅₀ = 150 µM). The results obtained with these polyamines show that positive charge is not the sole factor contributing to potency.

Additional studies provided insights into the structural features of molecules that impart activity on the parathyroid cell Ca²⁺ receptor. One of the structurally important features is the intramolecular distance between the nitrogens (which carry the positive charge). Thus, spermine is 50-fold more potent than triethylenetetramine (EC₅₀ = 8 mM) in evoking increases in [Ca²⁺]ᵢ in bovine parathyroid cells yet both molecules carry a net positive charge of +4. The only difference in structure between these two polyamines is the number of methylenes separating the nitrogens: in spermine it is 3-4-3 whereas in triethylenetetramine it is 2-2-2. This seemingly minor change in the spacing between nitrogens has profound implications for potency and suggests that the conformational relationships of nitrogens within the molecule are critical. Supporting this are results obtained with hexacyclen and pentaethylenehexamine. The former molecule is simply the cyclic analog of the latter and contains the same number of methylenes between all nitrogens, yet the presence of the ring structure increases potency 25-fold. These results indicate that positive charge per se is not the critical factor determining the activity of an organic molecule on the Ca²⁺ receptor.

Another series of experiments reveals the importance of aromatic groups in determining activity on the Ca²⁺ receptor. The results were obtained with two arylalkylamines isolated from the venom of the spider Argiope lobata. These molecules, argiotoxin 636 and argiotoxin 659, have identical polycationic portions linked to different aromatic groups (Fig. 24). Argiotoxin 659 evoked transient increases in [Ca²⁺]ᵢ in bovine parathyroid cells when tested at concentrations of 100 to 300 *µ*M. In contrast, argiotoxin 636 was without effect when tested at similar concentrations (Fig. 24). The only difference in structure between these two arylalkylamines is in the aromatic portion of the molecules: argiotoxin 659 contains a 4-hydroxyindole moiety whereas argiotoxin 636 contains a 2,4-dihydroxyphenyl group. The net positive charge on these two arylalkylamines is the same (+4), so their different potencies must result from the different aromatic groups. This shows that net positive charge alone does not determine potency. The real importance of these findings, however, is the discovery that aromatic groups contribute significantly to the ability of molecules to activate the Ca²⁺ receptor.

Agatoxin 489 (NPS 017) and Agatoxin 505 (NPS 015) both cause the mobilization of intracellular Ca²⁺ in parathyroid cells with EC₅₀'s of 6 and 22 *µ*M, respectively. The only difference in the structure of these molecules is a hydroxyl group on the indole moiety (Fig. 1). This shows that substitutions on the aromatic region of the molecule can influence potency. This indicates that further lead molecules to be studied will include those molecules having substituted aromatic moieties.

The structural features to be varied systematically from lead molecules described herein include (1) net positive charge, (2) number of methylenes separating nitrogens, and (3) cyclic versions of, e.g., polyamines, with and without changes in methylene spacing and net positive charge. In addition systematic variations in the structure and location of aromatic groups can be examined, e.g., in a variety of arylalkylamines isolated from the venoms of wasps and spiders; and synthetic molecules can be prepared by the coupling of commercially available aromatic moieties to the argiotoxin polyamine moiety. The argiotoxin polyamine moiety can be readily coupled to any aromatic moiety containing a carboxylic acid. Thus, it is simple to systematically screen the hydroxy and methoxy derivatives of phenylacetic acid and benzoic acid as well as the hydroxyindoleacetic acid series. Analogs containing heteroaromatic functionalities can also be prepared and assessed for activity.

Comparisons of potency and efficacy among such molecules will reveal the optimal structure and location of the aromatic group at a constant positive charge.

One of the structural variations on the polyamine motif that seems to increase potency is the presence of the cyclic version of the straight chain-parent molecule. Budmunchiamine A, isolated from the plant Albizia amara, is a cyclic derivative of spermine (Fig. 1). The addition of budmunchiamine A to bovine parathyroid cells caused a rapid and transient increase in [Ca²⁺]ᵢ that persisted in the absence of extracellular Ca²⁺ and was blunted by pretreatment with PMA. It therefore causes the mobilization of intracellular Ca²⁺ in parathyroid cells, probably by acting on the Ca²⁺ receptor. It is about equipotent with spermine (EC₅₀ about 200 *µ*M) yet carries one less positive charge (+3) than does spermine.

The results obtained with budmunchiamine A demonstrate the predictive power of the structure-activity studies and the novel structural information to be gained by testing natural products. Thus, screening of natural products, selected rationally on the basis of the structural information is readily performed e.g., molecules can be selected on the basis of well-established chemotaxonomic principles using appropriate data bases, such as Napralert. For example, macrocyclic polyamine alkaloids derived from papilionoid legumes related to Albizia, such as Pithecolobium and other plant-derived molecules can be screened.

Fig. 36 provides a second example of a series of molecules which were screened to determine useful molecules of this invention. These molecules were generally derived from fendiline and tested to determine their respective EC₅₀'s. Moreover, testing of related molecules, such as NPS 447 and NPS 448 reveals stereospecific effects of molecule structure. The most active compounds tested to date are the novel compounds designated NPS 467 and NPS 568 which have EC₅₀ values of less than 5µM. Those in the art, by reviewing this series of molecules, can determine other suitable derivatives which can be tested in the invention.

These examples demonstrate the general design and screening process useful in this invention, and indicate that additional compound and natural product libraries can be screened as desired by those in the art to determine other useful lead molecules or novel molecules of this invention.

As discussed above, examples of molecules useful as calcimimetics include branched or cyclic polyamines, positively charged polyamino acids, and arylalkylamines. In addition, other positively charged organic molecules, including naturally occurring molecules and their analogs, are useful calcimimetics. These naturally occurring molecules and their analogs preferably have positive charge-to-mass ratios that correlate with those ratios for the molecules exemplified herein. (Examples include material isolated from marine animals, arthropod venoms, terrestrial plants and fermentation broths derived from bacteria and fungi.) It is contemplated that one group of preferred naturally occurring molecules and analogs useful as calcimimetics will have a ratio of positive charge: molecular weight (in daltons) from about 1:40 to 1:200, preferably from about 1:40 to 1:100. More specific examples of such molecules are provided below.

### Polyamines

The polyamines useful as calcimimetics in this invention may be either branched or cyclic. Branched or cyclic polyamines potentially have higher calcimimetic activity than their straight-chain analogs. That is, branched or cyclic polyamines tend to have a lower EC₅₀ than their corresponding linear polyamines with the same effective charge at physiological pH (see Table 1).

**Table 1**

| Molecule | Net (+) Charge | EC₅₀ (µM) |
|---|---|---|
| Neomycin | +6 | 20 or 40 |
| Hexacyclen | +6 | 20 |
| NPS 382 | +8 | 50 |
| NPS 381 | +10 | 100 |
| NPS 383 | +8 | 150 |
| Gentamicin | +5 | 150 |
| Spermine | +4 | 150 |
| Bekanamycin | +5 | 200 |
| Argiotoxin-659 | +4 | 300 |
| Pentaethylenehexamine (PEHA) | +6 | 500 |
| Streptomycin | +3 | 600 |
| Spermidine | +3 | 2000 |
| Tetraethylenepentamine (TEPA) | +5 | 2500 |
| 1,12-diaminododecane (DADD) | +2 | 3000 |
| Triethylenetramine (TETA) | +4 | 8000 |

"Branched polyamines" as used herein refers to a chain molecule consisting of short alkyl bridges or alkyl groups joined together by amino linkages, and also containing points at which the chain branches. These "branch points" can be located at either a carbon atom or a nitrogen atom, preferably at a nitrogen atom. A nitrogen atom branch point is typically a tertiary amine but it may also be quaternary. A branched polyamine may have 1 to 20 branch points, preferably 1 to 10 branch points.

Generally, the alkyl bridges and alkyl branches in a branched polyamine are from 1 to 50 carbon atoms in length, preferably from 2 to 6 carbon atoms. The alkyl branches may also be interrupted by one or more heteroatoms (nitrogen, oxygen or sulfur) or substituted with functional groups such as: halo, including fluoro, chloro, bromo, or iodo; hydroxy; nitro; acyloxy (R'COO-), acylamido (R'CONH-), or alkoxy (-OR'), where R' may contain from 1 to 4 carbon atoms. The alkyl branches may also be substituted with groups that are positively charged at physiological pH, such as amino or guanido. These functional substituents may add or change physical properties such as solubility to increase activity, delivery or bioavailability of the molecules.

The branched polyamines may have three or more chain and branch termination points. These termination points may be methyl groups or amino groups, preferably amino groups.

One preferred group of molecules is the group of branched polyamines having the formula:

H₂N-(CH₂)ⱼ-(NRᵢ-(CH₂)ⱼ)ₖ-NH₂

where k is an integer from 1 to 10, each j is the same or different and is an integer from 2 to 20, and each Rᵢ is the same or different and is selected from the group consisting of hydrogen and - (CH₂)ⱼ-NH₂, where j is as defined above, and at least one Rᵢ is not hydrogen.

Particularly preferred branched polyamines of this invention are the molecules N¹,N¹,N⁵,N¹⁰,N¹⁴,N¹⁴-hexakis-(3-aminopropyl)spermine and N¹,N¹,N⁵,N¹⁴,N¹⁴-tetrakis-(3-aminopropyl)spermine referred to as NPS 381 and NPS 382, respectively, in Figure 1.

"Cyclic polyamines" as used herein refer to heterocycles containing two or more heteroatoms (nitrogen, oxygen or sulfur), at least two of which are nitrogen atoms. The heterocycles are generally from about 6 to about 20 atoms in circumference, preferably from about 10 to about 18 atoms in circumference. The nitrogen heteroatoms are separated by 2 to 10 carbon atoms. The heterocycles may also be substituted at the nitrogen sites with aminoalkyl or aminoaryl groups (NH₂R-), wherein R is aminoaryl or a lower alkyl of 2 to 6 carbon atoms.

Particularly preferred cyclic polyamines of this invention are shown in Figure 1 as hexacyclen (1,4,7,10, 13,16-hexaaza-cyclooctadecane) and NPS 383.

### Polyamino Acids

The polyamino acids useful in this invention may contain two or more positively charged amino acid residues at physiological pH. These positively charged amino acids include histidine, lysine and arginine. These polypeptides will vary in length from 2 to 800 amino acids in length, more preferably from 20 to 300 amino acids in length. These polypeptides may consist of a single repeating amino acid residue, or may have the variety of a naturally occurring protein or enzyme.

The amino acid residues comprising the polyamino acids may be any of the twenty naturally occurring amino acids, or other alternative residues. Alternative residues include, for example, the ω-amino acids of the formula H₂N(CH₂)ₙCOOH, where n is from 2 to 6. These are neutral, nonpolar amino acids, as are sarcosine, t-butyl alanine, t-butyl glycine, N-methyl isoleucine, norleucine, phenyl glycine, citrulline, methionine sulfoxide, cyclohexyl alanine, and hydroxyproline. Ornithine is an alternative positively charged amino acid residue. The polyamino acids of this invention may also be chemically derivatized by known methods.

Particularly preferred polyamino acids of this invention include polyarginine, polylysine, and poly(argininyltyrosine), having 20-300 residues. Another preferred polyamino acid is protamine, or a protamine analog.

### Arylalkylamines

"Arylalkylamines" as used herein refer to a class of positively-charged toxins derived from arthropod venoms. Preferred arylalkylamines of this invention include philanthotoxin-433, argiotoxin-636, and argiotoxin-659, agatoxin 505, agatoxin 489, the structures of which are shown in Figure 1, and other synthetic molecules modeled after these natural products, such as NPS 019.

### Additional Components

Compositions of this invention include not only at least one calcimimetic or calcilytic, but may also include certain additional components. These additional components include targeting components, labels, and other functionalities which may be useful in the applications herein, e.g., for screening for agonists or antagonists of extracellular Ca²⁺.

For example, an immunoglobulin or portion thereof, or a ligand specific for parathyroid cells or a Ca²⁺ receptor can be used as a target-specific component. The immunoglobulin can be a polyclonal or monoclonal antibody and may comprise whole antibodies or immunologically reactive fragments of these antibodies such as F_{ab},, F_{ab}, or (F_{ab},)₂. Receptor-specific ligands may also be used.

The compositions of this invention may also contain components derivatized with a molecule or ion which acts as a label. A wide variety of labeling moieties can be used, including radioisotopes, chromophores, and fluorescent labels. Radioisotope labeling in particular can be readily detected in vivo. Radioisotopes may be coupled by coordination as cations in the porphyrin system. Useful cations include technetium, gallium, and indium. In the compositions, the positively charged molecule can be linked to or associated with a label.

### Methods of Synthesis

Strategies for the syntheses and the modification of polyamines involve the use of a variety of amine protecting groups (phthalimido, BOC, CBZ, benzyl, and nitrile) which can be removed selectively to construct functionalized molecules. The synthetic methods involved are modelled after those used to construct argiopines 636 and 659 and other arylalkylamines derived from spider venoms.

Chain extensions of 2-4 methylenes were typically accomplished by alkylation with the corresponding N-(bromoalkyl)phthalimide. A 1:1.2 mixture of amine to the bromoalkylphthalimide was refluxed in acetonitrile in the presence of 50% KF on Celite. Chain extensions were also accomplished by alkylation of a given amine with acrylonitrile or ethylacrylate. Reaction progress was monitored by TLC and intermediates purified on silica gel using combinations of dichloromethane, methanol, and isopropylamine. Final products were purified by cation exchange (HEMA-SB) and RP-HPLC (Vydac C-18). Purity and structure verification are accomplished by ¹H- and ¹³C-NMR and high-resolution mass spectrometry (EI, CI and/or FAB).

BOC protecting groups were added by the treatment of an amine (1° or 2°) with di-tert-butyl dicarbonate in dichloromethane in the presence of a catalytic amount of dimethylaminopyridine. Benzyl protecting groups were applied in one of two ways: (1) condensation of a 1° amine with benzaldehyde followed by sodium borohydride reduction or (2) alkylation of a 2° amine with benzylbromide in the presence of KF. Amide linkages and cyclizations were typically performed by the reaction of an amine (1° or 2°) with the N-hydroxysuccinimide ester of a given acid. This was accomplished directly (in the case of cyclizations) by treatment of the "amino acid" with dicyclohexylcarbodiimide under dilute conditions.

Deprotections of the phthalimido functionality were accomplished by reduction with hydrazine in refluxing methanol. Deprotections of the BOC functionality were accomplished in anhydrous TFA. Deprotection of benzyl, nitrile, and CBZ protecting functionalities was accomplished by reduction in glacial acetic acid under 55 psi hydrogen in the presence of a catalytic amount of palladium hydroxide on carbon. Nitrile functionalities (in the presence of benzyl and CBZ groups) were selectively reduced under hydrogen in the presence of sponge Raney nickel.

Specifically, branched polyamines are typically prepared from simple diaminoalkanes of the formula NH₂₋(CB₂)ₙ-NH₂, or simple polyamines such as spermidine or spermine. One of the two primary (terminal) amines is protected or "masked" with a protecting group such as BOC (t-butyloxycarbonyl), phthalimido, benzyl, 2-ethylnitrile (the Michael condensation production product of an amine and acrylonitrile), or amide. A typical reaction is the addition of a BOC protecting group by treatment with di-t-butyl-dicarbonate (BOC anhydride): 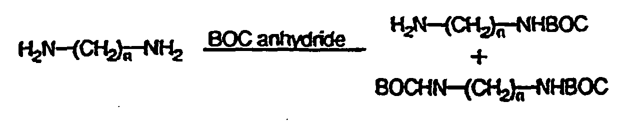 The monoprotected product is separated from the unprotected and diprotected products by simple chromatographic or distillation techniques.

The remaining free amine in the monoprotected product is then selectively alkylated (or acylated) with an alkylating (or acylating) agent. To ensure mono-alkylation, the free amine is partially protected by condensation with benzaldehyde followed by sodium borohydride reduction to form the N-benzyl derivative: 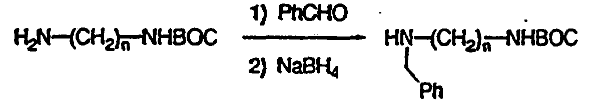 The N-benzyl derivative is then reacted with the alkylating agent. A typical alkylating agent is in an N-(bromoalkyl)phthalimide, which reacts as follows: 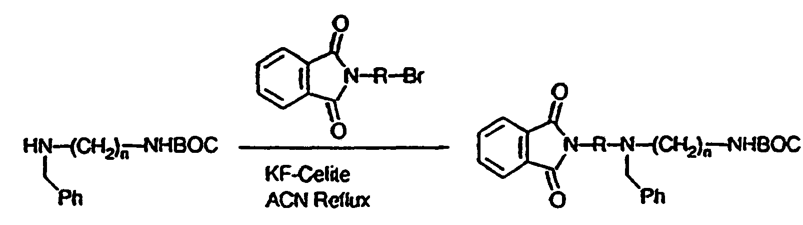 For example, N-(bromobutyl)phthalimide is used to extend or branch the chain with four methylene units. Alternatively, reaction with acrylonitrile followed by reduction of the cyano group will extend the chain by three methylenes and an amino group.

The protecting groups of the resulting chain-extended molecule can then be selectively cleaved to yield a new free amine. For example, trifluoroacetic acid is used to remove a BOC group; catalytic hydrogenation is used to reduce a nitrile functionality and remove a benzyl group; and hydrazine is used to remove phthalimido groups as follows:

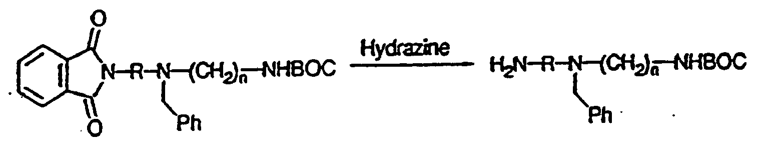

The new free amine may be alkylated (or acylated) further as above to increase the length of the polyamine. This process is repeated until the desired chain length and number of branches is obtained. In the final step, deprotection of the product results in the desired polyamine. However, further modifications may be effected at the protected end prior to deprotection in the following manner:

For example, prior to BOC-deprotection, the polyamine is acylated with the N-hydroxysuccinimide ester of 3,4-dimethoxyphenylacetic acid to yield a diprotected polyamine: 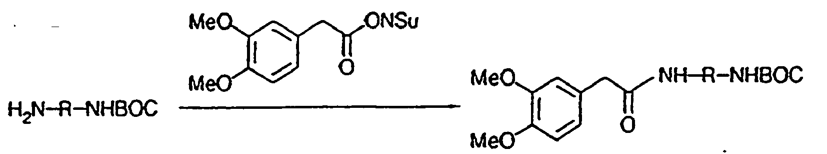 This ultimately will yield an aryl polyamine. The BOC group can then be selectively removed with trifluoroacetic acid to expose the other amino terminus which can be extended as above.

Certain branched polyamines may be formed by simultaneously alkylating or acylating the free primary and secondary amines in a polyamine formed as above. For example, treatment of spermine with excess acrylonitrile followed by catalytic reduction yields the following:

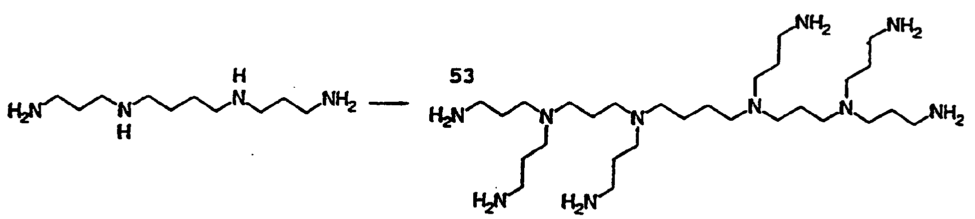

Cyclic polyamines may be prepared as above beginning with starting materials such as hexacylen (Aldrich Chem.).

The polyamino acids within the scope of the present invention can be made by recombinant techniques known in the art, or may be synthesized using standard solid-phase techniques known in the art. Solid-phase synthesis is commenced from the carboxy-terminal end of the peptide using an α-amino protected amino acid. BOC protective groups can be used for all amino groups even through other protective groups are suitable. For example, BOC-lys-OH can be esterified to chloromethylated polystyrene resin supports. The polystyrene resin support is preferably a copolymer of styrene with about 0.5 to 2% divinylbenzene as a cross-linking agent which causes the polystyrene polymer to be completely insoluble in certain organic solvents. See Stewart et al., Solid-Phase Peptide Synthesis (1969), W.H. Freeman Co., San Francisco; and Merrifield, J. Am. Chem. Soc. (1963) 85:2149-2154. These and other methods of peptide synthesis are also exemplified by U.S. Patent Nos. 3,862,925; 3,842,067; 3,972,859; and 4,105,602.

The polypeptide synthesis may use manual techniques or automatically employing, for example, an Applied Biosystems 403A Peptide Synthesizer (Foster City, California) or a Biosearch SAM II automatic peptide synthesizer (Biosearch, Inc., San Rafael, California), following the instructions provided in the instruction manual supplied by the manufacturer.

The arylalkylamines of the invention are natural products isolated by known techniques, or synthesized as described in Jasys et al., Tetrahedron Lett. (1988) 29 : 6223-6226, and Nason et al., Tetrahedron Lett. (1989) 30 : 2337-2340.

One general protocol for preparation of fendiline (or fendiline analogs shown in Fig. 36) is as follows. In a 10 ml round bottom blask equipped with a magnetic stir bar and rubber septum, 1.0 mmole 3,3'-bisphenylpropylamine (or primary alkyl amine) in 2 ml ethanol was treated with 1.1 mmole phenol and 1.0 mmole acetophenone (or substituted acetophenone). To this was added 2.0 mmoles MgSO₄ and 1.0 mmole NaCNBH₃. This was stirred under a nitrogen atmosphere at room temperature (about 20°C) for 24 hrs. The reaction was poured into 50 ml ether and washed 3 times with 1 N NaOH and once with brine. The ether layer was dried with anhydrous K₂CO₃ and reduced in vacuo. The product was then purified by column chromatography or HPLC incorporating silica stationary phase with combinations of CH₂Cl₂-Methanol-isopropylazine (typically 3% Methanol and 0.1% isopropylamine in methylene chloride).

A preferred procedure for preparing fendiline or fendiline analogs (such as those depicted in Figure 36) uses titanium(IV) isopropoxide and was modified from methods described in J. Org. Chem. 55:2552 (1990). For the synthesis of NPS 544, titanium tetrachloride (method described in Tetrahedron Letters 31:5547 (1990)) was used in place of titanium(IV) isopropoxide. The reaction scheme is depicted in Figure 43a. In Figure 43a, R,R' and R" depict hydrocarbyl groups. According to one embodiment, in a 4 ml vial, 1 mmole of amine (1) (typically a primary amine) and 1 mmole ketone or aldehyde (2) (generally acetophenone) are mixed, then treated with 1.25 mmoles titanium(IV) isopropoxide (3) and allowed to stand with occasional stirring at room temperature for about 30 minutes. Alternatively, a secondary amine may be used in place of (1). Note: some reactions will give heavy precipitates or solids which are warmed/heated (to their melting point) to allow for stirring/mixing several times over the course of the reaction. The reaction mixture is treated with 1 ml ethanol containing 1 mmole sodium cyanoborohydride (4) and the resulting mixture is then allowed to stand at room temperature with occasional stirring for about 16 hours. After this time the reaction is quenched by the addition of about 500 µl water. The reaction mixture is then diluted to about 4 ml total volume with ethyl ether and then centrifuged. The upper organic phase is removed and reduced on a rotovapor. The resulting product, (6), is partially purified by chromatography through a short column of silica (or alternatively by using preparative TLC on silica) using combination of dichloromethane:methanol:isopropylamine (typically 95:5:0.1), prior to purification by HPLC (normal phase using silica with dichloromethane:methanol:isopropylamine or reversed phase, C-18 with 0.1% TFA with acetonitrile or methanol).

If appropriate or desired, chiral resolution may be accomplished using methods such as those described in Example 21.

### Formulation and Administration

As demonstrated herein, the molecules of the invention may be used to: (a) mimic or antagonize one or more of the effect of extracellular Ca²⁺; (b) affect the extracellular free Ca²⁺ level in an individual; and (c) treat diseases such as hyperparathyroidism, osteoporosis and hypertension. While the molecules have generally been shown to have an effect on parathyroid cells, they may also modulate the Ca²⁺ receptors on other cells, including bone osteoclasts, juxtaglomerular kidney cells, proximal tubule kidney cells, keratinocytes, parafollicular thyroid cells, and placental trophoblasts.

While these molecules will typically be used in therapy for human patients, they may be used to treat similar or identical diseases in other warm-blooded animal species such as other primates, farm animals such as swine, cattle, and poultry; and sports animals and pets such as horses, dogs and cats.

In therapeutic and/or diagnostic applications, the molecules of the invention can be formulated for a variety of modes of administration, including systemic and topical or localized administration. Techniques and formulations generally may be found in Remington's Pharmaceutical Sciences, Mack Publishing Co., Easton, PA.

For systemic administration, oral administration is preferred. Alternatively, injection may be used, e.g., intramuscular, intravenous, intraperitoneal, and subcutaneous. For injection, the molecules of the invention are formulated in liquid solutions, preferably in physiologically compatible buffers such as Hank's solution or Ringer's solution. In addition, the molecules may be formulated in solid form and redissolved or suspended immediately prior to use. Lyophilized forms are also included.

Systemic administration can also be by transmucosal or transdermal means, or the molecules can be administrated orally. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays, for example, or using suppositories. For oral administration, the molecules are formulated into conventional oral administration forms such as capsules, tablets, and tonics.

For topical administration, the molecules of the invention are formulated into ointments, salves, gels, or creams, as is generally known in the art.

As shown in the examples below, the amounts of various compounds of this invention which must be administered can be determined by standard procedure. Generally it is an amount between about 1 and 50 mg/kg animal to be treated.

### Recombinant Ca²⁺ Receptors

Natural product screening has traditionally provided the lead structures for the development of diverse therapeutic molecules. However, high-throughput screening of natural product libraries or other molecule libraries for activity on the Ca²⁺ receptor has not previously been possible. To achieve this capability, it is best to clone the Ca²⁺ receptor cDNA and then create transfected cell lines suitable for high-throughput screening. The structure of the receptor can additionally be used to gain insight into the molecular geometry of the ligand binding site(s), and such information used to augment a rational drug design program as discussed above. Limited structure-activity studies and testing of selected natural product molecules will provide the initial structural data base necessary to guide rational natural product screening and drug design.

The bovine and human parathyroid cell Ca²⁺ receptor cDNA can be cloned by functional expression in Xenopus oocytes. It is possible to monitor an increase in intracellular Ca²⁺ in Xenopus oocytes indirectly by measuring current through the endogenous Ca²⁺-activated Cl⁻ channel. The amplification of the response afforded by this signal transduction pathway enables the detection of receptor proteins encoded by mRNA at very low levels. This allows the detection of receptor-specific cDNA clones without the need for high affinity ligands, specific antisera, or protein or nucleic acid sequence information. An example of such a procedure follows.

Adult female Xenopus laevis were obtained from Xenopus I (Ann Arbor, MI) and maintained according to standard procedures. Lobes of ovary were excised from hypothermically-anesthetized toads. Clusters of oocytes were transferred into modified Barth's saline (MBS). Individual oocytes were obtained by incubation in MBS containing 2 mg/ml collagenase (Sigma, Type 1A) for 2h at 21°C and stage V-VI oocytes were selected for injection.

Glass capillary tubes (1 mm diameter) were pulled to a fine tip and manually broken to achieve a tip diameter of about 15 µM. A droplet of mRNA (1 ng/nl in diethylpyrocarbonate (DEPC)-treated water) was placed onto para-film and drawn into the capillary tube by suction. The capillary tube was then connected to a picospritzer (WPI Instruments) and the volume of the air-pulsed droplets adjusted to deliver 50 ng of mRNA (typically 50 nl). A 35 mm culture dish with a patch of nylon stocking fixed to the bottom was used to secure the oocytes during injection of mRNA into the vegetal pole. The injected oocytes were placed into a 35 mm culture dish containing MBS, 100 ug/ml penicillin and 100 U/ml streptomycin and incubated at 18°C for 3 days.

Following incubation, an oocyte was placed into a 100 µl plastic chamber and superfused with MBS at a flow rate of 0.5 ml/min using a peristaltic pump. Test molecules or inorganic polycations were added by rapidly moving the tubing into different buffers. Recording and current-passing electrodes were constructed from thin wall capillary tubing pulled to a resistance of 1-3 Mohms and filled with 3 M KCl. Oocytes were impaled (in the animal pole) with both electrodes under microscopic observation and connected to an Axon Instruments Axoclamp 2A voltage-clamp amplifier which was used to set the holding potential (-70 to -80 mV) and to measure the currents that were passed to maintain the holding potential. Currents were recorded directly onto a strip chart recorder.

For mRNA preparation, tissue was obtained from calves or patients with secondary HPT undergoing surgical removal of the parathyroid glands. Purified cells need not be prepared; whole pieces of gland were used to prepare mRNA that directs the expression of the Ca²⁺ receptor in Xenopus oocytes. Total cellular RNA was prepared by acid guanidinium thiocyanate/phenol extraction of homogenized glands. Oligo-dT cellulose chromatography was used to select poly(A)⁺ mRNA by standard procedures. For size fractionation of mRNA, centrifugation through glycerol gradients was used. The mRNA was denatured with 20 mM methylmercuric hydroxide and loaded (50-100 ug at a concentration of 1 mg/ml) onto a linear 15-30% glycerol gradient prepared in Beckman TLS55 tubes. Following centrifugation at 34,000 rpm for 16 hrs, 0.3 ml gradient fractions were collected and diluted in an equal volume of water containing 5mM *beta*-mercaptoethanol. mRNA was then recovered by two cycles of ethanol precipitation. If desired, the mRNA (50-100 ug of poly(A)⁺) may be separated on a 1.2% agarose/6.0 M urea preparative gel, along with a range of RNA size markers. Following visualization of the mRNA by ethidium bromide staining, gel slices containing RNA in -1.5-2.0 kb size steps are excised. mRNA is recovered from the agarose gel slices using RNAid binding matrix (according to the supplier's standard protocol; Stratagene, Inc.) and recovered mRNA fractions eluted into DEPC-treated water.

Amounts of recovered mRNA were quantified by UV absorbance measurement. The size range of mRNA contained within each fraction was determined by formaldehyde/agarose gel electrophoresis using a small quantity (0.5 ug) of each sample. The integrity of the mRNA was assessed by in vitro translation of each sample. Reticulocyte lysates (commercially available kits; BRL) were used to translate 0.05-0.5 ug of each mRNA fraction. The resulting ³⁵S-labelled proteins were analyzed by SDS-PAGE. The intact mRNA was capable of directing the synthesis of proteins of a complete size range, corresponding roughly to the sizes of the individual mRNA fractions.

A cDNA library was constructed in the vector λ ZAPII, following modifications of the technique of Gubler and Hoffman. RNA from the fraction(s) giving the best response in the oocyte assay was used as starting material. First-strand cDNA syntheses was primed with an oligo-dT/NotI primer-linker. Second-strand synthesis was by the RNase H/DNA Polymerase I self-priming method. Double-stranded cDNA was blunted with T4 DNA polymerase and EcoRI adaptors blunt-end ligated to the cDNA with T4 ligase. Following NotI digestion to cleave the linker, full-length cDNA was size-selected by exclusion chromatography on Sephacryl 500 HA. First-strand cDNA was radiolabeled with α-³²P-dATP, and all synthesis and recovery steps monitored by following the incorporation of radioactivity. Full-length cDNA recovered from the sizing column was ligated to EcoRI/NotI digested λ ZAPII arms. The ligation mix was test packaged with commercially available high efficiency packaging extract (Stratagene, Inc.) and plated on the appropriate host strain (XL1-blue). The percentage of recombinant phage was determined by the ratio of white to blue plaques when the library is plated on IPTG and X-gal.

The average insert size was determined from ten randomly selected clones. Phage DNA "mini-preps" were digested with EcoRI and NotI to release the insert, and the size determined by agarose gel electrophoresis. The library consisted of >90% recombinant phage, and the insert size ranged from 1.5 to 4.2 kb. The recombinant ligation was packaged in large scale to generate 800,000 primary clones. The packaging mix was titered and plated at 50,000 plaques per 15 cm plate. Each pool of 50,000 clones was eluted in SM buffer and stored individually.

Plate lysate stocks of each of the clone pools were used for small scale phage DNA preparation. Phage particles are concentrated by polyethylene glycol precipitation, and phage DNA purified by proteinase K digestion followed by phenol:chloroform extraction. Twenty micrograms of DNA are digested with NotI, and used as template for in vitro transcription of sense-strand RNA. In vitro transcription is according to standard protocols, utilizing T7 RNA polymerase and 5' cap analog m⁷GpppG in a 50µl total reaction volume. Following Dnase I/Proteinase K digestion and phenol/chloroform extraction, the RNA is concentrated by ethanol precipitation and used for oocyte injection.

Oocytes were injected with synthetic mRNA (cRNA) from each of the 16 library subpools constituting 50,000 independent clones each. After incubation for 3 to 4 days, oocytes were assayed for the ability of 10 mM neomycin to elicit a Ca²⁺ dependent Cl-current. A pool designated 6 gave a positive signal and thus contains a cDNA clone encoding a functional calcium receptor. In order to decrease the complexity of pool 6 and thus proceed towards the purification of the calcium receptor clone contained within this pool, pool 6 phage were replated at -20,000 plaques per plate and 12 plates harvested. DNA was prepared from each of these subpools and cRNA synthesized. Again, oocytes were injected with cRNA and assayed 3-4 days later for the ability of 10 mM neomycin to elicit a Ca²⁺ dependent Cl-current. A subpool 6-3 was positive and this pool was subjected to a further round of plating reducing the complexity of pools to around 5,000 clones per pool. Pools were again assayed by preparation of cRNA and injection in oocytes. A subpool 6-3.4 was positive. In order to expedite further purification of the positive clone in pool 6-3.4, phage DNA from this pool was rescued as plasmid DNA by superinfection with the helper phage, ExAssist. Transfection of rescued plasmids into bacterial strain DH5alphaF' resulted in transformed bacterial colonies on ampicillin plates. These were harvested in pool of 900 clones each. Plasmid DNA was then prepared from each subpool and cRNA synthesized and assayed in the usual manner. Subpool 6-3.4.4 was positive. Bacteria containing the plasmid subpool 6-3.4.4 were subsequently plated in subpools of ^{∼}50 clones each. Continuation of this process is expected to result in a single clone encoding a functional calcium receptor.

Initial experiments used Xenopus oocytes injected with water or poly(A)⁺-enriched mRNA (50 ng) from bovine parathyroid cells. After three days, the oocytes were examined for their ability to increase intracellular Ca²⁺ in response to increases in the concentration of extracellular di- and trivalent cations. The oocytes were impaled with recording and current-passing electrodes and [Ca²⁺]ᵢ was assessed indirectly by measuring currents through the endogenous Ca²⁺-activated Cl⁻ channel. In oocytes injected with poly(A)⁺-enriched mRNA from bovine (or human, Fig. 26) parathyroid cells, increasing the concentration of extracellular Ca²⁺ from 0.7 to 3, 5 or 10 mM caused a rapid and transient increase in the Cl⁻ conductance which then oscillated around a higher basal conductance. Increasing the concentration of extracellular Mg²⁺ from 1 to 10 mM likewise evoked oscillatory increases in Cl⁻ conductance. The Cl⁻ conductance response to extracellular Mg²⁺ persisted when the extracellular Ca²⁺ concentration was reduced to < 1 µM (Fig. 25).

The impermeant trivalent cation Gd³⁺ (600 µM) also caused oscillatory increases in the Cl⁻ conductance (Fig. 25). Such increases in the Cl⁻ conductance which oscillate and persist in the nominal absence of extracellular Ca²⁺ are noted when oocytes have been allowed to express other Ca²⁺-mobilizing receptors and are stimulated with the appropriate ligand (e.g., substance K, Fig. 25). In these instances, the increase in Cl⁻ conductance reflects the mobilization of intracellular Ca²⁺. These initial studies likewise show that extracellular polycations mobilize intracellular Ca²⁺ in parathyroid cell mRNA-injected oocytes.

Oocytes injected with water did not show any change in the Cl⁻ current when exposed to extracellular Ca²⁺ (10 mM) or Mg²⁺ (20 or 30 mM). In one series of experiments, oocytes were injected with the mRNA encoding the substance K receptor. In these oocytes, extracellular Mg²⁺ (20 mM) did not evoke any current but the cells responded vigorously to the addition of substance K (Fig. 25). These experiments indicate that there is no endogenous sensitivity of the oocyte to extracellular Ca²⁺ or Mg²⁺.

Similar experiments were performed using oocytes injected with poly(A)⁺-enriched mRNA prepared from human parathyroid glands (hyperplastic tissue from a case of secondary HPT). In these oocytes, increasing the concentration of extracellular Ca²⁺ caused a reversible increase in the Cl⁻ conductance which oscillated (Fig. 26). The addition of 300 *µ*M La³⁺ likewise caused oscillatory increases in the Cl⁻ conductance. Increasing the concentration of extracellular Mg²⁺ from 1 to 10 mM evoked increases in the Cl⁻ conductance that persisted in the absence of extracellular Ca²⁺. Additional experiments suggest that the response to extracellular Ca²⁺ is concentration dependent. Thus, in three mRNA-injected oocytes, Cl⁻ conductance increased to a maximum of 111 ± 22 nA at 3 mM and 233 ± 101 nA at 10 mM extracellular Ca²⁺.

The results obtained in Xenopus oocytes demonstrate the presence of a mRNA(s) in parathyroid cells encoding a protein(s) which can impart, in normally unresponsive cells, sensitivity to extracellular Ca²⁺. Moreover, the ability of extracellular Mg²⁺ to evoke oscillatory increases in Cl⁻ current in the absence of extracellular Ca²⁺ demonstrates that the Cl⁻ current depends on the mobilization of intracellular Ca²⁺ rather than influx of extracellular Ca²⁺. The results obtained with La³⁺ likewise show that the expressed protein(s) is linked to the mobilization of intracellular Ca²⁺. Together, these data show that the expressed protein(s) acts as a cell surface receptor rather than a channel. These studies provide compelling evidence for the existence of a Ca²⁺ receptor protein on the surface of parathyroid cells and demonstrate the feasibility of using the Xenopus oocyte system to achieve the molecular cloning of the Ca²⁺ receptor cDNA.

In another series of experiments, parathyroid cell mRNA, denatured with methylmercuric hydroxide, was size-fractionated by centrifugation through a glycerol gradient. Ten fractions were collected. Each group was injected into Xenopus oocytes and after a three day incubation period the oocytes were assayed for expression of the Ca²⁺ receptor. Those oocytes injected with fractions 4-6 showed the largest and most consistent increases in Cl⁻ conductance in response to extracellular Ca²⁺ (Fig. 35). These results indicate that the Ca²⁺ receptor is encoded by mRNA in a size range of 2.5-3.5 kb. This indicates that a strategy using direct expression of RNA synthesized from a transcription vector cDNA library is feasible. Size-fractionation experiments of this sort were conducted and in each of three different fractionation experiments similar results were obtained.

The mRNA fractions obtained and characterized in the preceding experiments can be assayed by injection into oocytes. For each mRNA fraction, 10-20 oocytes are injected with 50 ng of RNA at a concentration of 1 ng/nl in water. Injected oocytes are maintained at 18° C for 48-72 h after which they are assessed for expression of the Ca²⁺ receptor using measurements of Cl⁻ current. For each group of injected oocytes the number positive for expression of the receptor, as well as the magnitude of the Ca²⁺-dependent Cl⁻ current measured, is determined. As negative controls, oocytes are injected with rat liver poly(A)⁺-enriched mRNA, yeast RNA, or water.

It is expected that an mRNA in the range of 2.5 - 3.5 kb will encode the receptor. mRNA of a larger size may necessitate a cloning approach based on hybrid depletion of parathyroid mRNA prior to oocyte injection. This strategy is not dependent upon the generation of full-length cDNA clones for success. If receptor expression is not obtained with a single size fraction of mRNA, oocytes are injected with mixed size fractions to determine a combination that does give rise to a functional receptor. If it does appear that multiple subunits are necessary for the formation of a functional receptor, the hybrid depletion expression cloning strategy is used. In this approach, clones are selected on the basis of their ability to deplete a specific mRNA species from the total mRNA population. A clone encoding a single subunit is identified by its ability to prevent the formation of the active multi-subunit complex. By exhaustive screening it is possible to identify clones encoding all of the necessary subunits.

This approach permits the isolation of clones encoding individual subunits required to form a functional receptor complex. Synthetic RNA from pools of clones are assayed for their ability to induce expression of the Ca²⁺ receptor in Xenopus oocytes by the same techniques used to analyze the original mRNA fractions. Originally, 10 pools representing 100,000 primary clones each are examined. Pools of clones showing a positive response are screened at lower (typically 4 to 10 fold) complexity, and again positive pools further subdivided and screened. This process of library sub-fractionation is followed until individual positive clones are identified. As a negative control for the oocyte expression assay, anti-sense transcripts are generated by T7 transcription of those DNA templates that induce a positive response. Anti-sense transcripts are unable to give rise to an authentic receptor, and this will control any non-specific positive signal arising from injection of synthetic RNA. Another concern is the fact that synthetic RNA can occasionally "poison" translation in injected oocytes, by an undefined mechanism. To control for this possibility, synthetic RNAs giving a negative response are co-injected at various dilutions with parathyroid cell mRNA, to determine if they are non-specifically interfering with the expression of the Ca²⁺ receptor.

When an individual clone encoding the Ca²⁺ receptor is identified, the cDNA insert will be excised from the λ vector and used for large scale production of synthetic RNA. oocyte injection of this single RNA species allows rigorous assessment of the characteristics of the expressed receptor.

If the size of the mRNA encoding the Ca²⁺ receptor is too large for cloning by direct transcription and expression, or if multiple subunits are involved, a hybrid-depletion technique of screening pools of clones is used. cDNA insert DNA will be prepared from pools of clones from the size-selected parathyroid cell cDNA library. This DNA is hybridized to parathyroid cell mRNA under conditions that permit the formation of DNA/RNA duplexes. The unannealed, hybrid-depleted RNA is recovered and used for oocyte injections. DNA from pools of clones containing sequences representing Ca²⁺ receptor mRNA is depleted from this mRNA from the total parathyroid cell mRNA population, and expression of the receptor is reduced or absent upon oocyte injection. A process of sub-fractionation is followed on pools of clones of decreasing complexity, at each step assaying for cloned DNA that depletes Ca²⁺ receptor-encoding mRNA from the total parathyroid cell mRNA population. The use of an internal control during the hybrid depletion assays ensures that the hybrid-depleted RNA is intact and capable of being translated in the oocyte.

Human parathyroid cells express a beta-adrenergic receptor coupled to adenylate cyclase. This receptor can be expressed in oocytes, where it is capable of agonist-induced activation of the endogenous adenylate cyclase. During the hybrid-depletion screening for Ca²⁺ receptor clones, oocytes injected with hybrid depleted mRNA are assayed for isoproterenol-induced adenylate cyclase activation. A positive response in this assay serves to indicate that any observed inhibition of Ca²⁺ receptor response is specific, and not due to a general inhibition of the total mRNA population.

The hybrid-depletion screening strategy can result in the isolation of clones that do not contain a complete protein coding region. Positive clones isolated by this screening strategy are sequenced to determine their protein coding capacity. Northern blot analysis of human parathyroid gland RNA permits the determination of the size of the complete mRNA corresponding to specific clones. If positive clones do not appear to be full-length, the cloned cDNA will be used as a hybridization probe to screen a parathyroid gland cDNA library for complete cDNAs.

A variety of cell lines are capable of coupling exogenously expressed receptors to endogenous functional responses. A number of these cell lines (e.g., NIH-3T3, HeLa, NG115, CHO, HEK, 293 and COS7) can be tested to confirm that they lack an endogenous Ca²⁺ receptor. Those lines lacking a response to external Ca²⁺ can be used to establish stably transfected cell lines expressing the cloned Ca²⁺ receptor.

Sequence analysis of Ca²⁺ receptor cDNA clones identified by expression cloning will delineate the open reading frame encoding the receptor protein. The coding region of the cDNA will be subcloned into multiple cloning site of the eukaryotic expression vector pMSG. This vector allows high level transcirption driven by the mouse mammary tumor virus (MMTV) promoter, and is active in a wide variety of mammalian cells. The vector also contains a gpt gene for resistance to mycophenolic acid which is under the control of the SV40 early promoter, and sequences necessary for selection and growth in bacteria. Large quantities of the expression vector/receptor cDNA plasmid construct will be grown and purified from E. coli.

The most effective method for transfection of eukaryotic cell lines with plasmid DNA varies with the given cell type. The Ca²⁺ receptor expression construct will be introduced into cultured cells by the appropriate technique, either Ca²⁺ phosphate precipitation, DEAE-dextran transfection, lipofection or electroporation. Following the transfection procedure, cells are grown in the presence of the antibiotic G418 to select for cells expressing the neomycin resistance gene. Colonies of G418 resistant transfectants will be subcloned and established as individual cell lines. Expression of the Ca²⁺ receptor protein in G418 resistant cells will be assessed by several methods. Southern blot and slot blot analysis will confirm the presence and the copy number of the receptor cDNA sequence. Northern blot analysis will be used to demonstrate that receptor mRNA is being transcribed from the plasmid construct. Functional expression of the receptor protein will be determined by measuring the mobilization of intracellular Ca²⁺ in response to externally applied Ca²⁺ receptor agonists.

Cloning the Ca²⁺ receptor enables both structural and functional studies of this novel receptor. Recombinantly produced receptor may be crystallized for structural studies. Stably transfected cell lines expressing the receptor can be used for high-throughput screening of natural product or other compound libraries. Molecules of the requisite potency and specificity can be labeled (radioactively or fluorescently). The ability of test molecules/extracts to displace such a labeled molecule will form the basis of a high-throughput assay for screening.

Given the appropriate cells or tissues expressing other calcium receptors, these receptors may be cloned in a manner analogous to that described above for the parathyroid cell calcium receptor. For example, mRNA from human osteoclastoma tissue encodes the osteoclast calcium receptor (Figure 34). Thus, to isolate a clone for the human osteoclast receptor, one need only isolate mRNA from osteoclastoma tissue, prepared a cDNA library and assay/fractionate subpools as described above. Furthermore, the preferred receptors for drug screening are of human origin. A clone encoding a calcium receptor from one species may be used to obtain the corresponding human cDNA clone by cross-hybridization as is well known by those skilled in the art. In addition, the clone of the parathyroid cell or other cell Ca²⁺ receptor allows isolation of genes encoding similar Ca²⁺-sensing proteins in other cells, and expression of those proteins. This is achieved by a variety of approaches. Southern blot analysis of human genomic DNA, utilizing the Ca²⁺ receptor cDNA as a hybridization probe, will give an indication of the number of related sequences encoded within the genome; hybridization at varying stringencies will give an indication of the degree of divergence among the related sequences. This will provide information about the potential number of genes encoding related receptor proteins. Northern blot analysis with Ca²⁺ receptor cDNA as probe will determine if the same or related transcripts are present in various tissues. If related transcripts homologous to the parathyroid cell Ca²⁺ receptor are detected, it is a relatively simple matter to obtain clones of these mRNAs, either by screening the appropriate cDNA libraries or by polymerase chain reaction (PCR) techniques. Novel receptor clones so obtained can be assessed functionally by expression, either in oocytes or in transfected cell lines. Transfected cell lines expressing a cell-specific Ca²⁺ receptor can then provide a means of high-throughput screening for molecules that act specifically on the Ca²⁺-sensing mechanism of, for example, osteoclasts or juxtaglomerular cells.

In an alternative method, the calcium receptor can be cloned by expression in eukaryotic cells. For example, a cDNA library can be prepared from parathyroid mRNA and cloned into the eukaryotic expression vector, pCDNA1. Subpools from this library can be transfected into eukaryotic cells such as COS7 or HEK293 cells resulting in relatively high level transient expression of encoded cDNA sequences. Cells transfected with a function calcium receptor clone will express the calcium receptor which can then be activated by calcium, neomycin or other calcimimetic compounds. If cells are first loaded with a fluorometric indicator for [Ca²⁺]ᵢ, activation of the calcium receptor results in increased fluorescence. Thus library subpools containing the calcium receptor are identified by their ability, upon transfection into eukaryotic cells, to induce a calcium or calcimimetic-specific increase in fluorescense. This fluorescense can be detected using either a fluorimeter or a fluorescence activated cell sorter (FACS).

In an alternative method, the Ca²⁺ receptor can be cloned by use of a monoclonal antibody generated against the receptor. Monoclonal antibodies provide powerful tools for the immunoaffinity purification of specific proteins. Once purified, limited amino acid sequence data can be obtained from the protein of interest, and used to design oligonucleotide sequence probes to screen for clones of the complete cDNA sequence.

For production of hybridomas, whole bovine parathyroid gland cells are used as the immunogen. Purified, dispersed cells are obtained, and live cell preparations are injected intraperitoneally into the appropriate mouse strain, according to established procedures. Standard protocols are followed for immunization schedules and for the production of hybridomas. A two-step screening procedure is used to identify hybridomas secreting monoclonal antibodies that recognize the Ca²⁺ receptor. The initial screen will identify those monoclonals that recognize parathyroid cell surface antigens. Immunohistochemical techniques are then used to screen hybridoma supernatants for the presence of mouse antibodies that bind to the surface of parathyroid cells. This screen can be performed on fixed sections of parathyroid gland tissue, or on dispersed cells in primary culture. The techniques for this assay are well established in the literature.

This screen will identify hybridomas producing monoclonal antibodies to a variety of cell surface determinants, and monoclonals specific for the Ca²⁺ receptor would be expected to comprise only a small subset of these. To identify monoclonal antibodies that bind to the Ca²⁺ receptor, hybridoma supernatants that test positive in the initial screen are assayed for their ability to block the response of cultured parathyroid cells to Ca²⁺ receptor agonists. Some antibodies that bind to the extracellular domain of the receptor are expected to inhibit or activate ligand binding or to otherwise interfere with or affect receptor activation.

Monoclonal antibodies positive in both screens are characterized through Western blotting, immunoprecipitation and immunohistochemistry. This permits the determination of the size of the antigen that is recognized and its tissue distribution. The appropriate monoclonal antibody is then used for purification of the Ca²⁺ receptor protein by immunoaffinity chromatography, following standard techniques. Sufficient quantities of protein are obtained to allow limited amino acid sequence determination. Degenerate oligonucleotide probes are then designed on the basis of the peptide sequence information. These probes are then used to screen parathyroid gland cDNA libraries for full length clones of the Ca²⁺ receptor. Clones obtained are characterized by DNA sequencing and by functional expression in the oocyte system and in cultured mammalian cell lines.

Alternatively, the antibodies can be used to screen expression libraries, e.g., cDNA libraries in λgt11 or its equivalent, to determine those clones expressing antigenically reactive protein. Such clones can then be sequenced to determine whether they encode a protein that might be a Ca²⁺ receptor.

It will also be appreciated by those skilled in the art that phage display libraries can be used to clone and analyze calcium receptors in place of monoclonal antibodies. In these libraries, antibody variable regions or random peptides are shotgun cloned into phage expression vectors such that the antibody regions or peptides are displayed on the surface of the phage particle. Phage which display antibody regions or peptides capable of high specific binding to calcium receptors will bind to cells which display these receptors (e.g. parathyroid cells, C-cells, osteoclasts, etc.). Millions of such phage can be panned against these cell types selecting only those phage which can bind to these cells (which includes those phage binding to calcium receptors). In this manner, the complexity of the library can be vastly reduced. Subsequently, the screens described above for monoclonal antibodies can be used to isolate phage which display calcium receptor-binding antibody or peptide regions, and these phage can be used to isolate the calcium receptor for purposes of structural identification and cloning. Kits to prepare such phage display libraries are commercially available (e.g. Stratacyte, or Cambridge Antibody Technology Limited). Recombinant phage endowed with such calcium receptor-binding properties can also be used in lieu of monoclonal antibodies in the various analyses of calcium receptors. Such phage can also be used in high throughput binding competition screens to identify organic compounds capable of functional binding to calcium receptors which can serve as structural leads for the development of human therapeutics acting at the calcium receptor.

In another alternative, affinity cross-linking of radioligands to their receptors can be used to isolate the receptor protein as described by Pilch & Czech, 1 Receptor Biochem. Methodol. 161, 1984. Covalent attachment of a radioligand allows extensive washing to remove non-specific binding. For example, a high affinity molecule, e.g., a random copolymer of arginine and tyrosine (MW = 22K; argtyr ratio = 4:1) which mobilizes intracellular Ca²⁺ with an EC₅₀ of about 100 nM or less, is iodinated with ¹²⁵I, and cross-linked. Protamines, because of their much smaller size, may be preferable in cross-linking studies and can be reductively alkylated as described by Dottavio-Martin & Ravel, 87 Analyt. Biochem. 562, 1978.

Nonspecific labelling is kept to a minimum by cross-linking in the presence of unlabeled polycations and di-and trivalent cations. At high concentrations of these molecules nonspecific interactions of the label with the cell surface might be reduced.

### Uses

Primary hyperparathyroidism (HPT) is characterized by hypercalcemia and elevated levels of circulating PTH. One of the major defects in HPT appears to be a diminished sensitivity of parathyroid cells to negative feedback regulation by extracellular Ca²⁺. Thus, in tissue from patients with primary HPT, the "set-point" for extracellular Ca²⁺ is shifted to the right so that higher than normal concentrations of extracellular Ca²⁺ are required to depress PTH secretion. Moreover, in primary HPT, even high concentrations of extracellular Ca²⁺ often depress PTH secretion only partially. In secondary (uremic) HPT, a similar increase in the set-point for extracellular Ca²⁺ is observed even though the degree to which Ca²⁺ suppresses PTH secretion is normal. The changes in PTH secretion are paralleled by changes in [Ca²⁺]ᵢ: the set-point for extracellular Ca²⁺-induced increases in [Ca²⁺]ᵢ is shifted to the right and the magnitude of such increases is reduced. Moreover, staining of tissue with a monoclonal antibody that appears to recognize the Ca²⁺ receptor is diminished in adenomatous and hyperplastic parathyroid cells.

The Ca²⁺ receptor constitutes a discrete molecular entity for pharmacological intervention. Molecules that mimic or antagonize the action of extracellular Ca²⁺ are beneficial in the long-term management of both primary and secondary HPT. Such molecules provide the added impetus required to suppress PTH secretion which the hypercalcemic condition alone cannot achieve. Such molecules with greater efficacy than extracellular Ca²⁺ may overcome the apparent nonsuppressible component of PTH secretion which is particularly troublesome in adenomatous tissue. Alternatively or additionally, such molecules can depress synthesis of PTH, as prolonged hypercalcemia has been shown to depress the levels of preproPTH mRNA in bovine and human adenomatous parathyroid tissue. Prolonged hypercalcemia also depresses parathyroid cell proliferation in vitro, so calcimimetics can also be effective in limiting the parathyroid cell hyperplasia characteristic of secondary HPT.

Other cells in the body can respond directly to physiological changes in the concentration of extracellular Ca²⁺. Calcitonin secretion from parafollicular cells in the thyroid (C-cells) is regulated by changes in the concentration of extracellular Ca²⁺. Renin secretion from juxtaglomerular cells in the kidney, like PTH secretion, is depressed by increased concentrations of extracellular Ca²⁺. Extracellular Ca²⁺ causes the mobilization of intracellular Ca²⁺ in these cells. Isolated osteoclasts respond to increases in the concentration of extracellular Ca²⁺ with corresponding increases in [Ca²⁺]ᵢ that arise partly from the mobilization of intracellular Ca²⁺. Increases in [Ca²⁺]ᵢ in osteoclasts are associated with an inhibition of functional responses (bone resorption) analogous to PTH secretion in parathyroid cells. Thus, there are sufficient indications to suggest that Ca²⁺, in addition to its ubiquitous role as an intracellular signal, also functions as an extracellular signal to regulate the responses of certain specialized cells. Molecules of this invention can be used in the treatment of diseases associated with disrupted Ca²⁺ responses in these cells.

Cloning the Ca²⁺ receptor on parathyroid cells and other cells will allow the presence of homologous proteins in other cells to be directly assesed. A family of structurally homologous Ca²⁺ receptor proteins can thus be obtained. Such receptors will allow understanding of how these cells detect extracellular Ca²⁺ and enable evaluation of the mechanism(s) as a site of action for the therapeutics described herein effective in the treatment of HPT, osteoporosis, and hypertension, and novel therapies for other bone and mineral-related diseases.

Other uses are discussed above. For example, recombinant Ca²⁺ receptor proteins may be used in therapy, and introduced by standard methods, e.g., by transfection of nucleic acid encoding that protein. In addition, such protein is useful in assays for calcimimetic molecules of this invention.

The following examples illustrate the invention but do not limit its scope.

### Examples

In the studies described herein, a variety of organic molecules were found to mobilize intracellular Ca²⁺ and depress PTH secretion in parathyroid cells. These molecules are structurally diverse but most have a net positive charge at physiological pH. The cationic nature of the organic molecules plays an important role but is not the sole factor determining activity.

### Example 1: Screening Calcimimetic Molecules on Bovine Parathyroid cells

Dissociated bovine parathyroid cells were purified on gradients of Percoll and cultured overnight in serum-free medium. The cells were subsequently loaded with fura-2 and the concentration of free intracellular Ca²⁺ measured fluorimetricly. Changes in [Ca²⁺]ᵢ were used to screen for molecules active at the Ca²⁺ receptor. To be considered a calcimimetic, a molecule was required to show the normal effects caused by increasing extracellular Ca²⁺ and triggered by the activation of the Ca²⁺ receptor. That is,
1) The molecule must elicit an increase in [Ca²⁺]ᵢ that persists in the absence of extracellular Ca²⁺ (demonstrating the mobilization of intracellular Ca²⁺) ;
2) The molecule must cause a decrease in isoproterenol-stimulated cyclic AMP formation which is blocked by pertussis toxin;
3) The molecule must inhibit PTH secretion over the same range of concentrations that cause the increase in [Ca²⁺]ᵢ; and
4) The concentration-response curves for Ca²⁺ mobilization and PTH secretion by the molecule must be shifted to the right by a PKC activator, such as phorbol myristate acetate (PMA).

Several structurally different classes of molecules were tested: polyamines, aminoglycoside antibiotics, protamine, and polymers of lysine or arginine. The structures of these molecules are depicted in Figure 1. Included in Figure 1 are the net positive charge of the molecules and their EC₅₀'s for evoking the mobilization of intracellular Ca²⁺ in bovine parathyroid cells.

In general, the greater the net positive charge on the molecule, the greater its potency in causing the mobilization of intracellular Ca²⁺. However, some striking exceptions to this apparent rule have been found as discussed below.

As can be seen from the figures, spermine, neomycin B, and protamine evoked rapid and transient increases in [Ca²⁺]ᵢ in fura-2-loaded bovine parathyroid cells (Figs. 6, 7, 11). They did not, however, cause sustained, steady-state increases in [Ca²⁺]ᵢ in bovine parathyroid cells (Fig. 6, 11), although they did in human parathyroid cells (Fig. 19). In this respect, they resembled the cytosolic Ca²⁺ response elicited by extracellular Mg²⁺, which causes the mobilization of intracellular Ca²⁺ unaccompanied by an influx of extracellular Ca²⁺ in bovine cells (Fig. 11b). Transient increases in [Ca²⁺]ᵢ elicited by spermine, neomycin B, or protamine were not blocked by low concentrations (1 µM) of La³⁺ or Gd³⁺ (Fig. 11f, g). Cytosolic Ca²⁺ transients elicited by the molecular polycations persisted in the absence of extracellular Ca²⁺ but were blocked when cellular stores of Ca²⁺ were depleted by pretreatment with ionomycin (Figs. 7; 11h, i). All these molecules therefore cause the mobilization of intracellular Ca²⁺ in parathyroid cells.

It was additionally shown that the molecular polycations mobilized the same pool of intracellular Ca²⁺ as that used by extracellular Ca²⁺. Thus, increasing the concentration of extracellular Ca²⁺ progressively inhibited the transient increases in [Ca²⁺]ᵢ evoked by spermine (Fig. 6). Conversely, a maximally effective concentration of spermine or neomycin B (Fig. 12) blocked transient, but not steady-state increases in [Ca²⁺]ᵢ evoked by extracellular Ca²⁺.

Significantly, spermine, neomycin B, and protamine inhibited PTH secretion to the same extent as extracellular Ca²⁺. These inhibitory effects on secretion were obtained at concentrations that caused the mobilization of intracellular Ca²⁺ (Figs. 8, 13). These findings are relevant to understanding the mechanisms contributing to the regulation of PTH secretion by extracellular Ca²⁺. Because a variety of inorganic polycations all inhibit secretion, yet only extracellular Ca²⁺ causes sustained, steady-state increases in [Ca²⁺]ᵢ, such increases in [Ca²⁺]ᵢ cannot be importantly involved in the regulation of secretion. Mobilization of intracellular Ca²⁺, rather than the influx of extracellular Ca²⁺, is the essential mechanism associated with the inhibition of PTH secretion. This is important because it defines the sufficient mechanism to be affected if a molecule is to affect PTH secretion; molecules stimulating selectively the influx of extracellular Ca²⁺ will be relatively ineffective in suppressing PTH secretion. In contrast, molecules causing solely the mobilization of intracellular Ca²⁺ should be just as efficacious as extracellular Ca²⁺ in suppressing PTH secretion.

Like the mobilization of intracellular Ca²⁺ elicited by extracellular Ca²⁺, that elicited by molecular polycations was depressed by PMA. A representative experiment showing the preferential inhibitory effects of PMA on cytosolic Ca²⁺ transients elicited by spermine is shown in Fig. 14. Cytosolic Ca²⁺ transients evoked by ATP were unaffected, even when a submaximal concentration of ATP was used. The effect of PMA on cytosolic Ca²⁺ transients elicited by the molecular polycations paralleled its effect on responses to extracellular Ca²⁺; in both cases there was a shift to the right in the concentration-response curve (Fig. 15). The depressive effects of PMA on [Ca²⁺]ᵢ were accompanied by potentiating effects on secretion which were overcome at higher concentrations of the organic polycations (Fig. 16).

The mobilization of intracellular Ca²⁺ elicited by molecular polycations was associated with increases in the formation of inositol phosphates. For example, protamine caused a rapid (within 30 s) increase in the formation of IP₃ which was accompanied by a rise in levels of IP₁. Both these effects were dependent on the concentration of extracellular protamine (Fig. 17). Moreover, pretreatment with PMA blunted the formation of inositol phosphates elicited by molecular polycations. Representative results obtained with spermine are presented in Fig. 18.

Spermine, neomycin B, and protamine depressed isoproterenol-induced increases in cyclic AMP. Like the inhibitory effects of extracellular Ca²⁺ on cyclic AMP formation, those caused by molecular polycations were blocked by pretreatment with pertussis toxin (Table 2).

**Table 2**

| | cyclic AMP (% of control) | |
|---|---|---|
| | control | +PTx |
| 0.5 mM Ca²⁺ | 100 | 106 ± 8 |
| 2.0 mM Ca²⁺ | 19 ± 4 | 94 ± 2 |
| 0.5 mM Ca²⁺, 200 µM Spermine | 23 ± 5 | 93 ± 6 |
| 0.5 mM Ca²⁺, 30 µM Neomycin B | 28 ± 8 | 87 ± 6 |
| 0.5 mM Ca²⁺, 2 µg/ml Protamine | 20 ± 4 | 89 ± 9 |
| Pertussis toxin (PTx) blocks the inhibitory effects of extracellular Ca²⁺ and molecular polycations on cyclic AMP formation. Bovine parathyroid cells were cultured for 16 h with or without 100 ng/ml pertussis toxin. The cells were subsequently washed and incubated for 15 min with 10 µM isoproterenol with or without the indicated concentrations of extracellular Ca²⁺ or molecular polycations. Total cyclic AMP (cells + supernatant) was determined by RIA and the results are expressed as a percentage of the levels obtained in 0.5 mM Ca²⁺ (112 ± 17 pmole/10⁶ cells). Each value is the mean ± SEM of three experiments. | | |

In human parathyroid cells, extracellular Mg²⁺ elicited a sustained, steady-state increase in [Ca²⁺]ᵢ in addition to a rapid transient increase (Fig. 10). As in bovine parathyroid cells responding to extracellular Ca²⁺, the steady-state increase in [Ca²⁺]ᵢ evoked by Mg²⁺ in human parathyroid cells results from Ca²⁺ influx through voltage-insensitive channels (Fig. 10a). This effect of Mg²⁺ on steady-state [Ca²⁺]ᵢ in human parathyroid cells is seen in both adenomatous and hyperplastic tissue.

Neomycin B and spermine were tested for effects on [Ca²⁺]ᵢ in human parathyroid cells prepared from adenomatous tissue. Representative results with neomycin B are shown in Fig. 19. Neomycin B caused not only a transient but additionally a steady-state increase in [Ca²⁺]ᵢ in human parathyroid cells (Fig. 19a). Thus, in human cells, the pattern of change in [Ca²⁺]ᵢ evoked by extracellular Ca²⁺, Mg²⁺ or neomycin B is very similar.

Cytosolic Ca²⁺ transients elicited by neomycin B persisted in the presence of La³⁺ (1µM) and absence of extracellular Ca²⁺. Neomycin B therefore causes the mobilization of intracellular Ca²⁺ in human parathyroid cells. Neomycin B inhibited PTH secretion from human parathyroid cells at concentrations that caused the mobilization of intracellular Ca²⁺ (Fig. 13). There were, however, some differences in the responses of human and bovine parathyroid cells to neomycin B. The EC₅₀ of neomycin B for the mobilization of intracellular Ca²⁺ was 40 µM in bovine and 20 µM in human parathyroid cells (cf. Figs. 13 and 15), whereas the potency of spermine was similar in bovine and human parathyroid cells (EC₅₀ = 150 µM). Thus, although bovine cells can be used for initial studies to screen test molecules for activity, it is important to perform follow-up studies using human parathyroid cells.

To assess the effects of molecular polycations on C-cells, a neoplastic cell line, derived from a rat medullary thyroid carcinoma (rMTC 6-23 cells) was used. Both spermine (10 mM) and neomycin B (5 mM) were without effect on basal [Ca²⁺]ᵢ in these cells. Nor did either molecule affect the response to the subsequent addition of extracellular Ca²⁺. Representative results documenting the lack of effect of neomycin B are shown in Fig 21. Neomycin B (1 mM) or spermine (1 or 5 mM) failed to evoke any increase in [Ca²⁺]ᵢ in osteoclasts (Fig. 23). In the trace shown, there appeared to be some potentiation of the response to a subsequent increase in the concentration of extracellular Ca²⁺, although this was not a consistent finding. In two other cells, spermine (5 mM) was again without effect on basal [Ca²⁺]ᵢ and caused a small inhibition (about 15%) of the extracellular Ca²⁺-induced increase in [Ca²⁺]ᵢ. In a third cell, neomycin B (5 mM) was without effect on basal [Ca²⁺]ᵢ and did not affect increases in [Ca²⁺]ᵢ elicited by extracellular Ca²⁺. The overall picture that develops from these studies is that spermine and neomycin B are without effect on basal or stimulated levels of cytosolic Ca²⁺ in osteoclasts.

The failure of the molecular polycations to affect the Ca²⁺-sensing mechanisms of C-cells or osteoclasts demonstrates the ability to discover or design novel lead molecules that act specifically on the parathyroid cell Ca²⁺ receptor or otherwise modulate one or more functions of the parathyroid cell's normal response to [Ca²⁺].

Screening of various other molecules is described in detail below and the results summarized in Table 1.

### Example 2: Polyamine screening

Straight chain polyamines (spermine, spermidine, TETA, TEPA, and PEHA) and two derivatives thereof (NPS 381 and NPS 382) were screened as in Example 1. These molecules were all found to mobilize intracellular Ca²⁺ in bovine parathyroid cells. Their order of potency is as follows, with the net positive charge listed in parentheses:

**Table 3**

| Molecule | EC₅₀ (in µM) |
|---|---|
| NPS 382 (+8) | 50 |
| NPS 381 (+10) | 100 |
| spermine (+4) | 150 |
| PEHA (+6) | 500 |
| spermidine (+3) | 2000 |
| TEPA (+5) | 2500 |
| TETA (+4) | 8000 |

Putrescine (+2) and cadaverine (+2) were inactive at a concentration of 2mM.

Another straight-chain polyamine, DADD, behaved somewhat differently from the other polyamines and is described in Example 7.

### Example 3: Cyclic Polyamine Screening

Two cyclic polyamines, hexacyclen and NPS 383, were screened as in Example 1. Hexacyclen (+6, EC₅₀ = 20 µM) is 7-fold more potent than NPS 383 (+8, EC₅₀ = 150 µM). The converse would be expected based solely on net positive charge as the structural characteristic for Ca²⁺ receptor activity.

### Example 4: Aminoglycoside Antibiotic Screening

Six antibiotics were screened as in Example 1. The resulting EC₅₀'s for the mobilization of intracellular Ca²⁺, in rank order of potency, were:

**Table 4**

| Antibiotic | EC₅₀ (in µM) |
|---|---|
| neomycin (+6) | 10 |
| gentamicin (+5) | 150 |
| bekanamycin (+5) | 200 |
| streptomycin (+3) | 600 |

Kanamycin (+4.5) and lincomycin (+1) were without effect at a concentration of 500 µM. Within the aminoglycoside series, there is a correlation between net positive charge and potency. However, neomycin is considerably more potent than various polyamines (NPS 381, NPS 382, NPS 383, PEHA) that have an equal or greater positive charge.

### Example 5: Peptide and Polvamino Acid Screening

Protamine and polymers of lysine or arginine varying in peptide length were screened for their ability to mobilize intracellular Ca²⁺ as in Example 1. The resulting EC₅₀'s for the mobilization of intracellular Ca²⁺, in rank order of potency, were:

**Table 5**

| Peptide (MW in kD) | EC₅₀ (in mM) |
|---|---|
| polyarg (100) | 4 |
| polyarg (40) | 15 |
| polyLys (27) | 30 |
| protamine (4.8) | 75 |
| polyArgTyr (22) | 200 |
| polyLys (14) | 1000 |
| polyLys (3.8) | 3000 |

The net positive charge of these polymers increases as the MW increases. Thus, as for the aminoglycosides, there is a direct correlation between net charge and potency among this series of polyamino acids. Protamine is essentially polyArg with a net positive charge of +21.

### Example 6: Arylalkylamine screening

Molecules selected from the class of arylalkylamine toxins derived from the venoms of wasps and spiders were screened as in Example 1.

Philanthotoxin-433 (+3) was without effect at a concentration of 500 *µ*M. It is similar in structure to the argiotoxins described below.

Argiotoxin-636 (400 µM) did not elicit increases in [Ca²⁺]ᵢ but it did potentiate cytosolic Ca²⁺ responses to the subsequent addition of extracellular Ca²⁺. This is a feature common to all molecules that activate the Ca²⁺ receptor and is also seen with a variety of extracellular divalent cations. This is considered in more detail in Example 7.

In contrast to argiotoxin-636, argiotoxin-659 elicited increases in [Ca²⁺]ᵢ with an EC₅₀ of 300 µM. Argiotoxin-659 differs from argiotoxin-636 in having a hydroxylated indole moiety rather than a dihydroxyphenyl group. This is the only difference in the structure of these two molecules. Thus, the difference in potency lies in the nature of the aromatic group, not in the polyamine chain which carries the positive charge.

### Example 7: Screening of Ca²⁺ Channel Blockers

Ca²⁺ channel blockers, i.e., those molecules which block influx of extracellular Ca²⁺ through voltage-sensitive Ca²⁺ channels, were screened as in Example 1. There are three structural classes of Ca²⁺ channel blockers: (1) dihydropyridines, (2) phenylalkylamines, and (3) benzothiazipines.

None of the dihydropyridines tested (nifedipine, nitrendipine, BAY K 8644, and (-) 202-791 and (+) 202-791) had any effect on basal [Ca²⁺]ᵢ or increases in [Ca²⁺]ᵢ evoked by extracellular Ca²⁺ when they were tested at 1 *µ*M. Previous studies showed that parathyroid cells lack voltage-sensitive Ca²⁺ channels, but do have voltage-insensitive Ca²⁺ channels that are regulated by the Ca²⁺ receptor.

The phenylalkylamines examined were verapamil, D-600 (a methoxy-derivative of verapamil), TMB-8, and an analog of TMB-8, NPS 384. The first three molecules were tested at a concentration of 100 µM. The phenylalkylamines behaved differently from other molecules examined. They evoked no change in [Ca²⁺]ᵢ when added to cells bathed in buffer containing a low concentration of extracellular Ca²⁺ (0.5 mM). However, verapamil, D-600, and TMB-8 potentiated the mobilization of intracellular Ca²⁺ elicited by extracellular divalent cations and they additionally blocked the influx of extracellular Ca²⁺. At intermediate levels of extracellular Ca²⁺ (1-1.5 mM), these molecules were capable of evoking a small but robust increase in [Ca²⁺]ᵢ that arose from the mobilization of intracellular Ca²⁺.

The phenylalkylamines act differently than organic polycations like neomycin. The data suggest that verapamil, D-600 and TMB-8 are partial agonists at the Ca²⁺ receptor, in contrast to the other molecules examined which are full agonists.

Molecule NPS 384, at a concentration of 300 *µ*M, did not evoke an increase in [Ca²⁺]ᵢ but it blocked influx of extracellular Ca²⁺. Testing at higher concentrations may reveal an ability of this molecule to cause the mobilization of intracellular Ca²⁺.

While the ability of these molecules to block influx is intriguing and not entirely unexpected, it is the ability of these molecules to evoke transient increases in [Ca²⁺]ᵢ (arising from intracellular Ca²⁺ mobilization) that is important. Considerable experience with measurements of [Ca²⁺]ᵢ in parathyroid cells shows that transient increases in [Ca²⁺]ᵢ almost invariably result from the mobilization of intracellular Ca²⁺ and therefore reflects activation of the Ca²⁺ receptor.

The benzothiazipine examined, diltiazem, was similar in all respects to verapamil and D-600 and was also effective at 100 µM.

It should be mentioned that with the exception of the phenylalkylamines, all the active molecules tested above evoke increase in [Ca²⁺]ᵢ that are of magnitude similar to that evoked by a maximally effective concentration of extracellular Ca²⁺. This shows that these molecules are equally efficacious as extracellular divalent cations. This contrasts with the activity of phenylalkylamines, which seem to act only as partial agonists.

Amongst the phenyalkylamines, some interesting structure-activity relationships emerge. Significant is the different potencies of molecules like TMB-8 and NPS 384. TMB-8 potentiated transient increases in [Ca²⁺]ᵢ at 100 µM whereas NPS 384 fails to do so even at 300 µM, yet these molecules carry the same net positive charge. It follows that some other structural feature, unrelated to net charge, imparts greater potency to TMB-8.

### Example 8: Molecule Screening on Human Parathyroid Cells

Spermine and neomycin were tested for effects on [ca²⁺]ᵢ in human parathyroid cells obtained from glands removed by surgery and prepared as in Example 1. In human parathyroid cells, spermine was found to cause only a small increase in [Ca²⁺]ᵢ when tested at a concentration of 300 *µ*M.

Neomycin, on the other hand, evoked a large increase in [Ca²⁺]ᵢ in human parathyroid cells when tested at a concentration of 20 µM. The magnitude of the response elicited by neomycin was equal to that evoked by a maximally effective concentration of extracellular Ca²⁺.

### Example 9: Molecule Screening on Xenopus Oocytes

Oocytes injected with mRNA from human parathyroid cells express the Ca²⁺ receptor and mobilize intracellular Ca²⁺ in response to a variety of extracellular inorganic di- and trivalent cations. Using this screen allows one to test for an action directly on the Ca²⁺ receptor. Oocytes expressing the Ca²⁺ receptor also responded to several molecules active on intact parathyroid cells when screened as follows. Hexacyclen caused the mobilization of intracellular Ca²⁺ at a concentration of 135 µM. Neomycin (100 µM) and NPS 382 (5 mM) were also effective. This offers rather compelling evidence showing that these molecules act on the Ca²⁺ receptor or on some other protein intimately associated with its function.

For example, we have been able to detect Ca²⁺ receptor expression in oocytes by measuring ⁴⁵Ca²⁺ mobilization. In these experiments, oocytes were injected with bovine parathyroid mRNA or water and, after 72 hours exposed to serum or 10 mM neomycin. Prior to being stimulated, oocytes were loaded with ⁴⁵Ca²⁺. Stimulation with serum for 20 min resulted in intracellular ⁴⁵Ca²⁺ release representing a 45% increase compared to mock challenge with buffer. Challenge with 10 mM neomycin for 20 min. resulted in a 76% increase in ⁴⁵Ca²⁺ release. The assay is sensitive enough for use in cloning the Ca²⁺ receptor, and has the advantage of a higher throughout than the electrophysiological measurement of Ca²⁺ activated Cl⁻ current.

In another example, human osteoclastoma tissue was obtained from bone biopsy tissue. Oocytes injected with mRNA isolated from this tissue were challenged with 30 mM Ca²⁺. Controls did not respond while 8 of 12 oocytes injected with osteoclastoma mRNA responded appropriately (Fig. 34). These experiments provide the first evidence that the Ca²⁺ response of osteoclasts to extracellular Ca²⁺ is in fact genetically encoded. The results also indicate that the osteoclast Ca²⁺ receptor may be cloned by expression in Xenopus oocytes.

### Example 10: Molecule Screening on Rat Osteoclasts

However, the different sensitivities of parathyroid cells and osteoclasts to extracellular Ca²⁺ suggest that their Ca²⁺ receptors are different. While parathyroid cells respond to extracellular Ca²⁺ concentrations between 0.5 and 3 mM, osteoclasts respond only when the level of extracellular Ca²⁺ increases beyond 5 mM. This rather high concentration of Ca²⁺ is nonetheless physiological for osteoclasts; as they resorb bone, the local concentration of extracellular Ca²⁺ may reach levels as high as 30 mM.

Molecule screening with osteoclasts was performed as follows. Osteoclasts were obtained from the long bones of neonatal rats. [Ca²⁺]ᵢ was measured in single cells using the fluorimetric indicator indo-1. Spermine, spermidine, neomycin, and verapamil were tested, and none of these caused any large increase in [Ca²⁺]ᵢ in osteoclasts (although small responses were detected).

At a concentration of 1 mM, spermidine caused a small increase in [Ca²⁺]ᵢ (about 10% of that evoked by a maximal concentration of extracellular Ca²⁺) . Neither Neomycin (10 mM) nor Spermine (10 or 20 mM) caused increases in [Ca²⁺]ᵢ in rat osteoclasts. Neomycin (10 mM) did not block the increase in [Ca²⁺]ᵢ elicited by the subsequent addition of 25 mM extracellular Ca²⁺. Pretreatment with spermine (20 mM) however, did depress the response to extracellular Ca²⁺. Verapamil (100 µM) caused no detectable increase in [Ca²⁺]ᵢ but it did block the response to extracellular Ca²⁺.

Comparisons between osteoclasts and parathyroid cells show that molecules active on the latter are relatively ineffective in osteoclasts. This demonstrates that drugs that target a specific Ca²⁺ receptor without affecting those receptor types present on other Ca²⁺-sensing cells are readily developed. Similarly, drugs active at two or more such Ca²⁺ receptors may also be developed.

### Other Ca²⁺ Receptor Examples

The following examples demonstrate that, just as there are subtypes of receptors for molecular ligands, so too do there appear to be subtypes of Ca²⁺ receptors that can be differentially affected by drugs. The parathyroid cell Ca²⁺ receptor senses levels of extracellular Ca²⁺ around 1.5 mM whereas the Ca²⁺ receptor on the osteoclast responds to levels around 10 mM (Fig. 22). Neomycin or spermine, which activate the parathyroid cell Ca²⁺ receptor, fail to affect the Ca²⁺ receptors on C-cells or osteoclasts (Figs. 21 and 23). These data constitute the first evidence for pharmacologically distinct subtypes of Ca²⁺ receptors and these data are being used to design and develop drugs that act selectively on a particular type of Ca²⁺ receptor. Indeed, testing of lead molecules demonstrate such cell-specific effects. For example, NPS 449, which elicits increases in [Ca²⁺]ᵢ in osteoclasts is without effect on [Ca²⁺]ᵢ in parathyroid cells. Conversely, NPS 447, which activates the parathyroid cell Ca²⁺ receptor, is effective in activating the osteoclast Ca²⁺ receptor only at concentrations 10-fold higher. Finally, agatoxin 489, although not very potent in activating the C-cell Ca²⁺ receptor (EC₅₀ = 150 µM), is a quite potent activator of the parathyroid cell Ca²⁺ receptor (EC₅₀ = 3 µM). The lead molecules presently under development will affect selectively the activity of a specific type of Ca²⁺-sensing cell in vivo.

Drugs with less specificity might not necessarily be therapeutically undesirable. Thus, depressing osteoclast activity and stimulating calcitonin secretion are two different approaches to inhibiting bone resorption. Drugs that target the Ca²⁺ receptors on both of these cells might be very effective therapies for osteoporosis. Because PTH is also involved in regulating bone metabolism, drugs acting on the parathyroid cell Ca²⁺ receptor may also be useful in the treatment and/or prevention of osteoporosis.

Results of some test molecules are shown below. In Table 6, the comparative activity of calcimimetic molecules is shown. Bovine parathyroid cells and C-cells (rMT 6-23 cells) were loaded with fura-2, and rat osteoclasts with indo-1 and the potency of the indicated molecules to mobilize intracellular Ca²⁺ determined by constructing cumulative concentration-response curves. Molecules listed as "inactive" did not alter [Ca²⁺]ᵢ when tested at a concentration of 1 mM.

**Table 6**

| | EC₅₀ (µM) | | |
|---|---|---|---|
| COMPOUND | PARATHYROID | OSTEOCLAST | C-CELL |
| NPS 568 (EE) | 0.78 | 200 | >300 |
| NPS 568 (LE) | 30 | - | - |
| NPS 467 (EE) | 2 | >100 | - |
| NPS 467 (LE) | >30 | - | - |
| NPS 017 | 6 | Inactive | 150 |
| NPS 447 | 9 | 150 | - |
| NPS 456* | 15 | 200 | >100 |
| NPS 015 | 22 | - | inactive |
| NPS 109 | 40 | >300 | 5 |
| NPS 449 | inactive | 150 | - |
| NPS 468* | 30 | 250 | - |
| spermine | 150 | inactive | inactive |
| neomycin | 40 | inactive | inactive |

| | | | |
|---|---|---|---|
| ⁻racemic mixture; "inactive" is defined as causing no increase in cytosolio Ca2+ at a concentration of 1-5 mM; EE is early eluting; LE is late eluting. | | | |

### Example 11: Lead Molecules for Parathyroid Ca²⁺ Receptor

structure-activity studies using polyamines and arylalkylamines led to the testing of molecules structurally akin to NPS 456. NPS 456 is a potent activator of the parathyroid cell Ca²⁺ receptor. This molecule is notable because it possess only one positive charge yet is much more potent than many polybasic molecules. Brief (2 min) pretreatment with PMA shifts the concentration-response curve for NPS 456 to the right. This indicates that NPS 456 acts through the same mechanism used by extracellular Ca²⁺. NPS 456 evokes the mobilization of intracellular Ca²⁺ in Xenopus oocytes expressing the parathyroid cell Ca²⁺ receptor, which demonstrates a direct action on the Ca²⁺ receptor (Fig. 33). Moreover, NPS 456 contains a chiral carbon, and therefore exists in two isomeric forms. Both isomers have been synthesized and examined for activity. The R-isomer, NPS 447, is 12-times more potent than the S-isomer, NPS 448 (Fig. 28). This is the first demonstration that a Ca²⁺ receptor can recognize an organic molecule in a stereospecific manner.

Because NPS 447 is a structurally simple molecule with selective and potent effects on the parathyroid cell Ca²⁺ receptor, structure-activity studies around this lead molecule are simple. The aim of these studies is to generate an array of related molecules with various characteristics from which the final development candidate can be selected. This effort has already revealed some of the structural domains of NPS 447 that contribute to activity and potency. For example, the novel compound NPS 459 is an analog of NPS 447 that is smaller (MW < 240) yet nearly as potent as the parent molecule, whereas several other analogs are relatively inactive. The most interesting molecules from this analog project can be put into in vivo testing for effects on PTH secretion and serum Ca²⁺ levels (see Examples 15, 16, 17, 18 and 23).

The novel compound NPS 467 is an even smaller molecule than NPS 447 yet the former is about 3-fold more potent than the latter in causing the mobilization of intracellular Ca²⁺ in parathyroid cells. Like NPS 456, NPS 467 is a racemic mixture. It is anticipated that Resolution of NPS 467 into its enantiomers provides an isomer of even greater potency than the racemic mixture (see Example 16). NPS 551 is another novel compound as potent as NPS 467 in causing the mobilization of intracellular Ca²⁺ in parathyroid cells. NPS 551 is a racemic mixture and it is anticipated that the resolution of NPS 551 into its enantiomers will result in an isomer that is more potent than the racemic mixture. Further structure-activity studies on molecules related to NPS 447, NPS 467, NPS 551 and NPS 568 are expected to yield pure isomers with greater potency than these molecules in their racemate forms.

Results obtained with NPS 456 (Fig. 33) show that it elicits oscillatory increases in Cl⁻ current at concentrations of 100 µM. NPS 456 is the most potent molecule activate on Xenopus oocytes expressing the parathyroid cell Ca²⁺ receptor. The results obtained in this expression system with neomycin and NPS 456 demonstrate that these molecules act directly on the Ca²⁺ receptor.

### Example 12: Osteoclast Ca²⁺ Receptor Lead Molecules

The strategy used for elucidating the mechanism of action of extracellular Ca²⁺ on the osteoclast was similar to that proven effective in parathyroid cells. The first experiments examined the effects of La³⁺ on [Ca²⁺]ᵢ in single rat osteoclasts loaded with the fluorimetric indicator indo-1. As described above, trivalent cations like La³⁺ are impermeant and block Ca²⁺ influx. Low micromolar concentrations of La³⁺ partially depressed extracellular Ca²⁺-induced increases in [Ca²⁺]ᵢ (Fig. 29). The demonstration of a La³⁺-resistant increase in [Ca²⁺]ᵢ provides evidence for the mobilization of intracellular Ca²⁺. The results of these experiments parallel those obtained in parathyroid cells and suggest that similar mechanisms are used by extracellular Ca²⁺ to regulate [Ca²⁺]ᵢ in both cell types.

Another series of experiments showed that extracellular Mn²⁺ evoked transient increases in [Ca²⁺]ᵢ (Fig. 30(a)) that persisted in the absence of extracellular Ca²⁺ (Fig. 30B). These results are likewise indicative of the mobilization of intracellular Ca²⁺. Although Mn²⁺ can enter some cells, it is unlikely to do so in the osteoclast because Mn²⁺ quenches the fluorescence of indo-1. Thus, if Mn²⁺ penetrated intracellularly, a decrease, not an increase in the fluorescent signal would be observed.

The results obtained with a variety of di- and trivalent cations are all consistent with the presence of a Ca²⁺ receptor on the surface of the osteoclast that is coupled to the mobilization of intracellular Ca²⁺ and influx of extracellular Ca²⁺ through voltage-insensitive channels. Results show evidence for genetic material in human osteoclasts that encodes a Ca²⁺ receptor protein (see below). Transient increases in [Ca²⁺]ᵢ resulting from the mobilization of intracellular Ca²⁺, are sufficient to inhibit osteoclastic bone resorption in vitro. Thus, as with the parathyroid cell, activation of the Ca²⁺ receptor appears to be a viable means of inhibiting the activity of osteoclasts.

NPS 449 is presently the lead molecule for calcimimetic drugs on this receptor. It is a small molecule (MW < 425) and it mobilizes intracellular Ca²⁺ in rat osteoclasts with a EC₅₀ of 200 *µ*M (Figs. 31A and 31B). Although the potency of NPS 449 is relatively low, it has a simple structure with only one positive charge and is expected to have desirable pharmacodynamic and pharmacokinetic properties.

NPS 449 was examined for its ability to inhibit bone resorption in vitro. This was done by morphometric analysis of pit formation on thin slices of bovine cortical bone using scanning electron microscopy. Rat osteoclasts were incubated for 24 hours in slices of bone in the presence or absence of various concentrations of NPS 449. NPS 449 caused a concentration-dependent inhibition of bone resorption with an IC₅₀ of 10 *µ*M. The anticipated results provide the first demonstration that molecules acting at this novel site can inhibit osteoclastic bone resorption. More potent analogs of NPS 449 will be generated using synthetic chemistry and will be tested and assayed using the methods described herein.

### Example 13: C-Cell ca²⁺ Receptor Lead Molecules

Activation of the C-cell ca²⁺ receptor stimulates the secretion of calcitonin which then acts on osteoclasts to inhibit bone resorption. Calcimimetic drugs selectively affecting C-cells are useful in the treatment of osteoporosis.

The mobilization of intracellular Ca²⁺ is used as a functional index of Ca²⁺ receptor activity. The screening effort in C-cells is facilitated by the availability of cultured cell lines expressing the C-cell phenotype (e.g., rat medullary thyroid carcinoma cells; rMTC 6-23 cells). Selected for initial study were three arylalkylamine molecules. Two are naturally occurring (agatoxin 489 and agatoxin 505) and the other (NPS 019) is a synthetic agatoxin analog. Agatoxin 505 was found to block extracellular Ca²⁺-induced increases in [Ca²⁺]ᵢ, with an IC₅₀ of 3 *µ*M. The inhibitory effect resulted from a block of the L-type voltage-sensitive Ca²⁺ channel present in these cells. In contrast, agatoxin 489 was found to mobilize intracellular Ca²⁺ in rMTC cells with an EC₅₀ of 150 *µ*M. This was the first organic molecule discovered that was found to activate the C-cell Ca²⁺ receptor. The synthetic analog, NPS 019, was even more potent and mobilized intracellular Ca²⁺ with an EC₅₀ of 5 µM (Fig. 32). It is significant that the only structural difference between NPS 019 and agatoxin 489 is the presence or absence of an hydroxyl group. The fact that such subtle differences in structure affect profoundly the potency of molecules indicates a structurally specific binding site on the Ca²⁺ receptor. This, in turn, encourages the view that very potent and selective activators of Ca²⁺ receptors can be developed.

NPS 019, which is a small molecule (MW < 500), is a lead molecule for development of calcimimetics of the C-cell Ca²⁺ receptor and can be tested for its ability to stimulate calcitonin secretion in vitro. Subsequent in vivo testing will then determine the ability of this molecule to stimulate calcitonin secretion and inhibit bone resorption. These in vivo studies will be performed in rats. The results obtained in these studies, which are anticipated to be positive, will provide the first evi-dence showing that a small organic molecule acting on a novel receptor can stimulate calcitonin secretion and depress bone resorption.

### Example 14: Calcilytic Activity of NPS 021 on Parathyroid Cells

For a compound to be considered a calcilytic, it must block the effects of extracellular Ca²⁺ or a calcimimetic compound on an extracellular Ca²⁺-sensing cell. An example of a calcilytic compound is NPS 021, the structure of which is provided in Fig. 1. In bovine parathyroid cells loaded with fura-2, NPS 021 blocks increases in [Ca²⁺]ᵢ elicited by extracellular Ca²⁺. The IC₅₀ of NPS 021 for blocking this response is about 200 µM and, at concentrations around 500 µM, the increase in [Ca²⁺]ᵢ evoked by extracellular Ca²⁺ is abolished. Significantly, NPS 021 does not by itself cause any change in [Ca²⁺]ᵢ when tested at low [Ca²⁺] (0.5 mM; Fig. 37).

### Example 15: NPS 467 Lowers Serum Ionized Calcium.

Compounds shown to activate the bovine parathyroid cell Ca²⁺ receptor in vitro were tested for hypocalcemic activity in vivo. Male Sprague-Dawley rates (200 g) were maintained on a low calcium diet for one week prior to receiving test substance or vehicle as control. Blood was collected from the tail vein three hours after the intraperitoneal administration of NPS 467. Ionized Ca²⁺ in whole blood or serum was measured with a Ciba-Corning 634 Analyzer according to the instructions provided with the instrument. Serum total calcium, albumin and phosphate were measured by techniques well-known in the art.

NPS 467 caused a dose-dependent reduction in serum or whole blood Ca²⁺ (Fig. 38). The fall in blood Ca²⁺ at this time was paralleled by a proportional fall in the levels of blood total calcium. There was no change in serum albumin or phosphate levels at any of the doses examined. In preliminary studies, NPS 467, at doses effective in lowering blood Ca²⁺, caused a dose-dependent reduction in circulating levels of PTH (Fig. 39). The hypocalcemic effect of NPS 467 was maximal within three hours and returned toward control levels after 24 hours (Fig. 40).

NPS 467 (the EE isomer; see Example 16) was also effective in lowering serum ionized Ca²⁺ in rats maintained on a normal, calcium-replete diet. A single dose of NPS 467 (EE isomer, 10 mg/kg i.p.) caused a rapid fall in serum levels of ionized Ca²⁺ which were maximal by 1 hr (22%) decrease from the control level) and remained depressed at or near this level for up to 6 hours.

### Example 16: NPS 467 Lowers Serum Ionized Calcium in a stereospecific Manner

NPS 467 is a racemic mixture. Resolution of NPS 467 into its two enantiomers was achieved by separation on a chiral column. The EE-isomer (for "early eluting", see Example 21) was about 100-fold more potent than the LE-isomer (for "late-eluting") in activating the bovine parathyroid cell Ca²⁺ receptor in vitro as assessed by the ability of the enantiomers to evoke increases in the [Ca²⁺]ᵢ in parathyroid cells (Fig. 41). Likewise, similar resolution of the novel compound NPS 568 into its enantiomers showed that the EE-isomer was 40-fold more potent than the LE-isomer in causing the mobilization of intracellular Ca²⁺ in bovine parathyroid cells (see Table 6, supra).

The isomers of NPS 467 were examined for effects on serum Ca²⁺ as in Example 15. Consistent with the in vitro results, the EE-isomer or NPS 467 proved to be more potent than the LE-isomer in lowering serum Ca²⁺ in vivo (Fig. 42; each compound was tested at a concentration of 5 mg/kg body weight).

### Example 17: NPS 467 Lowers Serum Ionized Calcium in an in vivo Model of Secondary Hyperparathyroidism

An accepted and widely used animal model of secondary hyperparathyroidism arising from chronic renal failure is the 5/6 nephrectomized rat. Animals receiving such surgery become initially hypocalcemic and, to maintain serum Ca²⁺ levels, there is a compensatory hyperplasia of the parathyroid glands and elevated levels of circulating PTH. Male Sprague-Dawley rats (250 g) received a 5/6 nephrectomy and were allowed to recover for 2 weeks. At this time they were normocalcemic (due to elevated levels of serum PTH). The administration of NPS 467 (EE isomer; 10 mg/kg i.p.) caused a rapid (within 2 hours) fall in serum ionized Ca²⁺ levels to 83% of controls in an animal model of secondary hyperparathyroidism. This suggests that compounds of this sort will effectively depress PTH secretion in patients with secondary hyperparathyroidism and hyperplastic parathyroid glands.

### Example 18: NPS 467 Fails to Lower Serum Ionized Calcium Levels in Parathyroidectomized Animals

To determine the primary target tissue upon which NPS 467 acts to cause a hypocalcemic response, the parathyroid glands in rats were surgically removed. Animals receiving a total parathyroidectomy become hypocalcemic and are largely dependent upon dietary calcium to maintain serum Ca²⁺ homeostasis. Parathyroidectomized animals had serum ionized Ca²⁺ levels of 0.92 mM which fell gradually to 0.76 mM after 6 hours of fasting. The administration of a single dose of NPS 467 EE (10 mg/kg i.p.) did not cause any change in serum ionized Ca²⁺ levels over a period of 6 hours. These results demonstrate that intact parathyroid glands are required for the hypocalcemic effects of NPS 467 EE. The data additionally demonstrate that NPS 467 EE can target the parathyroid glands in vivo. The results are consistent with the view that NPS 467 EE acts on the parathyroid cell Ca²⁺ receptor in vivo to depress secretion of PTH and thereby cause serum levels of ionized Ca²⁺ to fall.

### Example 19: NPS 467 Increases Intracellular Calcium in Human Parathyroid Glands

Dissociated parathyroid cells were prepared from a parathyroid adenoma obtained by surgery from a patient with primary hyperparathyroidism. The cells were loaded with fura-2 and [Ca²⁺]ᵢ measured as described above. Both NPS 467 EE and NPS 568 EE caused concentration-dependent increase in [Ca²⁺]ᵢ. The EC₅₀'s for NPS 467 EE and NPS 568 EE were 20 and 3 µM, respectively. Both these compounds are thus able to increase [Ca²⁺]ᵢ in pathological human tissue and would thus be expected to decrease serum levels of PTH and Ca²⁺ in patients with primary hyperparathyroidism.

### Example 20: Mechanism of Action of NPS 467 at the Parathyroid Cell Calcium Receptor

Dissociated bovine parathyroid cells were used to further explore the mechanism of action of NPS 467 at the receptor level. In the presence of 0.5 mM extracellular Ca²⁺, NPS 467 EE caused a rapid and transient increase in [Ca²⁺]ᵢ which persisted in the presence of 1 *µ*M La³⁺ and was partially depressed by pretreatment with PMA (100 nM for 2 min.). Moreover, NPS 467 (EE isomer, 30 *µ*M) caused a rapid increase in Cl⁻ conductance in Xenopus oocytes injected with parathyroid cell mRNA. All these results are consistent with an action of NPS 467 on the Ca²⁺ receptor. However, the cytosolic Ca²⁺ response to NPS 467 was abolished when parathyroid cells were suspended in Ca²⁺-free buffer. This suggests that NPS 467 cannot, by itself, cause the mobilization of intracellular Ca²⁺. It does, however, elicit responses in parathyroid cells and in oocytes when a small amount of extracellular Ca²⁺ is present. This suggests that partial occupancy of the Ca²⁺⁻binding site is required for NPS 467 to elicit a response. To test this hypothesis, parathyroid cells were suspended in Ca²⁺-free buffer and exposed to a submaximal concentration of neomycin. Neomycin was used because it mimics, in nearly all respects, the effects of extracellular Ca²⁺ on parathyroid cells and on Xenopus oocytes expressing the parathyroid cell Ca²⁺ receptor. The addition of 10 *µ*M neomycin did not by itself cause an increase in [Ca²⁺]ᵢ under these conditions. However the subsequent addition of NPS 467 EE (30 *µ*M) now elicited a transient increase in [Ca²⁺]ᵢ which, because there was no extracellular Ca²⁺ present, must have come from the mobilization of intracellular Ca²⁺. When cells bathed in Ca²⁺-free buffer were exposed to 30 µM NPS 467 there was no increase in [Ca²⁺]ᵢ. This concentration of NPS 467 is maximally effective in increasing [Ca²⁺]ᵢ when extracellular Ca²⁺ (0.5 mM) is present. However, the subsequent addition of 10 *µ*M neomycin now evoked a transient increase in [Ca²⁺]ᵢ. Presumably, neomycin binds to the same site as extracellular Ca²⁺ and can functionally substitute for it. Using a submaximal concentration, which by itself causes no response, achieves partial occupancy of the Ca²⁺-binding site and allows activation of the Ca²⁺ receptor by NPS 467.

Additional studies to further define the mechanism of action of NPS 467 were performed. The cells were once again suspended in Ca²⁺-free buffer to insure that any observed increase in [Ca²⁺]ᵢ resulted from the mobilization of intracellular Ca²⁺. In these experiments, however, a maximally effective concentration (100 µM) of neomycin was used. In the absence of extracellular ca²⁺, 100 *µ*M neomycin evoked a rapid and transient increase in [Ca²⁺]ᵢ. The subsequent addition of 30 µM NPS 467 EE did not cause an increase in [Ca²⁺]ᵢ. In the converse experiment, 30 µM NPS 467 EE was added before 100 µM neomycin. As expected, NPS 467 EE did not cause any increase in [Ca²⁺]ᵢ. It did not, however, affect the increase in [Ca²⁺]ᵢ evoked by the subsequent addition of 100 µM neomycin. These results, obtained with maximally effective concentrations of NPS 467 and neomycin, suggest that these two compounds do not act at the same site. Rather, the results can be sufficiently explained by postulating two separate sites on the Ca²⁺ receptor, one to which extracellular Ca²⁺ and neomycin bind, and another to which NPS 467 and structurally related compounds (such as NPS 568) bind. Ligand binding to the former site can result in full activation of the Ca²⁺ receptor whereas ligand binding to the latter site can only occur and/or be functionally relevant when the extracellular Ca²⁺-binding site is occupied to some as yet undefined degree. It is possible that ligand binding to the extracellular Ca²⁺-binding site exposes a previously occluded binding site for NPS 467. It appears that the NPS 467-binding site is an allosteric site that augments receptor activation in response to ligand binding at the extracellular Ca²⁺-binding site.

The data demonstrate that the parathyroid cell Ca²⁺ receptor possesses at least two distinct sites for organic ligands. One site binds the physiological ligand, extracellular ca²⁺, and certain organic polycations like neomycin. Binding to this site result in full activation of the ca²⁺ receptor, an increase in [Ca²⁺]ᵢ, and the inhibition of PTH secretion. NPS 467 defines a previously unrecognized binding site on the Ca²⁺ receptor. Binding to this site can only occur and/or results in full activation of the Ca²⁺ receptor when the extracellular Ca²⁺-binding site is partially occupied. Ligands acting at either site are effective in suppressing serum Ca²⁺ levels in vivo.

### Example 21: Preparation of NPS 467

In a 250 ml round bottom flask, 10.0 g (100 mmoles) 3'-methoxy acetophenone and 13.5 g (100 mmoles) 3-phenylpropylamine were mixed and treated with 125 mmoles (35.5 g) titanium(IV) isopropoxide. The reaction mixture was stirred 30 minutes at room temperature under a nitrogen atmosphere. After this time 6.3 g (100 mmoles) sodium cyanoborohydride in 100 ml ethanol was added dropwise over the course of 2 minutes. The reaction was stirred room temperature under nitrogen for 16 hours. After this time the reaction mixture was transferred to a 2 L separatory funnel with 1.5 L ethyl ether and 0.5 L water. The phases were equilibrated and the ether layer removed. The remaining aqueous phase was thoroughly extracted with four 1 L portions of ether. The washes were combined, dried over anhydrous potassium carbonate and reduced to a clear, light amber oil.

TLC analysis of this material on silica using chloroform-methanol-isopropylamine (100:5:1) showed product at Rf 0.65 with traces of the two starting materials at Rf 0.99 (3' methoxy acetophenone) and Rf 0.0 (3-phenylpropylamine).

The reaction mixture was chromatographed through silica (48 x 4.6 cm) using a gradient of chloroformmethanol-isopropylamine (99:1:0.1) to (90:10:0.1) which yielded 13.66 g of purified NPS 467. This material was dissolved in Hexane-isopropanol (99:1) containing 0.1% diethylamine to yield a solution with a concentration of 50 mg/ml. Chiral resolution was accomplished by chromatography of 4 ml of this solution (200 mg, maximum to achieve separation) through ChiralCel OD (25 × 2 cm) using 0.7% isopropylamine, 0.07% diethylamine in hexane at 100 ml/min, monitoring optical density at 260 nm. Under these conditions (with injections of 100 mg material) the early eluting isomer (NPS 467EE) began to emerge from the column at - 26 min, the late eluting isomer (NPS 467LE) began to emerge at -34 minutes. Baseline resolution was accomplished with under these conditions. Each optical isomer (free base) was converted to the corresponding hydrochloride salt by dissolving 3 g of the free base in 100 ml ethanol and treating with it with 100 ml water containing 10 molar equivalents HCl. Lyophilization of this solution yielded a white solid.

### Example 22: Preparation of NPS 568

NPS 568 was prepared using the methods described in Example 21 substituting an equivalent amount of 3-(2-chlorophenyl)propylamine for 3-phenylpropylamine. It was found that allowing the mixture of 3'-methoxyacetophenone, 3-(2-chlorophenyl)propylamine and titanium(IV) isopropoxide to stir for 5 hours prior to treatment with NaCNBH₃/EtOH resulted in significantly greater yield (98%).

### Example 23: NPS 467 Lowers Serum Ionized Calcium When Administered orally

Rats (male, Sprague-Dawley, 250-300 g) were fed standard rat chow and fasted overnight prior to the experiment. NPS 467 (EE isomer) was suspended in corn oil and administered as a single oral dose through a gavage needle. Three hours later a sample of blood was taken from the tail vein and assessed for ionized Ca²⁺ levels. Fig. 44 shows that NPS 467 EE caused a dose-dependent reduction in serum levels of ionized Ca²⁺ when administered orally.

Other embodiments are within the following claims.

## Claims

1. A compound having the formula (1): 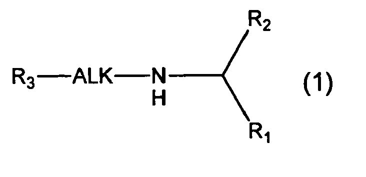 wherein:
ALK is a straight or branched alkylene of from 1 to 6 carbon atoms;
R₁ is lower alkyl of from 1 to 3 carbon atoms or lower haloalkyl of from 1 to 3 carbon atoms substituted with from 1 to 7 halogen atoms;
R₂ and R₃ are independently selected carbocyclic aryl or cycloalkyl groups, either monocyclic or bicyclic, having 5- or 6- membered rings optionally substituted with 1 to 5 substituents independently selected from lower alkyl of 1 to 3 carbon atoms, lower haloalkyl of from 1 to 3 carbon atoms substituted with from 1 to 7 halogen atoms, lower alkoxy of from 1 to 3 carbon atoms, halogen, nitro, amino, alkylamino, amido, lower alkylamido of from 1 to 3 carbon atoms, cyano, hydroxy, acyl of 2 to 4 carbon atoms, lower hydroxyalkyl of from 1 to 3 carbon atoms, or lower thioalkyl of 1 to 3 carbon atoms and pharmaceutically acceptable salts thereof,
provided that when R₂ is 4-aminophenyl, R₁ is lower alkyl of from 1 to 3 carbon atoms and ALK is methylene, then R₃ is other than unsubstituted cyclohexyl or cyclopentyl;
further provided that when R₂ and R₃ are unsubstituted phenyl and ALK is -(CH₂)₂-, then R₁ is other than methyl, ethyl, n-propyl or isopropyl;
further provided that when R₁ is methyl, R₂ is unsubstituted phenyl, and ALK is -CH₂CH₂CH(CH₃)- or -CH₂CH₂CH(CH₂CH₃)- then R₃ is other than 4-hydroxyphenyl;
further provided that when R₁ is methyl, R₂ is unsubstituted phenyl, and ALK is -CH₂CH₂CH(CH₃)- then R₃ is other than 4-loweralkoxyphenyl;
further provided that when R₁ is methyl, R₂ is 2-nitrophenyl, and ALK is CH₂, then R₃ is other than 4-methoxyphenyl;
further provided that when R₁ is methyl, R₂ is unsubstituted cyclohexyl, unsubstituted phenyl, 4-methylphenyl, 2-hydroxyphenyl, 4-bromophenyl, 4-methoxyphenyl or 3-methoxyphenyl, and ALK is CH₂, then R₃ is other than unsubstituted phenyl;
further provided that when R₁ is methyl, R₂ is 3,4-dimethoxyphenyl and ALK is C(CH₃)₂ then R₃ is other than unsubstituted phenyl;
further provided that when R₁ is bromomethyl, R₂ is unsubstituted phenyl, and ALK is CH₂, then R₃ is other than unsubstituted phenyl;
further provided that when R₁ is methyl, R₂ is unsubstituted phenyl, and ALK is CH₂, then R₃ is other than unsubstituted 1-naphthyl, 2-methoxyphenyl, 2-methylphenyl, 2-methylthiophenyl, 2-(dimethylamino)phenyl, 2,4,6-trimethylphenyl, 2-hydroxyphenyl, 3,4-dimethoxyphenyl, 2-chlorophenyl , 4-bromophenyl, 2-chlorophenyl or 4-chlorophenyl;
further provided that when R₁ is methyl, R₂ is unsubstituted α-naphthyl, and ALK is CH₂, CHCH₃, CHCH₂CH₃ or CH(CH₂)₃CH₃, then R₃ is other than 2-hydroxyphenyl;
further provided that when R₁ is methyl, R₂ is unsubstituted phenyl and ALK is CHCH₃, CHCH₂CH₃, CHCH(CH₃)₂, CH₂CH₂, CHC(CH₃)₃, CH(CH₂)₂CH₃, CH(CH₂)₃CH₃ or CHCH(CH₃)(CH₂CH₃), then R₃ is other than unsubstituted phenyl;
further provided that when R₁ is methyl, R₂ is unsubstituted 1-naphthyl, and R₃ is 2-hydroxyphenyl, then ALK is other than CH₂, CHCH₃, CHCH₂CH₃ and CHCH₂CH₂CH₂CH₃;
further provided that when R₁ is methyl, R₂ is unsubstituted phenyl, and R₃ is 2-hydroxyphenyl, then ALK is other than CH₂ and CHCH₃;
further provided that when R₁ is methyl, R₂ is unsubstituted phenyl, 2-chlorophenyl or 2,6-dichlorophenyl, and ALK is CH₂CH₂, then R₃ is other than 3,4-dimethoxyphenyl;
further provided that when R₁ is methyl, R₂ is unsubstituted phenyl and ALK is CHCH₃ or CHCH(CH₃)₂ then R₃ is other than 4-methylphenyl;
further provided that when R₁ is methyl or ethyl, R₂ is unsubstituted phenyl and ALK is CH₂CH₂, then R₃ is other than 3,4-dimethoxyphenyl;
further provided that when R₁ is methyl, R₂ is unsubstituted 1-naphthyl and ALK is CH₂CH₂, then R₃ is other than 3,4-dimethoxyphenyl;
further provided that when R₁ is CH₂CH₃ or CH(CH₃)₂, R₂ is unsubstituted phenyl and ALK is CH(CH₃) then R₃ is other than unsubstituted phenyl;
further provided that when R₁ is CH₂CH₃, R₂ is unsubstituted phenyl and ALK is CH₂ then R₃ is other than unsubstituted phenyl;
further provided that when R₁ is lower alkyl of from 1 to 3 carbon atoms, R₂ is naphthyl, cyclohexyl, or phenyl optionally substituted by halogen, nitro, lower alkyl of 1 to 3 carbon atoms, lower alkoxy of 1 to 3 carbon atoms, and ALK is CH₂, then R₃ is other than 4-hydroxyphenyl and 2-hydroxy-3-loweralkoxyphenyl;
further provided that when R₁ is methyl, R₂ is phenyl and ALK is CHCH₂CH(CH₃)₂, then R₃ is other than 2,5-dimethoxyphenyl;
further provided that when R₁ is CH(CH₃)₂, R₂ is 4-chlorophenyl, and ALK is CH₂ then R₃ is other than 2-methoxyphenyl;
further provided that when R₁ is CH(CH₃)₂, R₂ is unsubstituted phenyl and ALK is CHCH(CH₃)₂, then R₃ is other than unsubstituted phenyl;
further provided that when R₁ is methyl, R₂ is 4-methoxyphenyl or 4-chlorophenyl, and ALK is CH₂, then R₃ is other than unsubstituted phenyl;
further provided that when R₁ is methyl, R₂ is unsubstituted phenyl and ALK is CH(CH₃) then R₃ is other than unsubstituted phenyl, 4-chlorophenyl, 4-bromophenyl, 4-methoxyphenyl, and 4-nitrophenyl;
further provided that when R₁ is methyl, R₂ is unsubstituted phenyl and ALK is CHCH₃, then R₃ is other than 2,3,4-trimethoxyphenyl, 2,3-methylenedioxy-4-methoxyphenyl, 3,4,5-trimethoxyphenyl, 2-hydroxy-3,4-dimethoxyphenyl, 2-isopropoxy-3,4-dimethoxyphenyl, 2,5-dimethoxyphenyl, 3,5-dimethoxyphenyl, 2-hydroxy-6-methoxy-phenyl, 2,6-dimethoxyphenyl, 2-methylaminophenyl, 2-methylphenyl, 2-fluorophenyl, 2-chlorophenyl and 2,4,6-trimethylphenyl;
further provided that when R₁ is n-propyl and R₂ and R₃ are unsubstituted phenyl then ALK is other than (-CH₂-) or (-CH₂CH₂CH₂-);
further provided that when R₁ is ethyl, and R₂ and R₃ are unsubstituted phenyl then ALK is other than (-CH₂-);
further provided that when R₁ is methyl, R₂ is unsubstituted phenyl and ALK is CH₂CHCH₃CH₂, then R₃ is other than 4-isopropylphenyl;
further provided that when R₁ is methyl, R₂ is unsubstituted phenyl, and ALK is CH₂CH(CH₃) then R₃ is other than unsubstituted phenyl;
further provided that when R₁ is methyl, R₂ is unsubstituted phenyl, and ALK is CH₂CH(CH₂CH₃), then R₃ is other than 2,5-dimethoxy-4-methylphenyl;
and pharmaceutically acceptable salts thereof.

2. A compound according to claim 1 wherein ALK is n-propylene and pharmaceutically acceptable salts thereof.

3. A compound according to any preceding claim wherein R₁ is lower alkyl of from 1 to 3 carbon atoms and pharmaceutically acceptable salts thereof.

4. A compound according to any preceding claim wherein R₁ is methyl and pharmaceutically acceptable salts thereof.

5. A compound according to any preceding claim wherein ALK is n-propylene, R₁ is methyl, and R₂ and R₃ are independently carbocyclic aryl, either mono or bicyclic, having 5- or 6- membered rings optionally substituted with 1 to 5 substituents independently selected from lower alkyl of from 1 to 3 carbon atoms, lower haloalkyl of from 1 to 3 carbon atoms substituted with from 1 to 7 halogen atoms, lower alkoxy of from 1 to 3 carbon atoms, halogen, nitro, amino, alkylamino, amido, lower alkylamido of from 1 to 3 carbon atoms, cyano, hydroxy, acyl of 2 to 4 carbon atoms, lower hydroxyalkyl of from 1 to 3 carbon atoms, or lower thioalkyl of 1 to 3 carbon atoms and pharmaceutically acceptable salts thereof.

6. A compound according to any one of claims 1 to 4 wherein R₂ and R₃ are independently selected from phenyl, naphthyl, cyclopentyl, and cyclohexyl and pharmaceutically acceptable salts thereof.

7. A compound according to any one of claims 1 to 4 wherein R₂ and R₃ are independently selected from phenyl, naphthyl and cyclohexyl and pharmaceutically acceptable salts thereof.

8. A compound according to any preceding claim wherein R₂ and R₃ are independently phenyl and pharmaceutically acceptable salts thereof.

9. A compound according to any preceding claim wherein R₂ is monosubstituted phenyl and pharmaceutically acceptable salts thereof.

10. A compound according to any preceding claim wherein R₂ is meta-substituted phenyl and pharmaceutically acceptable salts thereof.

11. A compound according to any preceding claim wherein R₃ is unsubstituted or monosubstituted phenyl and pharmaceutically acceptable salts thereof.

12. A compound according to any preceding claim wherein R₃ is ortho-substituted phenyl and pharmaceutically acceptable salts thereof.

13. A compound according to any preceding claim wherein R₂ is substituted by halogen, haloalkyl, preferably trihalomethyl, or alkoxy and pharmaceutically acceptable salts thereof.

14. A compound according to any preceding claim wherein R₂ is substituted by methoxy and pharmaceutically acceptable salts thereof.

15. A compound according to any preceding claim wherein R₃ is substituted by halogen and pharmaceutically acceptable salts thereof.

16. A compound according to any preceding claim wherein the compound is an R-phenylpropyl-α-phenethylamine derivative and pharmaceutically acceptable salts thereof.

17. A compound according to any preceding claim having the formula 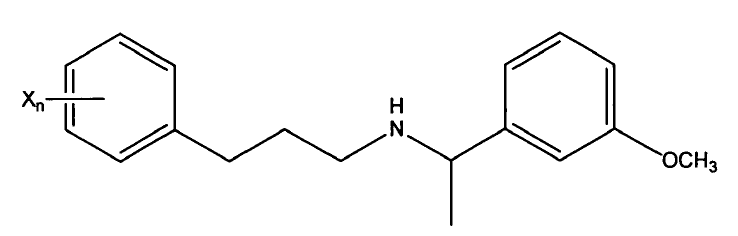 wherein
n is 0 or an integer from 1 to 5;
each group X is independently selected from the group consisting of lower alkyl of 1 to 3 carbon atoms, lower haloalkyl of from 1 to 3 carbon atoms substituted with from 1 to 7 halogen atoms, lower alkoxy of from 1 to 3 carbon atoms, halogen, nitro, amino, alkylamino, amido, lower alkylamido of from 1 to 3 carbon atoms, cyano, hydroxy, acyl of 2 to 4 carbon atoms, lower hydroxyalkyl of from 1 to 3 carbon atoms, or lower thioalkyl of 1 to 3 carbon atoms and pharmaceutically acceptable salts thereof.

18. A compound according to any preceding claim having the formula 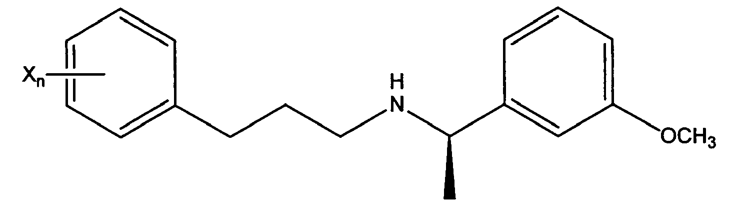 wherein
X and n are as defined in claim 17.
